# EUROPEAN PATENT APPLICATION

(11) **EP 3 543 334 A1**
(43) Date of publication of application: **25.09.2019**
(21) Application number: 18163608.5
(22) Date of filing: 23.03.2018
(51) Int. Cl.: C12N 9/38, A23C 9/12, A23C 21/02, C12N 9/52, C12N 15/77, C12R 1/15

(54) **A NOVEL CELL-BASED PLATFORM FOR CONVERTING LACTOSE CONTAINING FEEDSTOCKS INTO VALUE-ADDED COMPOUNDS**

(71) Applicant: Danmarks Tekniske Universitet, 2800 Kgs. Lyngby (DK)
(72) Inventor: CHEN, Jun, 2850 Nærum (DK); SHEN, Jing, 2850 Nærum (DK); JENSEN, Peter Ruhdal, 2820 Gentofte (DK); SOLEM, Christian, 2990 Nivå (DK)
(74) Representative: Guardian IP Consulting I/S

(57) **Abstract**

The present invention discloses a genetically modified Corynebacterium for hydrolyzing lactose, a mutant gene encoding a modified lactose transporter, as well as a method for hydrolyzing lactose.

SEQUENCE LISTING

<110>  Technical University of Denmark

<120>  A novel cell-based platform for converting lactose containing
       feedstocks into value-added compounds

<130>  P2371EP00

<160>  51

<170>  PatentIn version 3.5

<210>  1
<211>  1905
<212>  DNA
<213>  Streptococcus thermophilus LMD-9


<220>
<221>  CDS
<222>  (1)..(1905)
<223>  LacS gene encoding lac-transporter

<300>
<308>  GenBank CP000419.1
<309>  2014-01-28
<313>  (1273959)..(1275863)

<400>  1
atg gaa aaa tct aaa ggt cag atg aag tct cgt tta tcc tac gca gct      48
Met Glu Lys Ser Lys Gly Gln Met Lys Ser Arg Leu Ser Tyr Ala Ala
1               5                   10                  15

ggt gct ttt ggt aac gac gtc ttc tat gca acc ttg tca aca tac ttt      96
Gly Ala Phe Gly Asn Asp Val Phe Tyr Ala Thr Leu Ser Thr Tyr Phe
            20                  25                  30

atc atg ttt gtg aca act cac ttg ttt aac aca ggt gat cca aag caa      144
Ile Met Phe Val Thr Thr His Leu Phe Asn Thr Gly Asp Pro Lys Gln
        35                  40                  45

aat agt cac tac gta cta tta atc act aac att atc tct att ttg cgt      192
Asn Ser His Tyr Val Leu Leu Ile Thr Asn Ile Ile Ser Ile Leu Arg
    50                  55                  60

atc ttg gaa gta ttt atc gat cca ttg atc ggt aat atg att gat aac      240
Ile Leu Glu Val Phe Ile Asp Pro Leu Ile Gly Asn Met Ile Asp Asn
65                  70                  75                  80

act aat act aag tat ggt aaa ttc aaa cca tgg gta gtt ggt ggt ggt      288
Thr Asn Thr Lys Tyr Gly Lys Phe Lys Pro Trp Val Val Gly Gly Gly
                85                  90                  95

atc atc agt tct atc acc ttg ttg ctt ctc ttc acc gat tta ggt ggt      336
Ile Ile Ser Ser Ile Thr Leu Leu Leu Leu Phe Thr Asp Leu Gly Gly
            100                 105                 110

ttg aat aaa aca aat cct ttc ttg tac ctt gta ctt ttt gga att atc      384
Leu Asn Lys Thr Asn Pro Phe Leu Tyr Leu Val Leu Phe Gly Ile Ile
        115                 120                 125

tac ctt ata atg gat gtc ttc tac tcg att aaa gat atc ggt ttc tgg      432

Tyr Leu Ile Met Asp Val Phe Tyr Ser Ile Lys Asp Ile Gly Phe Trp
    130                 135                 140

tca atg ctt cct gcc ttg tct ctt gac agt cac gaa cgt gaa aaa atg      480
Ser Met Leu Pro Ala Leu Ser Leu Asp Ser His Glu Arg Glu Lys Met
145                 150                 155                 160

gca act ttt gcc cgt att ggt tct acg att ggt gcc aat att gta ggt      528
Ala Thr Phe Ala Arg Ile Gly Ser Thr Ile Gly Ala Asn Ile Val Gly
                165                 170                 175

gtt gcc atc atg cca atc gtt ttg ttc ttc tct atg acg aac agt agt      576
Val Ala Ile Met Pro Ile Val Leu Phe Phe Ser Met Thr Asn Ser Ser
            180                 185                 190

ggc tct gga gat aaa tct gga tgg ttc tgg ttt gca ttt atc gtt gca      624
Gly Ser Gly Asp Lys Ser Gly Trp Phe Trp Phe Ala Phe Ile Val Ala
        195                 200                 205

ctc att ggt gtg att aca tca att gct gtt ggt att ggt aca cgt gaa      672
Leu Ile Gly Val Ile Thr Ser Ile Ala Val Gly Ile Gly Thr Arg Glu
    210                 215                 220

gtt gag tca aaa ctt cgt gat aac aat gaa aaa act agc ctt aaa caa      720
Val Glu Ser Lys Leu Arg Asp Asn Asn Glu Lys Thr Ser Leu Lys Gln
225                 230                 235                 240

gtc ttt aaa gtt ctt ggt aaa aac gac caa ttg atg tgg ctg tct ctc      768
Val Phe Lys Val Leu Gly Lys Asn Asp Gln Leu Met Trp Leu Ser Leu
                245                 250                 255

gga tat tgg ttc tat ggt ctt ggt att aat aca ctt aat gct ctt caa      816
Gly Tyr Trp Phe Tyr Gly Leu Gly Ile Asn Thr Leu Asn Ala Leu Gln
            260                 265                 270

ctt tat tac ttc aca ttt atc ctt ggt gat tca ggg aaa tac tca atc      864
Leu Tyr Tyr Phe Thr Phe Ile Leu Gly Asp Ser Gly Lys Tyr Ser Ile
        275                 280                 285

ctt tac gga ttg aat aca gtt gtt ggt ttg gtt tca gtt tca ctg ttc      912
Leu Tyr Gly Leu Asn Thr Val Val Gly Leu Val Ser Val Ser Leu Phe
    290                 295                 300

cct agt ctt gct ggt aaa ttc aac cgt aaa cgt ttg ttc tat gga tgt      960
Pro Ser Leu Ala Gly Lys Phe Asn Arg Lys Arg Leu Phe Tyr Gly Cys
305                 310                 315                 320

att gca gta atg ctc ggt ggt atc gga atc ttt agt att gca ggt act     1008
Ile Ala Val Met Leu Gly Gly Ile Gly Ile Phe Ser Ile Ala Gly Thr
                325                 330                 335

tca ctt cca atg atc ttg act gca gct gaa ctc ttc ttc att cca caa     1056
Ser Leu Pro Met Ile Leu Thr Ala Ala Glu Leu Phe Phe Ile Pro Gln
            340                 345                 350

cct ctt gtg ttc ctt gtt atc ctt atg att atc tct gac tca gta gaa     1104
Pro Leu Val Phe Leu Val Ile Leu Met Ile Ile Ser Asp Ser Val Glu
        355                 360                 365

tat ggt caa tgg aaa ttg gga cac cgt gat gaa tca ctt act ttg tca     1152
Tyr Gly Gln Trp Lys Leu Gly His Arg Asp Glu Ser Leu Thr Leu Ser
    370                 375                 380

gtt cgt cca ctt gtt gat aaa ctt ggt ggt gcg atg tca aac tgg ctt     1200
Val Arg Pro Leu Val Asp Lys Leu Gly Gly Ala Met Ser Asn Trp Leu
385                 390                 395                 400

gtt tct aca att gcc gta gct gcc ggt atg aca aca ggt gcc tca gca     1248
Val Ser Thr Ile Ala Val Ala Ala Gly Met Thr Thr Gly Ala Ser Ala
                405                 410                 415

tca aca att aca aca cat caa cag tct att ttt aag ctt agc atg ttt     1296
Ser Thr Ile Thr Thr His Gln Gln Ser Ile Phe Lys Leu Ser Met Phe
            420                 425                 430

ggt ttc cca gca gca gca atg ctt atc ggt gcc ttc att att gct cgt     1344
Gly Phe Pro Ala Ala Ala Met Leu Ile Gly Ala Phe Ile Ile Ala Arg
        435                 440                 445

aaa atc act ttg act gaa gca cgt cac gct aaa att gtt gaa gaa ttg     1392
Lys Ile Thr Leu Thr Glu Ala Arg His Ala Lys Ile Val Glu Glu Leu
    450                 455                 460

gaa cat cgc ttt agc gta gca act tct gaa aat gaa gtt aaa gct aac     1440
Glu His Arg Phe Ser Val Ala Thr Ser Glu Asn Glu Val Lys Ala Asn
465                 470                 475                 480

gtc gta tct ctt gta acc cct aca act ggt tat ttg gtt gat ctc tca     1488
Val Val Ser Leu Val Thr Pro Thr Thr Gly Tyr Leu Val Asp Leu Ser
                485                 490                 495

agt gtt aat gat gaa cac ttt gct tca ggt agc atg ggt aaa ggt ttc     1536
Ser Val Asn Asp Glu His Phe Ala Ser Gly Ser Met Gly Lys Gly Phe
            500                 505                 510

gcc att aaa cct act gat gga gct gtc ttt gca cca att agt ggt acc     1584
Ala Ile Lys Pro Thr Asp Gly Ala Val Phe Ala Pro Ile Ser Gly Thr
        515                 520                 525

att cgt caa att ctt cct act cgc cat gca gtt ggt att gaa agt gaa     1632
Ile Arg Gln Ile Leu Pro Thr Arg His Ala Val Gly Ile Glu Ser Glu
    530                 535                 540

gat ggt gtc att gtt ctt atc cac gtt ggc atc gga aca gtt aaa ctt     1680
Asp Gly Val Ile Val Leu Ile His Val Gly Ile Gly Thr Val Lys Leu
545                 550                 555                 560

aat ggt gaa gga ttc att agt tac gta gaa caa ggt gat cgt gtt gaa     1728
Asn Gly Glu Gly Phe Ile Ser Tyr Val Glu Gln Gly Asp Arg Val Glu
                565                 570                 575

gtt gga caa aaa ctt ctt gag ttc tgg tca cca att att gag aaa aat     1776
Val Gly Gln Lys Leu Leu Glu Phe Trp Ser Pro Ile Ile Glu Lys Asn
            580                 585                 590

ggt ctt gat gac aca gta ctt gtc act gta act aat tca gaa aaa ttc     1824
Gly Leu Asp Asp Thr Val Leu Val Thr Val Thr Asn Ser Glu Lys Phe
        595                 600                 605

agt gct ttc cat ctt gaa caa aaa gtt gga gaa aag gta gaa gct ttg     1872
Ser Ala Phe His Leu Glu Gln Lys Val Gly Glu Lys Val Glu Ala Leu
    610                 615                 620

tct gaa gtt att acc ttc aaa aaa gga gaa taa                         1905
Ser Glu Val Ile Thr Phe Lys Lys Gly Glu
625                 630

<210>  2
<211>  634
<212>  PRT
<213>  Streptococcus thermophilus LMD-9

<400>  2

Met Glu Lys Ser Lys Gly Gln Met Lys Ser Arg Leu Ser Tyr Ala Ala
1               5                   10                  15

Gly Ala Phe Gly Asn Asp Val Phe Tyr Ala Thr Leu Ser Thr Tyr Phe
            20                  25                  30

Ile Met Phe Val Thr Thr His Leu Phe Asn Thr Gly Asp Pro Lys Gln
        35                  40                  45

Asn Ser His Tyr Val Leu Leu Ile Thr Asn Ile Ile Ser Ile Leu Arg
    50                  55                  60

Ile Leu Glu Val Phe Ile Asp Pro Leu Ile Gly Asn Met Ile Asp Asn
65                  70                  75                  80

Thr Asn Thr Lys Tyr Gly Lys Phe Lys Pro Trp Val Val Gly Gly Gly
                85                  90                  95

Ile Ile Ser Ser Ile Thr Leu Leu Leu Leu Phe Thr Asp Leu Gly Gly
            100                 105                 110

Leu Asn Lys Thr Asn Pro Phe Leu Tyr Leu Val Leu Phe Gly Ile Ile
        115                 120                 125

Tyr Leu Ile Met Asp Val Phe Tyr Ser Ile Lys Asp Ile Gly Phe Trp
    130                 135                 140

Ser Met Leu Pro Ala Leu Ser Leu Asp Ser His Glu Arg Glu Lys Met
145                 150                 155                 160

Ala Thr Phe Ala Arg Ile Gly Ser Thr Ile Gly Ala Asn Ile Val Gly
                165                 170                 175

Val Ala Ile Met Pro Ile Val Leu Phe Phe Ser Met Thr Asn Ser Ser
            180                 185                 190

Gly Ser Gly Asp Lys Ser Gly Trp Phe Trp Phe Ala Phe Ile Val Ala
        195                 200                 205

Leu Ile Gly Val Ile Thr Ser Ile Ala Val Gly Ile Gly Thr Arg Glu
    210                 215                 220

Val Glu Ser Lys Leu Arg Asp Asn Asn Glu Lys Thr Ser Leu Lys Gln
225                 230                 235                 240

Val Phe Lys Val Leu Gly Lys Asn Asp Gln Leu Met Trp Leu Ser Leu
                245                 250                 255

Gly Tyr Trp Phe Tyr Gly Leu Gly Ile Asn Thr Leu Asn Ala Leu Gln
            260                 265                 270

Leu Tyr Tyr Phe Thr Phe Ile Leu Gly Asp Ser Gly Lys Tyr Ser Ile
        275                 280                 285

Leu Tyr Gly Leu Asn Thr Val Val Gly Leu Val Ser Val Ser Leu Phe
    290                 295                 300

Pro Ser Leu Ala Gly Lys Phe Asn Arg Lys Arg Leu Phe Tyr Gly Cys
305                 310                 315                 320

Ile Ala Val Met Leu Gly Gly Ile Gly Ile Phe Ser Ile Ala Gly Thr
                325                 330                 335

Ser Leu Pro Met Ile Leu Thr Ala Ala Glu Leu Phe Phe Ile Pro Gln
            340                 345                 350

Pro Leu Val Phe Leu Val Ile Leu Met Ile Ile Ser Asp Ser Val Glu
        355                 360                 365

Tyr Gly Gln Trp Lys Leu Gly His Arg Asp Glu Ser Leu Thr Leu Ser
    370                 375                 380

Val Arg Pro Leu Val Asp Lys Leu Gly Gly Ala Met Ser Asn Trp Leu
385                 390                 395                 400

Val Ser Thr Ile Ala Val Ala Ala Gly Met Thr Thr Gly Ala Ser Ala
                405                 410                 415

Ser Thr Ile Thr Thr His Gln Gln Ser Ile Phe Lys Leu Ser Met Phe
            420                 425                 430

Gly Phe Pro Ala Ala Ala Met Leu Ile Gly Ala Phe Ile Ile Ala Arg
        435                 440                 445

Lys Ile Thr Leu Thr Glu Ala Arg His Ala Lys Ile Val Glu Glu Leu
    450                 455                 460

Glu His Arg Phe Ser Val Ala Thr Ser Glu Asn Glu Val Lys Ala Asn

465                 470                 475                 480

Val Val Ser Leu Val Thr Pro Thr Thr Gly Tyr Leu Val Asp Leu Ser
                485                 490                 495

Ser Val Asn Asp Glu His Phe Ala Ser Gly Ser Met Gly Lys Gly Phe
            500                 505                 510

Ala Ile Lys Pro Thr Asp Gly Ala Val Phe Ala Pro Ile Ser Gly Thr
        515                 520                 525

Ile Arg Gln Ile Leu Pro Thr Arg His Ala Val Gly Ile Glu Ser Glu
    530                 535                 540

Asp Gly Val Ile Val Leu Ile His Val Gly Ile Gly Thr Val Lys Leu
545                 550                 555                 560

Asn Gly Glu Gly Phe Ile Ser Tyr Val Glu Gln Gly Asp Arg Val Glu
                565                 570                 575

Val Gly Gln Lys Leu Leu Glu Phe Trp Ser Pro Ile Ile Glu Lys Asn
            580                 585                 590

Gly Leu Asp Asp Thr Val Leu Val Thr Val Thr Asn Ser Glu Lys Phe
        595                 600                 605

Ser Ala Phe His Leu Glu Gln Lys Val Gly Glu Lys Val Glu Ala Leu
    610                 615                 620

Ser Glu Val Ile Thr Phe Lys Lys Gly Glu
625                 630

<210>  3
<211>  1905
<212>  DNA
<213>  Streptococcus salivarius strain HSISS4

<220>
<221>  CDS
<222>  (1)..(1905)
<223>  LacS gene encoding lac-transporter

<300>
<308>  GenBank CP013216.1
<309>  2016-03-09
<313>  (1344163)..(1346067)

<400>  3
atg gga aaa tct aaa ggt caa atg aag tct cgt cta tcc tat tca gct        48
Met Gly Lys Ser Lys Gly Gln Met Lys Ser Arg Leu Ser Tyr Ser Ala
1               5                   10                  15

ggt gct ttt ggt aac gac gtc ttc tat gca acg ttg tca aca tac ttt 96
Gly Ala Phe Gly Asn Asp Val Phe Tyr Ala Thr Leu Ser Thr Tyr Phe
            20                  25                  30

atc atg ttt gtg aca act cac ttg ttt aac aca ggt gat cca aag caa 144
Ile Met Phe Val Thr Thr His Leu Phe Asn Thr Gly Asp Pro Lys Gln
        35                  40                  45

aat agt cac tac gta cta tta att act aac atc atc gct att ttg cgt 192
Asn Ser His Tyr Val Leu Leu Ile Thr Asn Ile Ile Ala Ile Leu Arg
    50                  55                  60

atc ttg gaa gta ttt atc gat cca ttg atc ggt aac atg att gat aac 240
Ile Leu Glu Val Phe Ile Asp Pro Leu Ile Gly Asn Met Ile Asp Asn
65                  70                  75                  80

act cat act aag tat ggt aaa ttc aaa cca tgg gta gtt ggt ggt ggt 288
Thr His Thr Lys Tyr Gly Lys Phe Lys Pro Trp Val Val Gly Gly Gly
                85                  90                  95

atc atc agt tct atc gcc ttg ttg ctt ctc ttc acc gat tta ggt ggt 336
Ile Ile Ser Ser Ile Ala Leu Leu Leu Leu Phe Thr Asp Leu Gly Gly
            100                 105                 110

ttg aat aaa aca aat cct ttc ttg tac ctt gta ctt ttc gga att atc 384
Leu Asn Lys Thr Asn Pro Phe Leu Tyr Leu Val Leu Phe Gly Ile Ile
        115                 120                 125

tac ctt gta atg gat gtc ttc tac tca att aaa gat atc ggt ttc tgg 432
Tyr Leu Val Met Asp Val Phe Tyr Ser Ile Lys Asp Ile Gly Phe Trp
    130                 135                 140

tca atg att cct gcc ttg tct ctt gac agt cat gaa cgt gaa aaa atg 480
Ser Met Ile Pro Ala Leu Ser Leu Asp Ser His Glu Arg Glu Lys Met
145                 150                 155                 160

gca act ttt gcc cgt att ggt tct acg att ggt gcc aat att gta ggt 528
Ala Thr Phe Ala Arg Ile Gly Ser Thr Ile Gly Ala Asn Ile Val Gly
                165                 170                 175

gtt gcc atc atg cca atc gtt ttg ttc ttc tct atg acg aac agt agt 576
Val Ala Ile Met Pro Ile Val Leu Phe Phe Ser Met Thr Asn Ser Ser
            180                 185                 190

ggc tct gga gat aaa tct gga tgg ttc tgg ttt gca ttt atc gtt gct 624
Gly Ser Gly Asp Lys Ser Gly Trp Phe Trp Phe Ala Phe Ile Val Ala
        195                 200                 205

ctc att ggt gtg att aca tca att gct gtt ggt att ggt aca cgt gaa 672
Leu Ile Gly Val Ile Thr Ser Ile Ala Val Gly Ile Gly Thr Arg Glu
    210                 215                 220

gtt gag tca aaa ctt cgt gat aac aaa gaa aaa act agc ctt aaa caa 720
Val Glu Ser Lys Leu Arg Asp Asn Lys Glu Lys Thr Ser Leu Lys Gln
225                 230                 235                 240

gtc ttt aaa gtt ctt ggt aaa aac gac caa ttg atg tgg ttg tct ctc 768
Val Phe Lys Val Leu Gly Lys Asn Asp Gln Leu Met Trp Leu Ser Leu
                245                 250                 255

gga tat tgg ttc tat ggt ctt ggt att aat aca ctt aat gct ctt caa 816
Gly Tyr Trp Phe Tyr Gly Leu Gly Ile Asn Thr Leu Asn Ala Leu Gln

260                 265                 270

ctt ttt tac ttc aca ttt atc ctt ggt gat cca ggg aaa tac tca atc       864
Leu Phe Tyr Phe Thr Phe Ile Leu Gly Asp Pro Gly Lys Tyr Ser Ile
        275                 280                 285

ctt tac gga ttg aat aca gtt gtt ggt ttg gtt tca gtt tca ctc ttc       912
Leu Tyr Gly Leu Asn Thr Val Val Gly Leu Val Ser Val Ser Leu Phe
    290                 295                 300

cct agc cta gct ggt aaa ttc aac cgt aaa cgt ttg ttc tat gga tgt       960
Pro Ser Leu Ala Gly Lys Phe Asn Arg Lys Arg Leu Phe Tyr Gly Cys
305                 310                 315                 320

att gca gta atg ctc ggt ggt atc gga atc ttt agt att gca gga aca      1008
Ile Ala Val Met Leu Gly Gly Ile Gly Ile Phe Ser Ile Ala Gly Thr
                325                 330                 335

tca ctt cca atg atc ttg act gct gct gaa ctc ttc ttt att cca caa      1056
Ser Leu Pro Met Ile Leu Thr Ala Ala Glu Leu Phe Phe Ile Pro Gln
            340                 345                 350

cct ctt gtg ttc ctt gtt atc ctc atg att atc tct gac tca gta gaa      1104
Pro Leu Val Phe Leu Val Ile Leu Met Ile Ile Ser Asp Ser Val Glu
        355                 360                 365

tat ggt caa tgg aaa ttg gga cac cgt gat gaa tca ctt act ttg tca      1152
Tyr Gly Gln Trp Lys Leu Gly His Arg Asp Glu Ser Leu Thr Leu Ser
    370                 375                 380

gtt cgt cca ctt gtt gat aaa ctt ggt ggt gcg atg tca aac tgg ctt      1200
Val Arg Pro Leu Val Asp Lys Leu Gly Gly Ala Met Ser Asn Trp Leu
385                 390                 395                 400

gtt tct aca att gcc gta gct gct ggt atg aca aca ggt gcc tca gca      1248
Val Ser Thr Ile Ala Val Ala Ala Gly Met Thr Thr Gly Ala Ser Ala
                405                 410                 415

tca aca att aca aca cat caa cag tct atc ttt aag ctt agc atg ttt      1296
Ser Thr Ile Thr Thr His Gln Gln Ser Ile Phe Lys Leu Ser Met Phe
            420                 425                 430

ggt ttc cca gca gca gca atg ctt atc gga gcc ttc att att gct cgt      1344
Gly Phe Pro Ala Ala Ala Met Leu Ile Gly Ala Phe Ile Ile Ala Arg
        435                 440                 445

aaa atc act ttg act gaa gca cgt cac gct gaa atc gtc gaa gaa ttg      1392
Lys Ile Thr Leu Thr Glu Ala Arg His Ala Glu Ile Val Glu Glu Leu
    450                 455                 460

gaa cat cgc ttt agc gta gca act tct gaa aat gaa gtt aaa gct aac      1440
Glu His Arg Phe Ser Val Ala Thr Ser Glu Asn Glu Val Lys Ala Asn
465                 470                 475                 480

gtc gta tct ctt gta aac cct aca act ggt cat ttg gtt gac ctc tca      1488
Val Val Ser Leu Val Asn Pro Thr Thr Gly His Leu Val Asp Leu Ser
                485                 490                 495

agt gtt aat gat gaa cac ttt gct tca ggt agc atg ggt aaa ggt ttc      1536
Ser Val Asn Asp Glu His Phe Ala Ser Gly Ser Met Gly Lys Gly Phe
            500                 505                 510

gcc att aaa cct act gat gga gct gtc ttt gca cca att agt ggt acc      1584

Ala Ile Lys Pro Thr Asp Gly Ala Val Phe Ala Pro Ile Ser Gly Thr
        515                 520                 525

att cgt caa gtt ctt cct act cgc cat gca gtt ggt att gaa agt gaa      1632
Ile Arg Gln Val Leu Pro Thr Arg His Ala Val Gly Ile Glu Ser Glu
    530                 535                 540

gat ggt gtc att gtt ctt atc cac gtt ggt atc gga aca gtt aaa ctt      1680
Asp Gly Val Ile Val Leu Ile His Val Gly Ile Gly Thr Val Lys Leu
545                 550                 555                 560

aaa ggt gaa ggg ttc att agt tac gta gaa caa ggt gat cgt gtt gaa      1728
Lys Gly Glu Gly Phe Ile Ser Tyr Val Glu Gln Gly Asp Arg Val Glu
                565                 570                 575

gtt gga caa aaa ctt ctt gag ttc tgg tca cca att att gag aaa aat      1776
Val Gly Gln Lys Leu Leu Glu Phe Trp Ser Pro Ile Ile Glu Lys Asn
            580                 585                 590

ggt ctt gat gac aca gta ctt gtc act gta act aat tca gaa aaa ttc      1824
Gly Leu Asp Asp Thr Val Leu Val Thr Val Thr Asn Ser Glu Lys Phe
        595                 600                 605

agt gct ttc cat ctt gaa caa gaa gtt gga gaa aag gta gaa gct ttg      1872
Ser Ala Phe His Leu Glu Gln Glu Val Gly Glu Lys Val Glu Ala Leu
    610                 615                 620

tct gaa gtt att acc ttc aaa aaa gga gaa taa                          1905
Ser Glu Val Ile Thr Phe Lys Lys Gly Glu
625                 630

<210>  4
<211>  634
<212>  PRT
<213>  Streptococcus salivarius strain HSISS4

<400>  4

Met Gly Lys Ser Lys Gly Gln Met Lys Ser Arg Leu Ser Tyr Ser Ala
1               5                   10                  15

Gly Ala Phe Gly Asn Asp Val Phe Tyr Ala Thr Leu Ser Thr Tyr Phe
            20                  25                  30

Ile Met Phe Val Thr Thr His Leu Phe Asn Thr Gly Asp Pro Lys Gln
        35                  40                  45

Asn Ser His Tyr Val Leu Leu Ile Thr Asn Ile Ile Ala Ile Leu Arg
    50                  55                  60

Ile Leu Glu Val Phe Ile Asp Pro Leu Ile Gly Asn Met Ile Asp Asn
65                  70                  75                  80

Thr His Thr Lys Tyr Gly Lys Phe Lys Pro Trp Val Val Gly Gly Gly
                85                  90                  95

Ile Ile Ser Ser Ile Ala Leu Leu Leu Leu Phe Thr Asp Leu Gly Gly
            100                 105                 110

Leu Asn Lys Thr Asn Pro Phe Leu Tyr Leu Val Leu Phe Gly Ile Ile
        115                 120                 125

Tyr Leu Val Met Asp Val Phe Tyr Ser Ile Lys Asp Ile Gly Phe Trp
    130                 135                 140

Ser Met Ile Pro Ala Leu Ser Leu Asp Ser His Glu Arg Glu Lys Met
145                 150                 155                 160

Ala Thr Phe Ala Arg Ile Gly Ser Thr Ile Gly Ala Asn Ile Val Gly
                165                 170                 175

Val Ala Ile Met Pro Ile Val Leu Phe Phe Ser Met Thr Asn Ser Ser
            180                 185                 190

Gly Ser Gly Asp Lys Ser Gly Trp Phe Trp Phe Ala Phe Ile Val Ala
        195                 200                 205

Leu Ile Gly Val Ile Thr Ser Ile Ala Val Gly Ile Gly Thr Arg Glu
    210                 215                 220

Val Glu Ser Lys Leu Arg Asp Asn Lys Glu Lys Thr Ser Leu Lys Gln
225                 230                 235                 240

Val Phe Lys Val Leu Gly Lys Asn Asp Gln Leu Met Trp Leu Ser Leu
                245                 250                 255

Gly Tyr Trp Phe Tyr Gly Leu Gly Ile Asn Thr Leu Asn Ala Leu Gln
            260                 265                 270

Leu Phe Tyr Phe Thr Phe Ile Leu Gly Asp Pro Gly Lys Tyr Ser Ile
        275                 280                 285

Leu Tyr Gly Leu Asn Thr Val Val Gly Leu Val Ser Val Ser Leu Phe
    290                 295                 300

Pro Ser Leu Ala Gly Lys Phe Asn Arg Lys Arg Leu Phe Tyr Gly Cys
305                 310                 315                 320

Ile Ala Val Met Leu Gly Gly Ile Gly Ile Phe Ser Ile Ala Gly Thr
                325                 330                 335

Ser Leu Pro Met Ile Leu Thr Ala Ala Glu Leu Phe Phe Ile Pro Gln
            340                 345                 350

Pro Leu Val Phe Leu Val Ile Leu Met Ile Ile Ser Asp Ser Val Glu
        355                 360                 365

Tyr Gly Gln Trp Lys Leu Gly His Arg Asp Glu Ser Leu Thr Leu Ser
    370                 375                 380

Val Arg Pro Leu Val Asp Lys Leu Gly Gly Ala Met Ser Asn Trp Leu
385                 390                 395                 400

Val Ser Thr Ile Ala Val Ala Ala Gly Met Thr Thr Gly Ala Ser Ala
                405                 410                 415

Ser Thr Ile Thr Thr His Gln Gln Ser Ile Phe Lys Leu Ser Met Phe
            420                 425                 430

Gly Phe Pro Ala Ala Ala Met Leu Ile Gly Ala Phe Ile Ile Ala Arg
        435                 440                 445

Lys Ile Thr Leu Thr Glu Ala Arg His Ala Glu Ile Val Glu Glu Leu
    450                 455                 460

Glu His Arg Phe Ser Val Ala Thr Ser Glu Asn Glu Val Lys Ala Asn
465                 470                 475                 480

Val Val Ser Leu Val Asn Pro Thr Thr Gly His Leu Val Asp Leu Ser
                485                 490                 495

Ser Val Asn Asp Glu His Phe Ala Ser Gly Ser Met Gly Lys Gly Phe
            500                 505                 510

Ala Ile Lys Pro Thr Asp Gly Ala Val Phe Ala Pro Ile Ser Gly Thr
        515                 520                 525

Ile Arg Gln Val Leu Pro Thr Arg His Ala Val Gly Ile Glu Ser Glu
    530                 535                 540

Asp Gly Val Ile Val Leu Ile His Val Gly Ile Gly Thr Val Lys Leu
545                 550                 555                 560

Lys Gly Glu Gly Phe Ile Ser Tyr Val Glu Gln Gly Asp Arg Val Glu
                565                 570                 575

Val Gly Gln Lys Leu Leu Glu Phe Trp Ser Pro Ile Ile Glu Lys Asn
            580                 585                 590

Gly Leu Asp Asp Thr Val Leu Val Thr Val Thr Asn Ser Glu Lys Phe
        595                 600                 605

Ser Ala Phe His Leu Glu Gln Glu Val Gly Glu Lys Val Glu Ala Leu
    610                 615                 620

Ser Glu Val Ile Thr Phe Lys Lys Gly Glu
625                 630

<210>  5
<211>  1905
<212>  DNA
<213>  Streptococcus infantarius subsp. infantarius strain CJ18

<220>
<221>  CDS
<222>  (1)..(1905)
<223>  LacS gene encoding lac-transporter

<300>
<308>  GenBank CP003295.1
<309>  2014-01-31
<313>  (959441)..(961345)

<400>  5
atg aga aaa tct aac gtt cag atg aag tct cgt cta tcc tat gca gcg      48
Met Arg Lys Ser Asn Val Gln Met Lys Ser Arg Leu Ser Tyr Ala Ala
1               5                   10                  15

ggt gct ttt ggt aac gac gtc ttc tat gca acg ttg tca aca tac ttt      96
Gly Ala Phe Gly Asn Asp Val Phe Tyr Ala Thr Leu Ser Thr Tyr Phe
            20                  25                  30

att atg ttt gtg aca act cac ttg ttt aac aca ggt gat cca aag caa      144
Ile Met Phe Val Thr Thr His Leu Phe Asn Thr Gly Asp Pro Lys Gln
        35                  40                  45

aat agt cac tat gta cta tta att act aac atc atc tct att ttg cgt      192
Asn Ser His Tyr Val Leu Leu Ile Thr Asn Ile Ile Ser Ile Leu Arg
    50                  55                  60

atc ttg gaa gta ttt atc gat cca ttg atc ggt aac atg att gat aat      240
Ile Leu Glu Val Phe Ile Asp Pro Leu Ile Gly Asn Met Ile Asp Asn
65                  70                  75                  80

act cat acc aag tat ggt aaa ttc aaa cca tgg gta gtt ggt ggt ggt      288
Thr His Thr Lys Tyr Gly Lys Phe Lys Pro Trp Val Val Gly Gly Gly
                85                  90                  95

atc atc agt tct atc acc ttg ttg ctt ctc ttc acc gat tta ggt ggt      336
Ile Ile Ser Ser Ile Thr Leu Leu Leu Leu Phe Thr Asp Leu Gly Gly
            100                 105                 110

ttg aat aaa aca aat cct ttc ttg tac ctt gta ctt ttc gga att atc      384
Leu Asn Lys Thr Asn Pro Phe Leu Tyr Leu Val Leu Phe Gly Ile Ile
        115                 120                 125

tac ctt gta atg gat gtc ttc tac tca att aaa gat atc ggt ttc tgg      432
Tyr Leu Val Met Asp Val Phe Tyr Ser Ile Lys Asp Ile Gly Phe Trp
    130                 135                 140

tca atg att cct gcc ttg tct ttt gac agt cat gaa cgt gaa aaa atg 480
Ser Met Ile Pro Ala Leu Ser Phe Asp Ser His Glu Arg Glu Lys Met
145                 150                 155                 160

gca act ttt gcc cgt att ggt tct acg att ggt gcc aat att gta ggt 528
Ala Thr Phe Ala Arg Ile Gly Ser Thr Ile Gly Ala Asn Ile Val Gly
                165                 170                 175

gtt gcc att atg cca atc gtt ttg ttc ttc tct atg acg aac agt agt 576
Val Ala Ile Met Pro Ile Val Leu Phe Phe Ser Met Thr Asn Ser Ser
            180                 185                 190

ggc tct gga gat aaa tct gga tgg ttc tgg ttt gca ttt atc gtt gct 624
Gly Ser Gly Asp Lys Ser Gly Trp Phe Trp Phe Ala Phe Ile Val Ala
        195                 200                 205

ctc att ggt gtg att aca tca att gct gtt ggt att ggt aca cgt gaa 672
Leu Ile Gly Val Ile Thr Ser Ile Ala Val Gly Ile Gly Thr Arg Glu
    210                 215                 220

gtt gag tca aaa ctt cgt gat aac aat gaa aaa act agc ctt aaa caa 720
Val Glu Ser Lys Leu Arg Asp Asn Asn Glu Lys Thr Ser Leu Lys Gln
225                 230                 235                 240

gtc ttt aaa gtt ctt ggt aaa aac gac caa ttg atg tgg ttg tct ctc 768
Val Phe Lys Val Leu Gly Lys Asn Asp Gln Leu Met Trp Leu Ser Leu
                245                 250                 255

gga tat tgg ttc tat ggt ctt ggt att aat aca ctt aat gct ctt caa 816
Gly Tyr Trp Phe Tyr Gly Leu Gly Ile Asn Thr Leu Asn Ala Leu Gln
            260                 265                 270

ctt tat tac ttc aca ttt atc ctt ggt gat tca gga aaa tac tca atc 864
Leu Tyr Tyr Phe Thr Phe Ile Leu Gly Asp Ser Gly Lys Tyr Ser Ile
        275                 280                 285

ctt tac gga ttg aat aca gtt gtt ggt ttg gtt tca gtt tca ctg ttc 912
Leu Tyr Gly Leu Asn Thr Val Val Gly Leu Val Ser Val Ser Leu Phe
    290                 295                 300

cct agt ctt gct ggt aaa ttc aac cgt aaa cgt ttg ttc tat gga tgt 960
Pro Ser Leu Ala Gly Lys Phe Asn Arg Lys Arg Leu Phe Tyr Gly Cys
305                 310                 315                 320

att gca gta atg atc ggt ggt atc gga atc ttt agt att gca ggt act 1008
Ile Ala Val Met Ile Gly Gly Ile Gly Ile Phe Ser Ile Ala Gly Thr
                325                 330                 335

tca ctt cca att atc ttg act gca gct gaa ctc ttc ttc att cca caa 1056
Ser Leu Pro Ile Ile Leu Thr Ala Ala Glu Leu Phe Phe Ile Pro Gln
            340                 345                 350

cct ctt gtg ttc ctt gtt atc ctt atg att atc tct gac tca gta gaa 1104
Pro Leu Val Phe Leu Val Ile Leu Met Ile Ile Ser Asp Ser Val Glu
        355                 360                 365

tat ggt caa tgg aaa ttg gga cac cgt gat gaa tca ctt act ttg tca 1152
Tyr Gly Gln Trp Lys Leu Gly His Arg Asp Glu Ser Leu Thr Leu Ser
    370                 375                 380

gtt cgt cca ctt gtt gat aaa ctt ggt ggt gcg atg tca aac tgg ctt 1200
Val Arg Pro Leu Val Asp Lys Leu Gly Gly Ala Met Ser Asn Trp Leu
385                 390                 395                 400

gtt tct aca att gcc gta gct gcc ggt atg aca aca ggt gcc tca gca 1248
Val Ser Thr Ile Ala Val Ala Ala Gly Met Thr Thr Gly Ala Ser Ala
                405                 410                 415

tca aca att aca aca cat caa cag tct att ttt aag ctt agc atg ttt 1296
Ser Thr Ile Thr Thr His Gln Gln Ser Ile Phe Lys Leu Ser Met Phe
            420                 425                 430

ggt ttc cca gca gca aca atg ctc atc gga gtc ttc att att gct cgt 1344
Gly Phe Pro Ala Ala Thr Met Leu Ile Gly Val Phe Ile Ile Ala Arg
        435                 440                 445

aaa atc act ttg act gaa gca cgt cac gct gaa att gtt gaa gaa ttg 1392
Lys Ile Thr Leu Thr Glu Ala Arg His Ala Glu Ile Val Glu Glu Leu
    450                 455                 460

gaa tat cgc ttt agt gca gca act tct gaa aag gaa gtt aat gct aat 1440
Glu Tyr Arg Phe Ser Ala Ala Thr Ser Glu Lys Glu Val Asn Ala Asn
465                 470                 475                 480

aac gtt tct ctt gtg aac cct aca act ggt aaa cta gtt gat ctt tca 1488
Asn Val Ser Leu Val Asn Pro Thr Thr Gly Lys Leu Val Asp Leu Ser
                485                 490                 495

agc gtt agt gat aaa cgc ttt gct tca ggt agc cta ggt aaa gga ttt 1536
Ser Val Ser Asp Lys Arg Phe Ala Ser Gly Ser Leu Gly Lys Gly Phe
            500                 505                 510

gca atc aaa cct tca gat ggt aag gtt ttt gcc cca att agt ggt aca 1584
Ala Ile Lys Pro Ser Asp Gly Lys Val Phe Ala Pro Ile Ser Gly Thr
        515                 520                 525

gtt cgt cag att cta cct act cgt cat gcg att ggt att gaa agt gat 1632
Val Arg Gln Ile Leu Pro Thr Arg His Ala Ile Gly Ile Glu Ser Asp
    530                 535                 540

gat ggt gtc gtt gtt ctc gta cac gtt ggt att gga aca gtt aaa ctt 1680
Asp Gly Val Val Val Leu Val His Val Gly Ile Gly Thr Val Lys Leu
545                 550                 555                 560

aaa ggt gaa ggg ttc att agt tac gta aaa caa ggt gaa cgt gtt gaa 1728
Lys Gly Glu Gly Phe Ile Ser Tyr Val Lys Gln Gly Glu Arg Val Glu
                565                 570                 575

gtt gga caa aaa ctt ctt gag ttc tgg tta cca att att gaa caa aat 1776
Val Gly Gln Lys Leu Leu Glu Phe Trp Leu Pro Ile Ile Glu Gln Asn
            580                 585                 590

gga ttg gat gat aca gta ctt gtt att gta act aat tct gaa aaa ttc 1824
Gly Leu Asp Asp Thr Val Leu Val Ile Val Thr Asn Ser Glu Lys Phe
        595                 600                 605

agt tct ttc caa ctt gaa caa aca gtt gga gaa gag gtt gaa gcc tta 1872
Ser Ser Phe Gln Leu Glu Gln Thr Val Gly Glu Glu Val Glu Ala Leu
    610                 615                 620

tct gaa gtt att act ttt aaa aaa gga gaa taa 1905
Ser Glu Val Ile Thr Phe Lys Lys Gly Glu
625                 630

<210> 6

<211>  634
<212>  PRT
<213>  Streptococcus infantarius subsp. infantarius strain CJ18

<400>  6

Met Arg Lys Ser Asn Val Gln Met Lys Ser Arg Leu Ser Tyr Ala Ala
1               5                   10                  15

Gly Ala Phe Gly Asn Asp Val Phe Tyr Ala Thr Leu Ser Thr Tyr Phe
            20                  25                  30

Ile Met Phe Val Thr Thr His Leu Phe Asn Thr Gly Asp Pro Lys Gln
        35                  40                  45

Asn Ser His Tyr Val Leu Leu Ile Thr Asn Ile Ile Ser Ile Leu Arg
    50                  55                  60

Ile Leu Glu Val Phe Ile Asp Pro Leu Ile Gly Asn Met Ile Asp Asn
65                  70                  75                  80

Thr His Thr Lys Tyr Gly Lys Phe Lys Pro Trp Val Val Gly Gly Gly
                85                  90                  95

Ile Ile Ser Ser Ile Thr Leu Leu Leu Leu Phe Thr Asp Leu Gly Gly
            100                 105                 110

Leu Asn Lys Thr Asn Pro Phe Leu Tyr Leu Val Leu Phe Gly Ile Ile
        115                 120                 125

Tyr Leu Val Met Asp Val Phe Tyr Ser Ile Lys Asp Ile Gly Phe Trp
    130                 135                 140

Ser Met Ile Pro Ala Leu Ser Phe Asp Ser His Glu Arg Glu Lys Met
145                 150                 155                 160

Ala Thr Phe Ala Arg Ile Gly Ser Thr Ile Gly Ala Asn Ile Val Gly
                165                 170                 175

Val Ala Ile Met Pro Ile Val Leu Phe Phe Ser Met Thr Asn Ser Ser
            180                 185                 190

Gly Ser Gly Asp Lys Ser Gly Trp Phe Trp Phe Ala Phe Ile Val Ala
        195                 200                 205

Leu Ile Gly Val Ile Thr Ser Ile Ala Val Gly Ile Gly Thr Arg Glu
    210                 215                 220

Val Glu Ser Lys Leu Arg Asp Asn Asn Glu Lys Thr Ser Leu Lys Gln

225                 230                 235                 240

Val Phe Lys Val Leu Gly Lys Asn Asp Gln Leu Met Trp Leu Ser Leu
                245                 250                 255

Gly Tyr Trp Phe Tyr Gly Leu Gly Ile Asn Thr Leu Asn Ala Leu Gln
            260                 265                 270

Leu Tyr Tyr Phe Thr Phe Ile Leu Gly Asp Ser Gly Lys Tyr Ser Ile
        275                 280                 285

Leu Tyr Gly Leu Asn Thr Val Val Gly Leu Val Ser Val Ser Leu Phe
    290                 295                 300

Pro Ser Leu Ala Gly Lys Phe Asn Arg Lys Arg Leu Phe Tyr Gly Cys
305                 310                 315                 320

Ile Ala Val Met Ile Gly Gly Ile Gly Ile Phe Ser Ile Ala Gly Thr
                325                 330                 335

Ser Leu Pro Ile Ile Leu Thr Ala Ala Glu Leu Phe Phe Ile Pro Gln
            340                 345                 350

Pro Leu Val Phe Leu Val Ile Leu Met Ile Ile Ser Asp Ser Val Glu
        355                 360                 365

Tyr Gly Gln Trp Lys Leu Gly His Arg Asp Glu Ser Leu Thr Leu Ser
    370                 375                 380

Val Arg Pro Leu Val Asp Lys Leu Gly Gly Ala Met Ser Asn Trp Leu
385                 390                 395                 400

Val Ser Thr Ile Ala Val Ala Ala Gly Met Thr Thr Gly Ala Ser Ala
                405                 410                 415

Ser Thr Ile Thr Thr His Gln Gln Ser Ile Phe Lys Leu Ser Met Phe
            420                 425                 430

Gly Phe Pro Ala Ala Thr Met Leu Ile Gly Val Phe Ile Ile Ala Arg
        435                 440                 445

Lys Ile Thr Leu Thr Glu Ala Arg His Ala Glu Ile Val Glu Glu Leu
    450                 455                 460

Glu Tyr Arg Phe Ser Ala Ala Thr Ser Glu Lys Glu Val Asn Ala Asn
465                 470                 475                 480

Asn Val Ser Leu Val Asn Pro Thr Thr Gly Lys Leu Val Asp Leu Ser
                485                 490                 495

Ser Val Ser Asp Lys Arg Phe Ala Ser Gly Ser Leu Gly Lys Gly Phe
            500                 505                 510

Ala Ile Lys Pro Ser Asp Gly Lys Val Phe Ala Pro Ile Ser Gly Thr
        515                 520                 525

Val Arg Gln Ile Leu Pro Thr Arg His Ala Ile Gly Ile Glu Ser Asp
    530                 535                 540

Asp Gly Val Val Val Leu Val His Val Gly Ile Gly Thr Val Lys Leu
545                 550                 555                 560

Lys Gly Glu Gly Phe Ile Ser Tyr Val Lys Gln Gly Glu Arg Val Glu
                565                 570                 575

Val Gly Gln Lys Leu Leu Glu Phe Trp Leu Pro Ile Ile Glu Gln Asn
            580                 585                 590

Gly Leu Asp Asp Thr Val Leu Val Ile Val Thr Asn Ser Glu Lys Phe
        595                 600                 605

Ser Ser Phe Gln Leu Glu Gln Thr Val Gly Glu Glu Val Glu Ala Leu
    610                 615                 620

Ser Glu Val Ile Thr Phe Lys Lys Gly Glu
625                 630

<210>  7
<211>  3081
<212>  DNA
<213>  Streptococcus thermophilus strain APC151

<220>
<221>  CDS
<222>  (1)..(3081)
<223>  LacZ gene encoding beta-galactosidase

<300>
<308>  GenBank CP019935.1
<309>  2017-03-03
<313>  (463146)..(466226)

<400>  7
atg aac atg act gaa aaa att caa act tat tta aac gat cca aag att        48
Met Asn Met Thr Glu Lys Ile Gln Thr Tyr Leu Asn Asp Pro Lys Ile
1               5                   10                  15

gtt agc gtt aat act gtt gat gct cac tca gat cat aag tat ttt gaa        96
Val Ser Val Asn Thr Val Asp Ala His Ser Asp His Lys Tyr Phe Glu

20                  25                  30

tct ctt gaa gaa ttt tct gaa ggg gag atg aag tta aga caa tct ctt          144
Ser Leu Glu Glu Phe Ser Glu Gly Glu Met Lys Leu Arg Gln Ser Leu
        35                  40                  45

aat gga aaa tgg aaa att cac tat gct cag aat aca aat cag gtt tta          192
Asn Gly Lys Trp Lys Ile His Tyr Ala Gln Asn Thr Asn Gln Val Leu
    50                  55                  60

aaa gac ttt tat aaa aca gaa ttt gat gaa act gat ttg aat ttc atc          240
Lys Asp Phe Tyr Lys Thr Glu Phe Asp Glu Thr Asp Leu Asn Phe Ile
65                  70                  75                  80

aat gta cca ggt cat tta gag ctt caa ggt ttt ggt tct cca caa tat          288
Asn Val Pro Gly His Leu Glu Leu Gln Gly Phe Gly Ser Pro Gln Tyr
                85                  90                  95

gtg aat acc caa tat cct tgg gat ggt aaa gaa ttc ctt cgt cca cct          336
Val Asn Thr Gln Tyr Pro Trp Asp Gly Lys Glu Phe Leu Arg Pro Pro
            100                 105                 110

caa gtt cct caa gaa tca aat gct gtt gca tca tac gtt aaa cat ttt          384
Gln Val Pro Gln Glu Ser Asn Ala Val Ala Ser Tyr Val Lys His Phe
        115                 120                 125

acc ttg aat gat gca tta aaa gat aaa aaa gta ttt atc tca ttc caa          432
Thr Leu Asn Asp Ala Leu Lys Asp Lys Lys Val Phe Ile Ser Phe Gln
    130                 135                 140

ggg gtt gct act tcc atc ttt gta tgg gtc aat ggt aac ttc gta gga          480
Gly Val Ala Thr Ser Ile Phe Val Trp Val Asn Gly Asn Phe Val Gly
145                 150                 155                 160

tac agt gaa gat tca ttt aca cct agt gaa ttt gaa att agt gat tac          528
Tyr Ser Glu Asp Ser Phe Thr Pro Ser Glu Phe Glu Ile Ser Asp Tyr
                165                 170                 175

ctt gtt gaa ggt gat aac aag ttg gcg gta gct gtt tat cgt tac tct          576
Leu Val Glu Gly Asp Asn Lys Leu Ala Val Ala Val Tyr Arg Tyr Ser
            180                 185                 190

aca gca agc tgg ttg gaa gac caa gac ttc tgg aga ctt tac ggt att          624
Thr Ala Ser Trp Leu Glu Asp Gln Asp Phe Trp Arg Leu Tyr Gly Ile
        195                 200                 205

ttt aga gat gtt tac ttg tat gct att cca aaa gtt cac gtt caa gat          672
Phe Arg Asp Val Tyr Leu Tyr Ala Ile Pro Lys Val His Val Gln Asp
    210                 215                 220

ctc ttt gtt aag gga gat tat gat tac caa aca aaa gca ggt caa ttg          720
Leu Phe Val Lys Gly Asp Tyr Asp Tyr Gln Thr Lys Ala Gly Gln Leu
225                 230                 235                 240

gat att gat ttg aag act gtt ggt gat tat gaa gac aag aag att aaa          768
Asp Ile Asp Leu Lys Thr Val Gly Asp Tyr Glu Asp Lys Lys Ile Lys
                245                 250                 255

tat gtt ctt tca gat tat gaa ggc atc gtt aca gaa ggt gat gca tct          816
Tyr Val Leu Ser Asp Tyr Glu Gly Ile Val Thr Glu Gly Asp Ala Ser
            260                 265                 270

gtt aat ggt gac ggt gaa cta tct gta agt ctt gaa aat ctt aaa atc          864

Val Asn Gly Asp Gly Glu Leu Ser Val Ser Leu Glu Asn Leu Lys Ile
        275                 280                 285

aaa cct tgg agt gct gaa agt cct aaa ctt tac gat ttg atc ctt cat         912
Lys Pro Trp Ser Ala Glu Ser Pro Lys Leu Tyr Asp Leu Ile Leu His
    290                 295                 300

gtt ttg gat gat gac caa gtt gtt gaa gtc gtt cca gtt aaa gtt gga         960
Val Leu Asp Asp Asp Gln Val Val Glu Val Val Pro Val Lys Val Gly
305                 310                 315                 320

ttc aga cgc ttt gaa att aaa gat aaa ctt atg ctt ttg aat ggt aag        1008
Phe Arg Arg Phe Glu Ile Lys Asp Lys Leu Met Leu Leu Asn Gly Lys
                325                 330                 335

aga att gtc ttt aaa ggg gtt aac aga cac gaa ttt aac gct aga aca        1056
Arg Ile Val Phe Lys Gly Val Asn Arg His Glu Phe Asn Ala Arg Thr
            340                 345                 350

gga cgt tgt atc act gaa gaa gat atg ctt tgg gat atc aaa gtg atg        1104
Gly Arg Cys Ile Thr Glu Glu Asp Met Leu Trp Asp Ile Lys Val Met
        355                 360                 365

aag caa cat aac atc aat gct gtt cgt act tca cac tat cct aac caa        1152
Lys Gln His Asn Ile Asn Ala Val Arg Thr Ser His Tyr Pro Asn Gln
    370                 375                 380

aca cgt tgg tat gaa ttg tgt gat gaa tat gga ctt tat gtt atc gat        1200
Thr Arg Trp Tyr Glu Leu Cys Asp Glu Tyr Gly Leu Tyr Val Ile Asp
385                 390                 395                 400

gaa gcc aac ctt gaa aca cac ggt aca tgg caa aaa ctt ggt cta tgc        1248
Glu Ala Asn Leu Glu Thr His Gly Thr Trp Gln Lys Leu Gly Leu Cys
                405                 410                 415

gaa cct tca tgg aat atc cca gct agt gaa cca gaa tgg ttg cct gct        1296
Glu Pro Ser Trp Asn Ile Pro Ala Ser Glu Pro Glu Trp Leu Pro Ala
            420                 425                 430

tgt ttg gat cgt gcc aat aac atg ttc caa cgc gat aag aac cac gct        1344
Cys Leu Asp Arg Ala Asn Asn Met Phe Gln Arg Asp Lys Asn His Ala
        435                 440                 445

agt gtt atc att tgg tct tgt ggt aat gaa tca tat gct ggt aaa gat        1392
Ser Val Ile Ile Trp Ser Cys Gly Asn Glu Ser Tyr Ala Gly Lys Asp
    450                 455                 460

att gct gac atg gct gat tac ttc cgt agt gtt gac aat act cgt cca        1440
Ile Ala Asp Met Ala Asp Tyr Phe Arg Ser Val Asp Asn Thr Arg Pro
465                 470                 475                 480

gtt cac tat gaa ggt gtt gca tgg tgt cgt gag ttt gat tac att aca        1488
Val His Tyr Glu Gly Val Ala Trp Cys Arg Glu Phe Asp Tyr Ile Thr
                485                 490                 495

gac atc gaa agt cgt atg tat gcg aaa cca gct gat atc gaa gaa tac        1536
Asp Ile Glu Ser Arg Met Tyr Ala Lys Pro Ala Asp Ile Glu Glu Tyr
            500                 505                 510

ctc aca act ggt aaa cta gtt gat ctt tca agc gtt agt gat aaa cac        1584
Leu Thr Thr Gly Lys Leu Val Asp Leu Ser Ser Val Ser Asp Lys His
        515                 520                 525

ttt gct tca ggt aac cta act aac aaa cct caa aaa cct tat att tca 1632
Phe Ala Ser Gly Asn Leu Thr Asn Lys Pro Gln Lys Pro Tyr Ile Ser
    530                 535                 540

tgt gaa tac atg cac aca atg ggt aac tct ggt ggt gga ttg caa ctc 1680
Cys Glu Tyr Met His Thr Met Gly Asn Ser Gly Gly Gly Leu Gln Leu
545                 550                 555                 560

tac act gac tta gag aaa tat cca gaa tac caa ggt gga ttt att tgg 1728
Tyr Thr Asp Leu Glu Lys Tyr Pro Glu Tyr Gln Gly Gly Phe Ile Trp
                565                 570                 575

gac ttc att gac caa gct att tac aaa aca ctt cca aat ggt agc gaa 1776
Asp Phe Ile Asp Gln Ala Ile Tyr Lys Thr Leu Pro Asn Gly Ser Glu
            580                 585                 590

ttc cta tca tat ggt ggt gac tgg cat gat aga cct tct gac tac gaa 1824
Phe Leu Ser Tyr Gly Gly Asp Trp His Asp Arg Pro Ser Asp Tyr Glu
        595                 600                 605

ttt tgt gga aat ggt atc gtc ttt gca gat cgt acc cta act cca aaa 1872
Phe Cys Gly Asn Gly Ile Val Phe Ala Asp Arg Thr Leu Thr Pro Lys
    610                 615                 620

ctt caa aca gtt aaa cat ctt tac tct aat att aag att gct gtt gat 1920
Leu Gln Thr Val Lys His Leu Tyr Ser Asn Ile Lys Ile Ala Val Asp
625                 630                 635                 640

gaa aaa tca gta act atc aag aat gat aat ctc ttc gaa gat ctt tct 1968
Glu Lys Ser Val Thr Ile Lys Asn Asp Asn Leu Phe Glu Asp Leu Ser
                645                 650                 655

gct tat act ttc cta gct aga gtt tac gaa gat ggt aga aaa gtt agt 2016
Ala Tyr Thr Phe Leu Ala Arg Val Tyr Glu Asp Gly Arg Lys Val Ser
            660                 665                 670

gaa agt gaa tat cac ttt gat gtt aaa cca ggc gaa gaa gca aca ttc 2064
Glu Ser Glu Tyr His Phe Asp Val Lys Pro Gly Glu Glu Ala Thr Phe
        675                 680                 685

cca gtt aac ttt gta gtc gag gct tca aat tct gaa caa att tac gaa 2112
Pro Val Asn Phe Val Val Glu Ala Ser Asn Ser Glu Gln Ile Tyr Glu
    690                 695                 700

gtt gct tgt gtt ctg agg gaa gca act gaa tgg gct cct aaa ggt cat 2160
Val Ala Cys Val Leu Arg Glu Ala Thr Glu Trp Ala Pro Lys Gly His
705                 710                 715                 720

gaa att gtt cgt ggt caa tat gtt gtt gaa aag att agc act gaa aca 2208
Glu Ile Val Arg Gly Gln Tyr Val Val Glu Lys Ile Ser Thr Glu Thr
                725                 730                 735

cca gtt aaa gca cct ttg aat gtt gtt gaa ggc gac ttc aac atc ggt 2256
Pro Val Lys Ala Pro Leu Asn Val Val Glu Gly Asp Phe Asn Ile Gly
            740                 745                 750

att caa gga caa aac ttc tca atc ttg ctt tca cgt gca caa aat act 2304
Ile Gln Gly Gln Asn Phe Ser Ile Leu Leu Ser Arg Ala Gln Asn Thr
        755                 760                 765

tta gta tct gct aag tat aat ggt gtt gaa ttc att gag aaa ggt cct 2352
Leu Val Ser Ala Lys Tyr Asn Gly Val Glu Phe Ile Glu Lys Gly Pro
    770                 775                 780

aaa ctt agc ttc act cgt gct tac act gac aac gat cgt ggt gct gga 2400
Lys Leu Ser Phe Thr Arg Ala Tyr Thr Asp Asn Asp Arg Gly Ala Gly
785                 790                 795                 800

tat cca ttc gaa atg gca ggc tgg aag gtt gct gga aac tat agt aaa 2448
Tyr Pro Phe Glu Met Ala Gly Trp Lys Val Ala Gly Asn Tyr Ser Lys
                805                 810                 815

gtt aca gat act caa att caa atc gaa gac gac tct gtt aaa gtg act 2496
Val Thr Asp Thr Gln Ile Gln Ile Glu Asp Asp Ser Val Lys Val Thr
            820                 825                 830

tat gtt cat gaa ttg cca ggc ttg tct gat gtc gaa gtt aag gta act 2544
Tyr Val His Glu Leu Pro Gly Leu Ser Asp Val Glu Val Lys Val Thr
        835                 840                 845

tat caa gtt gat tac aag ggt cga atc ttt gtt act gca aac tat gat 2592
Tyr Gln Val Asp Tyr Lys Gly Arg Ile Phe Val Thr Ala Asn Tyr Asp
    850                 855                 860

ggt aaa gca ggt ttg cca aac ttc cct gaa ttt ggt cta gaa ttt gct 2640
Gly Lys Ala Gly Leu Pro Asn Phe Pro Glu Phe Gly Leu Glu Phe Ala
865                 870                 875                 880

atc ggt tca caa ttt aca aac ctt agc tat tat gga tac ggt gca gaa 2688
Ile Gly Ser Gln Phe Thr Asn Leu Ser Tyr Tyr Gly Tyr Gly Ala Glu
                885                 890                 895

gaa agc tac cgt gat aaa ctt cct ggt gcc tat ctt ggt cga tat gaa 2736
Glu Ser Tyr Arg Asp Lys Leu Pro Gly Ala Tyr Leu Gly Arg Tyr Glu
            900                 905                 910

aca tct gtt gaa aag aca ttt gct cca tat cta atg cca caa gaa tct 2784
Thr Ser Val Glu Lys Thr Phe Ala Pro Tyr Leu Met Pro Gln Glu Ser
        915                 920                 925

ggt aat cac tat ggt act cgt gaa ttc aca gta tct gat gat aac cat 2832
Gly Asn His Tyr Gly Thr Arg Glu Phe Thr Val Ser Asp Asp Asn His
    930                 935                 940

aat ggt ctt aaa ttc acc gca ctt aat aaa gca ttc gaa ttc agt gct 2880
Asn Gly Leu Lys Phe Thr Ala Leu Asn Lys Ala Phe Glu Phe Ser Ala
945                 950                 955                 960

ttg cgt aac agt act gaa caa att gaa aat gct cgt cac caa tat gag 2928
Leu Arg Asn Ser Thr Glu Gln Ile Glu Asn Ala Arg His Gln Tyr Glu
                965                 970                 975

ttg caa gaa tct gat gct aca tgg att aaa gtt ctt gct gct caa atg 2976
Leu Gln Glu Ser Asp Ala Thr Trp Ile Lys Val Leu Ala Ala Gln Met
            980                 985                 990

ggt gta ggt ggt gac gac aca tgg ggt gct cca gtt cat gac gaa ttc 3024
Gly Val Gly Gly Asp Asp Thr Trp Gly Ala Pro Val His Asp Glu Phe
        995                 1000                1005

ttg ctt agc tca gca gat agc tat caa tta agc ttc atg att gaa 3069
Leu Leu Ser Ser Ala Asp Ser Tyr Gln Leu Ser Phe Met Ile Glu
    1010                1015                1020

cca cta aat tag 3081
Pro Leu Asn

    1025

<210> 8
<211> 1026
<212> PRT
<213> Streptococcus thermophilus strain APC151

<400> 8

Met Asn Met Thr Glu Lys Ile Gln Thr Tyr Leu Asn Asp Pro Lys Ile
1               5                   10                  15

Val Ser Val Asn Thr Val Asp Ala His Ser Asp His Lys Tyr Phe Glu
            20                  25                  30

Ser Leu Glu Glu Phe Ser Glu Gly Glu Met Lys Leu Arg Gln Ser Leu
        35                  40                  45

Asn Gly Lys Trp Lys Ile His Tyr Ala Gln Asn Thr Asn Gln Val Leu
    50                  55                  60

Lys Asp Phe Tyr Lys Thr Glu Phe Asp Glu Thr Asp Leu Asn Phe Ile
65                  70                  75                  80

Asn Val Pro Gly His Leu Glu Leu Gln Gly Phe Gly Ser Pro Gln Tyr
                85                  90                  95

Val Asn Thr Gln Tyr Pro Trp Asp Gly Lys Glu Phe Leu Arg Pro Pro
            100                 105                 110

Gln Val Pro Gln Glu Ser Asn Ala Val Ala Ser Tyr Val Lys His Phe
        115                 120                 125

Thr Leu Asn Asp Ala Leu Lys Asp Lys Lys Val Phe Ile Ser Phe Gln
    130                 135                 140

Gly Val Ala Thr Ser Ile Phe Val Trp Val Asn Gly Asn Phe Val Gly
145                 150                 155                 160

Tyr Ser Glu Asp Ser Phe Thr Pro Ser Glu Phe Glu Ile Ser Asp Tyr
                165                 170                 175

Leu Val Glu Gly Asp Asn Lys Leu Ala Val Ala Val Tyr Arg Tyr Ser
            180                 185                 190

Thr Ala Ser Trp Leu Glu Asp Gln Asp Phe Trp Arg Leu Tyr Gly Ile
        195                 200                 205

Phe Arg Asp Val Tyr Leu Tyr Ala Ile Pro Lys Val His Val Gln Asp

210                 215                 220

Leu Phe Val Lys Gly Asp Tyr Asp Tyr Gln Thr Lys Ala Gly Gln Leu
225                 230                 235                 240

Asp Ile Asp Leu Lys Thr Val Gly Asp Tyr Glu Asp Lys Lys Ile Lys
                245                 250                 255

Tyr Val Leu Ser Asp Tyr Glu Gly Ile Val Thr Glu Gly Asp Ala Ser
            260                 265                 270

Val Asn Gly Asp Gly Glu Leu Ser Val Ser Leu Glu Asn Leu Lys Ile
        275                 280                 285

Lys Pro Trp Ser Ala Glu Ser Pro Lys Leu Tyr Asp Leu Ile Leu His
    290                 295                 300

Val Leu Asp Asp Asp Gln Val Val Glu Val Val Pro Val Lys Val Gly
305                 310                 315                 320

Phe Arg Arg Phe Glu Ile Lys Asp Lys Leu Met Leu Leu Asn Gly Lys
                325                 330                 335

Arg Ile Val Phe Lys Gly Val Asn Arg His Glu Phe Asn Ala Arg Thr
            340                 345                 350

Gly Arg Cys Ile Thr Glu Glu Asp Met Leu Trp Asp Ile Lys Val Met
        355                 360                 365

Lys Gln His Asn Ile Asn Ala Val Arg Thr Ser His Tyr Pro Asn Gln
    370                 375                 380

Thr Arg Trp Tyr Glu Leu Cys Asp Glu Tyr Gly Leu Tyr Val Ile Asp
385                 390                 395                 400

Glu Ala Asn Leu Glu Thr His Gly Thr Trp Gln Lys Leu Gly Leu Cys
                405                 410                 415

Glu Pro Ser Trp Asn Ile Pro Ala Ser Glu Pro Glu Trp Leu Pro Ala
            420                 425                 430

Cys Leu Asp Arg Ala Asn Asn Met Phe Gln Arg Asp Lys Asn His Ala
        435                 440                 445

Ser Val Ile Ile Trp Ser Cys Gly Asn Glu Ser Tyr Ala Gly Lys Asp
    450                 455                 460

Ile Ala Asp Met Ala Asp Tyr Phe Arg Ser Val Asp Asn Thr Arg Pro
465                 470                 475                 480

Val His Tyr Glu Gly Val Ala Trp Cys Arg Glu Phe Asp Tyr Ile Thr
                485                 490                 495

Asp Ile Glu Ser Arg Met Tyr Ala Lys Pro Ala Asp Ile Glu Glu Tyr
            500                 505                 510

Leu Thr Thr Gly Lys Leu Val Asp Leu Ser Ser Val Ser Asp Lys His
        515                 520                 525

Phe Ala Ser Gly Asn Leu Thr Asn Lys Pro Gln Lys Pro Tyr Ile Ser
    530                 535                 540

Cys Glu Tyr Met His Thr Met Gly Asn Ser Gly Gly Gly Leu Gln Leu
545                 550                 555                 560

Tyr Thr Asp Leu Glu Lys Tyr Pro Glu Tyr Gln Gly Gly Phe Ile Trp
                565                 570                 575

Asp Phe Ile Asp Gln Ala Ile Tyr Lys Thr Leu Pro Asn Gly Ser Glu
            580                 585                 590

Phe Leu Ser Tyr Gly Gly Asp Trp His Asp Arg Pro Ser Asp Tyr Glu
        595                 600                 605

Phe Cys Gly Asn Gly Ile Val Phe Ala Asp Arg Thr Leu Thr Pro Lys
    610                 615                 620

Leu Gln Thr Val Lys His Leu Tyr Ser Asn Ile Lys Ile Ala Val Asp
625                 630                 635                 640

Glu Lys Ser Val Thr Ile Lys Asn Asp Asn Leu Phe Glu Asp Leu Ser
                645                 650                 655

Ala Tyr Thr Phe Leu Ala Arg Val Tyr Glu Asp Gly Arg Lys Val Ser
            660                 665                 670

Glu Ser Glu Tyr His Phe Asp Val Lys Pro Gly Glu Glu Ala Thr Phe
        675                 680                 685

Pro Val Asn Phe Val Val Glu Ala Ser Asn Ser Glu Gln Ile Tyr Glu
    690                 695                 700

Val Ala Cys Val Leu Arg Glu Ala Thr Glu Trp Ala Pro Lys Gly His
705                 710                 715                 720

Glu Ile Val Arg Gly Gln Tyr Val Val Glu Lys Ile Ser Thr Glu Thr
                725                 730                 735

Pro Val Lys Ala Pro Leu Asn Val Val Glu Gly Asp Phe Asn Ile Gly
            740                 745                 750

Ile Gln Gly Gln Asn Phe Ser Ile Leu Leu Ser Arg Ala Gln Asn Thr
        755                 760                 765

Leu Val Ser Ala Lys Tyr Asn Gly Val Glu Phe Ile Glu Lys Gly Pro
    770                 775                 780

Lys Leu Ser Phe Thr Arg Ala Tyr Thr Asp Asn Asp Arg Gly Ala Gly
785                 790                 795                 800

Tyr Pro Phe Glu Met Ala Gly Trp Lys Val Ala Gly Asn Tyr Ser Lys
                805                 810                 815

Val Thr Asp Thr Gln Ile Gln Ile Glu Asp Asp Ser Val Lys Val Thr
            820                 825                 830

Tyr Val His Glu Leu Pro Gly Leu Ser Asp Val Glu Val Lys Val Thr
        835                 840                 845

Tyr Gln Val Asp Tyr Lys Gly Arg Ile Phe Val Thr Ala Asn Tyr Asp
    850                 855                 860

Gly Lys Ala Gly Leu Pro Asn Phe Pro Glu Phe Gly Leu Glu Phe Ala
865                 870                 875                 880

Ile Gly Ser Gln Phe Thr Asn Leu Ser Tyr Tyr Gly Tyr Gly Ala Glu
                885                 890                 895

Glu Ser Tyr Arg Asp Lys Leu Pro Gly Ala Tyr Leu Gly Arg Tyr Glu
            900                 905                 910

Thr Ser Val Glu Lys Thr Phe Ala Pro Tyr Leu Met Pro Gln Glu Ser
        915                 920                 925

Gly Asn His Tyr Gly Thr Arg Glu Phe Thr Val Ser Asp Asp Asn His
    930                 935                 940

Asn Gly Leu Lys Phe Thr Ala Leu Asn Lys Ala Phe Glu Phe Ser Ala
945                 950                 955                 960

Leu Arg Asn Ser Thr Glu Gln Ile Glu Asn Ala Arg His Gln Tyr Glu
                965                 970                 975

Leu Gln Glu Ser Asp Ala Thr Trp Ile Lys Val Leu Ala Ala Gln Met
            980                 985                 990

Gly Val Gly Gly Asp Asp Thr Trp  Gly Ala Pro Val His  Asp Glu Phe
        995                 1000                1005

Leu Leu  Ser Ser Ala Asp Ser  Tyr Gln Leu Ser Phe  Met Ile Glu
    1010                1015                1020

Pro Leu  Asn
    1025

<210>  9
<211>  3081
<212>  DNA
<213>  Streptococcus infantarius subsp. infantarius CJ18

<220>
<221>  CDS
<222>  (1)..(3081)
<223>  LacZ gene encoding beta-galactosidase

<300>
<308>  GenBank CP003295.1
<309>  2014-01-31
<313>  (956358)..(959438)

<400>  9
gtg aaa aca att gaa aaa att caa act tac tta aac gat cca aag att        48
Val Lys Thr Ile Glu Lys Ile Gln Thr Tyr Leu Asn Asp Pro Lys Ile
1               5                   10                  15

gtt agc gtt aat aca gtt gac gct cac tca gat cat aag tat ttt gaa        96
Val Ser Val Asn Thr Val Asp Ala His Ser Asp His Lys Tyr Phe Glu
            20                  25                  30

tca ctt gca gaa ttt tct gaa gga gag atg aag tta aga caa tct ctc       144
Ser Leu Ala Glu Phe Ser Glu Gly Glu Met Lys Leu Arg Gln Ser Leu
        35                  40                  45

aat gga gaa tgg aaa att cac tat gct cag aat aca aat caa gtt tta       192
Asn Gly Glu Trp Lys Ile His Tyr Ala Gln Asn Thr Asn Gln Val Leu
    50                  55                  60

aaa gac ttt tat aaa aca gaa ttt gat gaa act gac ttg aat ttc atc       240
Lys Asp Phe Tyr Lys Thr Glu Phe Asp Glu Thr Asp Leu Asn Phe Ile
65                  70                  75                  80

aat gta cca ggt cat ttg gaa ctt caa ggt ttt ggt tct cca caa tat       288
Asn Val Pro Gly His Leu Glu Leu Gln Gly Phe Gly Ser Pro Gln Tyr
                85                  90                  95

gtg aat gtc caa tat cct tgg gat ggt aaa gaa ttc ctt cgc cca cct       336
Val Asn Val Gln Tyr Pro Trp Asp Gly Lys Glu Phe Leu Arg Pro Pro
            100                 105                 110

caa att cct caa gaa tca aat gct gtt gca tca tac gtt aaa cat ttt 384
Gln Ile Pro Gln Glu Ser Asn Ala Val Ala Ser Tyr Val Lys His Phe
        115                 120                 125

acc ttg aat gat gca tta aaa gat aaa aaa gta ttt atc tca ttc caa 432
Thr Leu Asn Asp Ala Leu Lys Asp Lys Lys Val Phe Ile Ser Phe Gln
    130                 135                 140

ggg gtt gcc act tcc atc ttt gta tgg gtc aat ggt aac ttc gta gga 480
Gly Val Ala Thr Ser Ile Phe Val Trp Val Asn Gly Asn Phe Val Gly
145                 150                 155                 160

tac agt gaa gat tca ttt aca cct agt gaa ttt gaa att agt gat tac 528
Tyr Ser Glu Asp Ser Phe Thr Pro Ser Glu Phe Glu Ile Ser Asp Tyr
                165                 170                 175

ctt gtt gaa ggt gat aac aag ttg gcg gta gct gtt tat cgt tac tct 576
Leu Val Glu Gly Asp Asn Lys Leu Ala Val Ala Val Tyr Arg Tyr Ser
            180                 185                 190

aca gca agc tgg ttg gaa gac caa gac ttc tgg aga ctg tac ggt att 624
Thr Ala Ser Trp Leu Glu Asp Gln Asp Phe Trp Arg Leu Tyr Gly Ile
        195                 200                 205

ttt aga gat gtt tac ttg tac gct att cca aaa gtt cac gtt caa gat 672
Phe Arg Asp Val Tyr Leu Tyr Ala Ile Pro Lys Val His Val Gln Asp
    210                 215                 220

ctc ttt gtt aag gga gat tat gat tac caa aca aaa gca ggt caa ttg 720
Leu Phe Val Lys Gly Asp Tyr Asp Tyr Gln Thr Lys Ala Gly Gln Leu
225                 230                 235                 240

gat att gat ttg aag gct gtt ggt gat tac gaa gat aag aaa att aaa 768
Asp Ile Asp Leu Lys Ala Val Gly Asp Tyr Glu Asp Lys Lys Ile Lys
                245                 250                 255

tat gtt ctt aca gat cat gaa ggt gtg gtt aca gaa ggt gat gca tct 816
Tyr Val Leu Thr Asp His Glu Gly Val Val Thr Glu Gly Asp Ala Ser
            260                 265                 270

gtt aat gct gac ggt gaa cta tct gta agt ctt gaa gat ttg caa atc 864
Val Asn Ala Asp Gly Glu Leu Ser Val Ser Leu Glu Asp Leu Gln Ile
        275                 280                 285

aaa cct tgg agt gct gaa agt cct aaa ctt tac aat ttg acc ctt cat 912
Lys Pro Trp Ser Ala Glu Ser Pro Lys Leu Tyr Asn Leu Thr Leu His
    290                 295                 300

gtt ttg gat gat gac caa gtt gtt gaa gtt gtt cca gtt aaa gtt gga 960
Val Leu Asp Asp Asp Gln Val Val Glu Val Val Pro Val Lys Val Gly
305                 310                 315                 320

ttt aga cgc ttt gaa att aaa gat aaa ctc atg ctt ttg aat ggt aag 1008
Phe Arg Arg Phe Glu Ile Lys Asp Lys Leu Met Leu Leu Asn Gly Lys
                325                 330                 335

aga att gtc ttt aaa ggg gtt aac aga cac gaa ttt aac gct aga aca 1056
Arg Ile Val Phe Lys Gly Val Asn Arg His Glu Phe Asn Ala Arg Thr
            340                 345                 350

gga cgt tgt atc act gaa gaa gat atg ctt tgg gat atc aaa gtg atg 1104
Gly Arg Cys Ile Thr Glu Glu Asp Met Leu Trp Asp Ile Lys Val Met
        355                 360                 365

aaa caa cat aac atc aat gct gtt cgt act tct cac tat cct aac caa     1152
Lys Gln His Asn Ile Asn Ala Val Arg Thr Ser His Tyr Pro Asn Gln
    370                 375                 380

aca cgt tgg tat gaa ttg tgt gat gaa tat gga ctt tat gtt atc gat     1200
Thr Arg Trp Tyr Glu Leu Cys Asp Glu Tyr Gly Leu Tyr Val Ile Asp
385                 390                 395                 400

gaa gcc aac ctt gaa aca cac ggt aca tgg caa aaa ctt aat cta tgc     1248
Glu Ala Asn Leu Glu Thr His Gly Thr Trp Gln Lys Leu Asn Leu Cys
                405                 410                 415

gaa cct tca tgg aat atc cca gct agt gaa cca gaa tgg ttg cct gct     1296
Glu Pro Ser Trp Asn Ile Pro Ala Ser Glu Pro Glu Trp Leu Pro Ala
            420                 425                 430

tgt ttg gat cgt gcc aat aac atg ttc caa cgt gat aag aac cac act     1344
Cys Leu Asp Arg Ala Asn Asn Met Phe Gln Arg Asp Lys Asn His Thr
        435                 440                 445

agt gtt atc att tgg tct tgt ggt aat gaa tca tat gct ggt aaa gat     1392
Ser Val Ile Ile Trp Ser Cys Gly Asn Glu Ser Tyr Ala Gly Lys Asp
    450                 455                 460

att gct gac atg gct gat tac ttc cgt agt gtt gac aat act cgt cca     1440
Ile Ala Asp Met Ala Asp Tyr Phe Arg Ser Val Asp Asn Thr Arg Pro
465                 470                 475                 480

gtt cac tat gaa ggt gtt aca tgg tgt cgt gag ttt gat tac att aca     1488
Val His Tyr Glu Gly Val Thr Trp Cys Arg Glu Phe Asp Tyr Ile Thr
                485                 490                 495

gac atc gaa agt cgt atg tat gca aaa cca gct gat atc gaa gaa tac     1536
Asp Ile Glu Ser Arg Met Tyr Ala Lys Pro Ala Asp Ile Glu Glu Tyr
            500                 505                 510

ctc aca act ggt aaa cta gtt gat ctt tca agc ggt agt gat aaa cac     1584
Leu Thr Thr Gly Lys Leu Val Asp Leu Ser Ser Gly Ser Asp Lys His
        515                 520                 525

ttt gct tca ggt aaa cta act aac agt cct caa aaa cct tat att tca     1632
Phe Ala Ser Gly Lys Leu Thr Asn Ser Pro Gln Lys Pro Tyr Ile Ser
    530                 535                 540

tgt gaa tac atg cac aca atg ggt aac tct ggt ggt gga ttg gaa ctc     1680
Cys Glu Tyr Met His Thr Met Gly Asn Ser Gly Gly Gly Leu Glu Leu
545                 550                 555                 560

tac act gac tta gag aaa tat cca gaa tac caa ggt gga ttt att tgg     1728
Tyr Thr Asp Leu Glu Lys Tyr Pro Glu Tyr Gln Gly Gly Phe Ile Trp
                565                 570                 575

gac ttc att gac caa gct att tac aaa aca ctt cca aat ggt agc gaa     1776
Asp Phe Ile Asp Gln Ala Ile Tyr Lys Thr Leu Pro Asn Gly Ser Glu
            580                 585                 590

ttc cta tca tat gga ggt gac tgg cat gat aga cct tct gac tac gaa     1824
Phe Leu Ser Tyr Gly Gly Asp Trp His Asp Arg Pro Ser Asp Tyr Glu
        595                 600                 605

ttt tgt gga aat ggt att gtc ttt gca gat cgt acc cta act cca aaa     1872
Phe Cys Gly Asn Gly Ile Val Phe Ala Asp Arg Thr Leu Thr Pro Lys

610                 615                 620

ctt caa aca gtt aaa cat ctt tac tct aat att aag att gct gtt gat      1920
Leu Gln Thr Val Lys His Leu Tyr Ser Asn Ile Lys Ile Ala Val Asp
625                 630                 635                 640

gaa aaa tca gta act atc aag aat gat aat gtc ttc gag gat ctt tct      1968
Glu Lys Ser Val Thr Ile Lys Asn Asp Asn Val Phe Glu Asp Leu Ser
                645                 650                 655

gct tat act ttc cta gct aga gtt tac gaa gat ggt aga aaa gtt agt      2016
Ala Tyr Thr Phe Leu Ala Arg Val Tyr Glu Asp Gly Arg Lys Val Ser
            660                 665                 670

gaa agt gag tat cac ttt gat gtt aaa cca ggc gaa gaa gca aca ttc      2064
Glu Ser Glu Tyr His Phe Asp Val Lys Pro Gly Glu Glu Ala Thr Phe
        675                 680                 685

cca gtt aac ttt gta gtc gag gct tca aat tct gaa caa att tac gaa      2112
Pro Val Asn Phe Val Val Glu Ala Ser Asn Ser Glu Gln Ile Tyr Glu
    690                 695                 700

gtt gct tgt gtt ctg agg gaa gca act gaa tgg gct cct aaa ggt cac      2160
Val Ala Cys Val Leu Arg Glu Ala Thr Glu Trp Ala Pro Lys Gly His
705                 710                 715                 720

gaa att gtt cgt ggt caa tat gtt gtt gaa aag att agc act gaa aca      2208
Glu Ile Val Arg Gly Gln Tyr Val Val Glu Lys Ile Ser Thr Glu Thr
                725                 730                 735

cca gtt aaa gca cct ttg aat gtt gtt gaa ggc gac ttc aac atc ggt      2256
Pro Val Lys Ala Pro Leu Asn Val Val Glu Gly Asp Phe Asn Ile Gly
            740                 745                 750

att caa gga caa aac ttc tca atc ttg ctt tca cgt gca caa aat act      2304
Ile Gln Gly Gln Asn Phe Ser Ile Leu Leu Ser Arg Ala Gln Asn Thr
        755                 760                 765

tta gta tct gct aag tat aat ggt gtt gaa ttc att gag aaa ggt cct      2352
Leu Val Ser Ala Lys Tyr Asn Gly Val Glu Phe Ile Glu Lys Gly Pro
    770                 775                 780

aaa ctt aac ttc act cgt gct tac act gac aac gat cgt ggt gct gga      2400
Lys Leu Asn Phe Thr Arg Ala Tyr Thr Asp Asn Asp Arg Gly Ala Gly
785                 790                 795                 800

tat cca ttc gaa atg gca ggc tgg aag gtt gct gga aac tat agt aaa      2448
Tyr Pro Phe Glu Met Ala Gly Trp Lys Val Ala Gly Asn Tyr Ser Lys
                805                 810                 815

gtt aca gat act caa att caa atc gaa gac gac tct gtt aaa gtg act      2496
Val Thr Asp Thr Gln Ile Gln Ile Glu Asp Asp Ser Val Lys Val Thr
            820                 825                 830

tat gtt cat gaa ttg cca ggc ttg tct gat gtc gaa gtt aag gta act      2544
Tyr Val His Glu Leu Pro Gly Leu Ser Asp Val Glu Val Lys Val Thr
        835                 840                 845

tat caa gtt gac tac aag ggt cga atc ttt gtt act gca aac tat gat      2592
Tyr Gln Val Asp Tyr Lys Gly Arg Ile Phe Val Thr Ala Asn Tyr Asp
    850                 855                 860

ggt aaa gca ggt ttg cca aac ttc cct gaa ttt ggt cta gaa ttt gct      2640

Gly Lys Ala Gly Leu Pro Asn Phe Pro Glu Phe Gly Leu Glu Phe Ala
865                 870                 875                 880

atc ggt tca caa ttt aca aac ctt agc tat tat gga tac ggt gca gaa       2688
Ile Gly Ser Gln Phe Thr Asn Leu Ser Tyr Tyr Gly Tyr Gly Ala Glu
                885                 890                 895

gaa agc tac cgt gat aaa ctt cct ggt gcc tat ctt ggt cga tat gaa       2736
Glu Ser Tyr Arg Asp Lys Leu Pro Gly Ala Tyr Leu Gly Arg Tyr Glu
            900                 905                 910

aca tct gtt gaa aag aca ttt gct cca tat cta atg cca caa gaa tct       2784
Thr Ser Val Glu Lys Thr Phe Ala Pro Tyr Leu Met Pro Gln Glu Ser
        915                 920                 925

ggc aat cac tat ggt act cgt gaa ttc aca gta tct gat gat aac cat       2832
Gly Asn His Tyr Gly Thr Arg Glu Phe Thr Val Ser Asp Asp Asn His
    930                 935                 940

aat ggt ctt aaa ttc acc gcc ctt aat aaa gca ttc gaa ttc agt gct       2880
Asn Gly Leu Lys Phe Thr Ala Leu Asn Lys Ala Phe Glu Phe Ser Ala
945                 950                 955                 960

ttg cgt aac agt act gaa caa att gaa aat gct cgt cac caa tat gag       2928
Leu Arg Asn Ser Thr Glu Gln Ile Glu Asn Ala Arg His Gln Tyr Glu
                965                 970                 975

ttg caa gaa tct gat gct aca tgg atc aaa gtt ctt gct gct caa atg       2976
Leu Gln Glu Ser Asp Ala Thr Trp Ile Lys Val Leu Ala Ala Gln Met
            980                 985                 990

ggt gta ggt ggt gac gac tca tgg  ggt gct cca gtt cat  gat gaa ttc       3024
Gly Val Gly Gly Asp Asp Ser Trp  Gly Ala Pro Val His  Asp Glu Phe
        995                 1000                1005

ttg gtt  agc tca gca gat agc  tat caa ttg agc ttc  atg att gaa       3069
Leu Val  Ser Ser Ala Asp Ser  Tyr Gln Leu Ser Phe  Met Ile Glu
    1010                1015                1020

cca ctc  aac taa                                                      3081
Pro Leu  Asn
    1025


<210>  10
<211>  1026
<212>  PRT
<213>  Streptococcus infantarius subsp. infantarius CJ18

<400>  10

Val Lys Thr Ile Glu Lys Ile Gln Thr Tyr Leu Asn Asp Pro Lys Ile
1               5                   10                  15


Val Ser Val Asn Thr Val Asp Ala His Ser Asp His Lys Tyr Phe Glu
            20                  25                  30


Ser Leu Ala Glu Phe Ser Glu Gly Glu Met Lys Leu Arg Gln Ser Leu
        35                  40                  45

Asn Gly Glu Trp Lys Ile His Tyr Ala Gln Asn Thr Asn Gln Val Leu
    50                  55                  60

Lys Asp Phe Tyr Lys Thr Glu Phe Asp Glu Thr Asp Leu Asn Phe Ile
65                  70                  75                  80

Asn Val Pro Gly His Leu Glu Leu Gln Gly Phe Gly Ser Pro Gln Tyr
                85                  90                  95

Val Asn Val Gln Tyr Pro Trp Asp Gly Lys Glu Phe Leu Arg Pro Pro
            100                 105                 110

Gln Ile Pro Gln Glu Ser Asn Ala Val Ala Ser Tyr Val Lys His Phe
        115                 120                 125

Thr Leu Asn Asp Ala Leu Lys Asp Lys Lys Val Phe Ile Ser Phe Gln
    130                 135                 140

Gly Val Ala Thr Ser Ile Phe Val Trp Val Asn Gly Asn Phe Val Gly
145                 150                 155                 160

Tyr Ser Glu Asp Ser Phe Thr Pro Ser Glu Phe Glu Ile Ser Asp Tyr
                165                 170                 175

Leu Val Glu Gly Asp Asn Lys Leu Ala Val Ala Val Tyr Arg Tyr Ser
            180                 185                 190

Thr Ala Ser Trp Leu Glu Asp Gln Asp Phe Trp Arg Leu Tyr Gly Ile
        195                 200                 205

Phe Arg Asp Val Tyr Leu Tyr Ala Ile Pro Lys Val His Val Gln Asp
    210                 215                 220

Leu Phe Val Lys Gly Asp Tyr Asp Tyr Gln Thr Lys Ala Gly Gln Leu
225                 230                 235                 240

Asp Ile Asp Leu Lys Ala Val Gly Asp Tyr Glu Asp Lys Lys Ile Lys
                245                 250                 255

Tyr Val Leu Thr Asp His Glu Gly Val Val Thr Glu Gly Asp Ala Ser
            260                 265                 270

Val Asn Ala Asp Gly Glu Leu Ser Val Ser Leu Glu Asp Leu Gln Ile
        275                 280                 285

Lys Pro Trp Ser Ala Glu Ser Pro Lys Leu Tyr Asn Leu Thr Leu His
    290                 295                 300

Val Leu Asp Asp Asp Gln Val Val Glu Val Val Pro Val Lys Val Gly
305                 310                 315                 320

Phe Arg Arg Phe Glu Ile Lys Asp Lys Leu Met Leu Leu Asn Gly Lys
                325                 330                 335

Arg Ile Val Phe Lys Gly Val Asn Arg His Glu Phe Asn Ala Arg Thr
            340                 345                 350

Gly Arg Cys Ile Thr Glu Glu Asp Met Leu Trp Asp Ile Lys Val Met
        355                 360                 365

Lys Gln His Asn Ile Asn Ala Val Arg Thr Ser His Tyr Pro Asn Gln
    370                 375                 380

Thr Arg Trp Tyr Glu Leu Cys Asp Glu Tyr Gly Leu Tyr Val Ile Asp
385                 390                 395                 400

Glu Ala Asn Leu Glu Thr His Gly Thr Trp Gln Lys Leu Asn Leu Cys
                405                 410                 415

Glu Pro Ser Trp Asn Ile Pro Ala Ser Glu Pro Glu Trp Leu Pro Ala
            420                 425                 430

Cys Leu Asp Arg Ala Asn Asn Met Phe Gln Arg Asp Lys Asn His Thr
        435                 440                 445

Ser Val Ile Ile Trp Ser Cys Gly Asn Glu Ser Tyr Ala Gly Lys Asp
    450                 455                 460

Ile Ala Asp Met Ala Asp Tyr Phe Arg Ser Val Asp Asn Thr Arg Pro
465                 470                 475                 480

Val His Tyr Glu Gly Val Thr Trp Cys Arg Glu Phe Asp Tyr Ile Thr
                485                 490                 495

Asp Ile Glu Ser Arg Met Tyr Ala Lys Pro Ala Asp Ile Glu Glu Tyr
            500                 505                 510

Leu Thr Thr Gly Lys Leu Val Asp Leu Ser Ser Gly Ser Asp Lys His
        515                 520                 525

Phe Ala Ser Gly Lys Leu Thr Asn Ser Pro Gln Lys Pro Tyr Ile Ser
    530                 535                 540

Cys Glu Tyr Met His Thr Met Gly Asn Ser Gly Gly Gly Leu Glu Leu
545                 550                 555                 560

Tyr Thr Asp Leu Glu Lys Tyr Pro Glu Tyr Gln Gly Gly Phe Ile Trp
                565                 570                 575

Asp Phe Ile Asp Gln Ala Ile Tyr Lys Thr Leu Pro Asn Gly Ser Glu
            580                 585                 590

Phe Leu Ser Tyr Gly Gly Asp Trp His Asp Arg Pro Ser Asp Tyr Glu
        595                 600                 605

Phe Cys Gly Asn Gly Ile Val Phe Ala Asp Arg Thr Leu Thr Pro Lys
    610                 615                 620

Leu Gln Thr Val Lys His Leu Tyr Ser Asn Ile Lys Ile Ala Val Asp
625                 630                 635                 640

Glu Lys Ser Val Thr Ile Lys Asn Asp Asn Val Phe Glu Asp Leu Ser
                645                 650                 655

Ala Tyr Thr Phe Leu Ala Arg Val Tyr Glu Asp Gly Arg Lys Val Ser
            660                 665                 670

Glu Ser Glu Tyr His Phe Asp Val Lys Pro Gly Glu Glu Ala Thr Phe
        675                 680                 685

Pro Val Asn Phe Val Val Glu Ala Ser Asn Ser Glu Gln Ile Tyr Glu
    690                 695                 700

Val Ala Cys Val Leu Arg Glu Ala Thr Glu Trp Ala Pro Lys Gly His
705                 710                 715                 720

Glu Ile Val Arg Gly Gln Tyr Val Val Glu Lys Ile Ser Thr Glu Thr
                725                 730                 735

Pro Val Lys Ala Pro Leu Asn Val Val Glu Gly Asp Phe Asn Ile Gly
            740                 745                 750

Ile Gln Gly Gln Asn Phe Ser Ile Leu Leu Ser Arg Ala Gln Asn Thr
        755                 760                 765

Leu Val Ser Ala Lys Tyr Asn Gly Val Glu Phe Ile Glu Lys Gly Pro
    770                 775                 780

Lys Leu Asn Phe Thr Arg Ala Tyr Thr Asp Asn Asp Arg Gly Ala Gly
785                 790                 795                 800

Tyr Pro Phe Glu Met Ala Gly Trp Lys Val Ala Gly Asn Tyr Ser Lys

805                 810                 815

Val Thr Asp Thr Gln Ile Gln Ile Glu Asp Asp Ser Val Lys Val Thr
            820                 825                 830

Tyr Val His Glu Leu Pro Gly Leu Ser Asp Val Glu Val Lys Val Thr
        835                 840                 845

Tyr Gln Val Asp Tyr Lys Gly Arg Ile Phe Val Thr Ala Asn Tyr Asp
    850                 855                 860

Gly Lys Ala Gly Leu Pro Asn Phe Pro Glu Phe Gly Leu Glu Phe Ala
865                 870                 875                 880

Ile Gly Ser Gln Phe Thr Asn Leu Ser Tyr Tyr Gly Tyr Gly Ala Glu
                885                 890                 895

Glu Ser Tyr Arg Asp Lys Leu Pro Gly Ala Tyr Leu Gly Arg Tyr Glu
            900                 905                 910

Thr Ser Val Glu Lys Thr Phe Ala Pro Tyr Leu Met Pro Gln Glu Ser
        915                 920                 925

Gly Asn His Tyr Gly Thr Arg Glu Phe Thr Val Ser Asp Asp Asn His
    930                 935                 940

Asn Gly Leu Lys Phe Thr Ala Leu Asn Lys Ala Phe Glu Phe Ser Ala
945                 950                 955                 960

Leu Arg Asn Ser Thr Glu Gln Ile Glu Asn Ala Arg His Gln Tyr Glu
                965                 970                 975

Leu Gln Glu Ser Asp Ala Thr Trp Ile Lys Val Leu Ala Ala Gln Met
            980                 985                 990

Gly Val Gly Gly Asp Asp Ser Trp  Gly Ala Pro Val His  Asp Glu Phe
        995                 1000                1005

Leu Val  Ser Ser Ala Asp Ser  Tyr Gln Leu Ser Phe  Met Ile Glu
    1010                1015                1020

Pro Leu  Asn
    1025

<210>  11
<211>  3018
<212>  DNA
<213>  Streptococcus salivarius CCHSS3

<220>
<221>  CDS
<222>  (1)..(3018)
<223>  LacZ gene endocing beta-galactosidase

<300>
<308>  GenBank FR873481.1
<309>  2015-02-27
<313>  (809610)..(812627)

<400>  11
atg aaa aca act gaa aaa ctt caa act tac tta aac gat cca aag att      48
Met Lys Thr Thr Glu Lys Leu Gln Thr Tyr Leu Asn Asp Pro Lys Ile
1               5                   10                  15

gtt agc gtt aat aca gtt gac gct cac tca gat cat aag tat ttt gaa      96
Val Ser Val Asn Thr Val Asp Ala His Ser Asp His Lys Tyr Phe Glu
            20                  25                  30

tca ctt aca gaa ttt tct gaa gga gag atg aag tta aga caa tct ctt      144
Ser Leu Thr Glu Phe Ser Glu Gly Glu Met Lys Leu Arg Gln Ser Leu
        35                  40                  45

aat gga aaa tgg aaa att cac tat gct cag aat aca aat cag gtt tta      192
Asn Gly Lys Trp Lys Ile His Tyr Ala Gln Asn Thr Asn Gln Val Leu
    50                  55                  60

aaa gac ttt tat aaa aca gaa ttt gat gaa act gat ttg aat ttc atc      240
Lys Asp Phe Tyr Lys Thr Glu Phe Asp Glu Thr Asp Leu Asn Phe Ile
65                  70                  75                  80

aat gta cca ggt cat ttg gaa ctt caa ggt ttt ggt tct cca caa tat      288
Asn Val Pro Gly His Leu Glu Leu Gln Gly Phe Gly Ser Pro Gln Tyr
                85                  90                  95

gtg aat acc caa tat cct tgg gat ggt aaa gaa ttc ctt cgt cca cct      336
Val Asn Thr Gln Tyr Pro Trp Asp Gly Lys Glu Phe Leu Arg Pro Pro
            100                 105                 110

caa gtt cct caa gaa tca aat gct gtt gca tca tac gtt aaa cat ttt      384
Gln Val Pro Gln Glu Ser Asn Ala Val Ala Ser Tyr Val Lys His Phe
        115                 120                 125

acc ttg aat gat gca tta aaa gac aaa aaa gta ttt atc tca ttc caa      432
Thr Leu Asn Asp Ala Leu Lys Asp Lys Lys Val Phe Ile Ser Phe Gln
    130                 135                 140

ggg gtt gcc act tcc atc ttt gta tgg gtc aat ggt aac ttc gta gga      480
Gly Val Ala Thr Ser Ile Phe Val Trp Val Asn Gly Asn Phe Val Gly
145                 150                 155                 160

tac agt gaa gat tca ttt aca cct agt gaa ttt gaa att agt gat tac      528
Tyr Ser Glu Asp Ser Phe Thr Pro Ser Glu Phe Glu Ile Ser Asp Tyr
                165                 170                 175

ctt gtt gga ggt gat aac aag ttg gcg gta gct gtt tat cgt tac tct      576
Leu Val Gly Gly Asp Asn Lys Leu Ala Val Ala Val Tyr Arg Tyr Ser
            180                 185                 190

aca gca agc tgg ttg gaa gac caa gac ttc tgg aga ctt tac ggt att      624
Thr Ala Ser Trp Leu Glu Asp Gln Asp Phe Trp Arg Leu Tyr Gly Ile

195                 200                 205

ttc aga gat gtt tac ttg tac gct att cca aaa gtt cac gtt caa gat      672
Phe Arg Asp Val Tyr Leu Tyr Ala Ile Pro Lys Val His Val Gln Asp
    210                 215                 220

ctc ttt gtt aag gga gat tat gat tac caa aca aaa gca ggt caa ttg      720
Leu Phe Val Lys Gly Asp Tyr Asp Tyr Gln Thr Lys Ala Gly Gln Leu
225                 230                 235                 240

gat att gat ttg aag act gtt ggt gat tac gaa gat aag aaa att aaa      768
Asp Ile Asp Leu Lys Thr Val Gly Asp Tyr Glu Asp Lys Lys Ile Lys
                245                 250                 255

tat gtt ctt tca gat cat gaa ggc atc gtt aaa gaa ggt gat gca tct      816
Tyr Val Leu Ser Asp His Glu Gly Ile Val Lys Glu Gly Asp Ala Ser
            260                 265                 270

gtt aat gct gac ggt gaa cta tct gta agt ctt gaa gat ttg caa atc      864
Val Asn Ala Asp Gly Glu Leu Ser Val Ser Leu Glu Asp Leu Gln Ile
        275                 280                 285

aaa cct tgg agt gct gaa agt cct aaa ctt tac aat ttg acc ctt cat      912
Lys Pro Trp Ser Ala Glu Ser Pro Lys Leu Tyr Asn Leu Thr Leu His
    290                 295                 300

gtt ttg gat gat ggc caa gtt gtt gaa gtc gtt cca gtt aag gtt gga      960
Val Leu Asp Asp Gly Gln Val Val Glu Val Val Pro Val Lys Val Gly
305                 310                 315                 320

ttt aga cac ttc gaa att aaa gat aaa ctc atg ctt ttg aat ggt aag     1008
Phe Arg His Phe Glu Ile Lys Asp Lys Leu Met Leu Leu Asn Gly Lys
                325                 330                 335

aga att gtc ttt aaa ggg gtt aac aga cac gaa ttt aac gct aga aca     1056
Arg Ile Val Phe Lys Gly Val Asn Arg His Glu Phe Asn Ala Arg Thr
            340                 345                 350

gga cgt tgt atc act gaa gaa gat atg ctt tgg gat atc aaa gtg atg     1104
Gly Arg Cys Ile Thr Glu Glu Asp Met Leu Trp Asp Ile Lys Val Met
        355                 360                 365

aaa caa cat aac atc aat gct gtt cgt act tct cac tat cct aac caa     1152
Lys Gln His Asn Ile Asn Ala Val Arg Thr Ser His Tyr Pro Asn Gln
    370                 375                 380

aca cgt tgg tat gaa ttg tgt gat gaa tat gga ctt tat gtt atc gat     1200
Thr Arg Trp Tyr Glu Leu Cys Asp Glu Tyr Gly Leu Tyr Val Ile Asp
385                 390                 395                 400

gaa gcc aac ctt gaa aca cac ggt aca tgg caa aaa ctt ggt cta agc     1248
Glu Ala Asn Leu Glu Thr His Gly Thr Trp Gln Lys Leu Gly Leu Ser
                405                 410                 415

gaa cct tca tgg aat atc cca gct agt gaa cca gaa tgg ttg cct gct     1296
Glu Pro Ser Trp Asn Ile Pro Ala Ser Glu Pro Glu Trp Leu Pro Ala
            420                 425                 430

tgt ttg gat cgt gcc aat aac atg ttc caa cgt gat aag aac cat gct     1344
Cys Leu Asp Arg Ala Asn Asn Met Phe Gln Arg Asp Lys Asn His Ala
        435                 440                 445

agt gtt atc att tgg tct tgt ggt aat gaa tca tat gct ggt aaa gat     1392

Ser Val Ile Ile Trp Ser Cys Gly Asn Glu Ser Tyr Ala Gly Lys Asp
    450                 455                 460

att gct gac atg gct gat tac ttc cgt agt gtt gac aat act cgt cca      1440
Ile Ala Asp Met Ala Asp Tyr Phe Arg Ser Val Asp Asn Thr Arg Pro
465                 470                 475                 480

gtt cac tat gaa ggt gtt aca tgg tgt cgt gaa ttt gat tac att aca      1488
Val His Tyr Glu Gly Val Thr Trp Cys Arg Glu Phe Asp Tyr Ile Thr
                485                 490                 495

gac atc gaa agt cgt atg tat gcg aaa cca gct gat att gaa gaa tac      1536
Asp Ile Glu Ser Arg Met Tyr Ala Lys Pro Ala Asp Ile Glu Glu Tyr
            500                 505                 510

ctc act aac aat cct caa aaa cct tat att tca tgt gaa tac atg cac      1584
Leu Thr Asn Asn Pro Gln Lys Pro Tyr Ile Ser Cys Glu Tyr Met His
        515                 520                 525

aca atg ggt aac tct ggt ggt gga ttg aaa ctc tac act gac tta gag      1632
Thr Met Gly Asn Ser Gly Gly Gly Leu Lys Leu Tyr Thr Asp Leu Glu
    530                 535                 540

aaa tat cca gaa tac caa ggt gga ttt att tgg gac ttc att gac caa      1680
Lys Tyr Pro Glu Tyr Gln Gly Gly Phe Ile Trp Asp Phe Ile Asp Gln
545                 550                 555                 560

gct att tac aaa cca ctt cca aat ggt agc gaa ttc cta tca tat ggt      1728
Ala Ile Tyr Lys Pro Leu Pro Asn Gly Ser Glu Phe Leu Ser Tyr Gly
                565                 570                 575

ggt gac tgg cat gat aga cct tct gac tac gaa ttt tgt gga aat ggt      1776
Gly Asp Trp His Asp Arg Pro Ser Asp Tyr Glu Phe Cys Gly Asn Gly
            580                 585                 590

att gtc ttc gca gat cgt acc cta act cca aaa ctt caa aca gtt aaa      1824
Ile Val Phe Ala Asp Arg Thr Leu Thr Pro Lys Leu Gln Thr Val Lys
        595                 600                 605

cat ctt tac tct aat att aag att gct gtt gat gaa aaa tca gta act      1872
His Leu Tyr Ser Asn Ile Lys Ile Ala Val Asp Glu Lys Ser Val Thr
    610                 615                 620

atc aag aat gat aat gtc ttt gaa gat ctt tct gct tat act ttc cta      1920
Ile Lys Asn Asp Asn Val Phe Glu Asp Leu Ser Ala Tyr Thr Phe Leu
625                 630                 635                 640

gct aga gtt tac gaa gat ggt aga aaa gtt agc gaa agt gaa tat cac      1968
Ala Arg Val Tyr Glu Asp Gly Arg Lys Val Ser Glu Ser Glu Tyr His
                645                 650                 655

ttt gat gtt aaa cca ggt gaa gaa gca aca ttc cca gta gaa ttc gct      2016
Phe Asp Val Lys Pro Gly Glu Glu Ala Thr Phe Pro Val Glu Phe Ala
            660                 665                 670

gtt gga gct tca aat gct gaa cga att tat gaa gtt gct tgt gtt cag      2064
Val Gly Ala Ser Asn Ala Glu Arg Ile Tyr Glu Val Ala Cys Val Gln
        675                 680                 685

aat gaa gca act gaa tgg gct cca aaa gga cat gaa att gtt cgt ggt      2112
Asn Glu Ala Thr Glu Trp Ala Pro Lys Gly His Glu Ile Val Arg Gly
    690                 695                 700

caa tat gtt gct gaa aag att agc act gaa aca cca gtt aaa gca cct 2160
Gln Tyr Val Ala Glu Lys Ile Ser Thr Glu Thr Pro Val Lys Ala Pro
705                 710                 715                 720

ttg aat gtt gtt gaa ggc gac ttc aac atc gga att caa gga caa aac 2208
Leu Asn Val Val Glu Gly Asp Phe Asn Ile Gly Ile Gln Gly Gln Asn
                725                 730                 735

ttc tct atc ttg ctt tca cgt gca caa aat acg cta gta tct gct aag 2256
Phe Ser Ile Leu Leu Ser Arg Ala Gln Asn Thr Leu Val Ser Ala Lys
            740                 745                 750

tat aac ggt gtt gaa ttc att gag aaa ggt cct aaa ctt aac ttt act 2304
Tyr Asn Gly Val Glu Phe Ile Glu Lys Gly Pro Lys Leu Asn Phe Thr
        755                 760                 765

cgt gct tac acc gac aac gat cgt ggt gct gga tat cca ttc gaa atg 2352
Arg Ala Tyr Thr Asp Asn Asp Arg Gly Ala Gly Tyr Pro Phe Glu Met
    770                 775                 780

gca ggc tgg aaa gtt gct gga aac tat agt aag gtt aca gat act caa 2400
Ala Gly Trp Lys Val Ala Gly Asn Tyr Ser Lys Val Thr Asp Thr Gln
785                 790                 795                 800

att caa att gaa gat gac tct gtt aaa gtg act tat gtt cat gaa ttg 2448
Ile Gln Ile Glu Asp Asp Ser Val Lys Val Thr Tyr Val His Glu Leu
                805                 810                 815

cct ggt ttt tct gat gtc gaa gtt aag gta act tat caa gtt gat tac 2496
Pro Gly Phe Ser Asp Val Glu Val Lys Val Thr Tyr Gln Val Asp Tyr
            820                 825                 830

aag ggt cga atc ttt gtt act aca agc tat gat ggt aaa gca ggt ttg 2544
Lys Gly Arg Ile Phe Val Thr Thr Ser Tyr Asp Gly Lys Ala Gly Leu
        835                 840                 845

cca aac ttc cct gaa ttt ggt cta gaa ttt gct atc ggt tca caa ttt 2592
Pro Asn Phe Pro Glu Phe Gly Leu Glu Phe Ala Ile Gly Ser Gln Phe
    850                 855                 860

aca aac cta agc tat tat gga tac ggt gca gaa gaa agc tac ctt gat 2640
Thr Asn Leu Ser Tyr Tyr Gly Tyr Gly Ala Glu Glu Ser Tyr Leu Asp
865                 870                 875                 880

aaa ctt cct ggt gcc tat ctt ggt cga tat gaa aca tct gtt gaa aag 2688
Lys Leu Pro Gly Ala Tyr Leu Gly Arg Tyr Glu Thr Ser Val Glu Lys
                885                 890                 895

aca ttt gct cca tat cta atg cca caa gaa tct ggt aat cac tat ggt 2736
Thr Phe Ala Pro Tyr Leu Met Pro Gln Glu Ser Gly Asn His Tyr Gly
            900                 905                 910

act cgt gaa ttt aca gta tct gat gat aac cat aat ggt ctt aaa ttc 2784
Thr Arg Glu Phe Thr Val Ser Asp Asp Asn His Asn Gly Leu Lys Phe
        915                 920                 925

acc gca cta aat aag gca ttc gaa ttc agt gct ttg ctt aac agt act 2832
Thr Ala Leu Asn Lys Ala Phe Glu Phe Ser Ala Leu Leu Asn Ser Thr
    930                 935                 940

gaa caa att gaa aat gct cgt cat caa tat gaa ttg caa caa tct gat 2880
Glu Gln Ile Glu Asn Ala Arg His Gln Tyr Glu Leu Gln Gln Ser Asp
945                 950                 955                 960

gct aca tgg att aaa gtt ctt gct gtt caa atg ggt gtc ggt ggt gac 2928
Ala Thr Trp Ile Lys Val Leu Ala Val Gln Met Gly Val Gly Gly Asp
                965                 970                 975

gac tca tgg ggt gct cca gtt cat gac gaa ttc ttg gtt agc tca gca 2976
Asp Ser Trp Gly Ala Pro Val His Asp Glu Phe Leu Val Ser Ser Ala
            980                 985                 990

gat agc tat caa ttg agc ttc gtg att gaa cca ctc aac taa 3018
Asp Ser Tyr Gln Leu Ser Phe Val Ile Glu Pro Leu Asn
        995                 1000                1005

<210> 12
<211> 1005
<212> PRT
<213> Streptococcus salivarius CCHSS3

<400> 12

Met Lys Thr Thr Glu Lys Leu Gln Thr Tyr Leu Asn Asp Pro Lys Ile
1               5                   10                  15

Val Ser Val Asn Thr Val Asp Ala His Ser Asp His Lys Tyr Phe Glu
            20                  25                  30

Ser Leu Thr Glu Phe Ser Glu Gly Glu Met Lys Leu Arg Gln Ser Leu
        35                  40                  45

Asn Gly Lys Trp Lys Ile His Tyr Ala Gln Asn Thr Asn Gln Val Leu
    50                  55                  60

Lys Asp Phe Tyr Lys Thr Glu Phe Asp Glu Thr Asp Leu Asn Phe Ile
65                  70                  75                  80

Asn Val Pro Gly His Leu Glu Leu Gln Gly Phe Gly Ser Pro Gln Tyr
                85                  90                  95

Val Asn Thr Gln Tyr Pro Trp Asp Gly Lys Glu Phe Leu Arg Pro Pro
            100                 105                 110

Gln Val Pro Gln Glu Ser Asn Ala Val Ala Ser Tyr Val Lys His Phe
        115                 120                 125

Thr Leu Asn Asp Ala Leu Lys Asp Lys Lys Val Phe Ile Ser Phe Gln
    130                 135                 140

Gly Val Ala Thr Ser Ile Phe Val Trp Val Asn Gly Asn Phe Val Gly
145                 150                 155                 160

Tyr Ser Glu Asp Ser Phe Thr Pro Ser Glu Phe Glu Ile Ser Asp Tyr
                165                 170                 175

Leu Val Gly Gly Asp Asn Lys Leu Ala Val Ala Val Tyr Arg Tyr Ser
            180                 185                 190

Thr Ala Ser Trp Leu Glu Asp Gln Asp Phe Trp Arg Leu Tyr Gly Ile
        195                 200                 205

Phe Arg Asp Val Tyr Leu Tyr Ala Ile Pro Lys Val His Val Gln Asp
    210                 215                 220

Leu Phe Val Lys Gly Asp Tyr Asp Tyr Gln Thr Lys Ala Gly Gln Leu
225                 230                 235                 240

Asp Ile Asp Leu Lys Thr Val Gly Asp Tyr Glu Asp Lys Lys Ile Lys
                245                 250                 255

Tyr Val Leu Ser Asp His Glu Gly Ile Val Lys Glu Gly Asp Ala Ser
            260                 265                 270

Val Asn Ala Asp Gly Glu Leu Ser Val Ser Leu Glu Asp Leu Gln Ile
        275                 280                 285

Lys Pro Trp Ser Ala Glu Ser Pro Lys Leu Tyr Asn Leu Thr Leu His
    290                 295                 300

Val Leu Asp Asp Gly Gln Val Val Glu Val Val Pro Val Lys Val Gly
305                 310                 315                 320

Phe Arg His Phe Glu Ile Lys Asp Lys Leu Met Leu Leu Asn Gly Lys
                325                 330                 335

Arg Ile Val Phe Lys Gly Val Asn Arg His Glu Phe Asn Ala Arg Thr
            340                 345                 350

Gly Arg Cys Ile Thr Glu Glu Asp Met Leu Trp Asp Ile Lys Val Met
        355                 360                 365

Lys Gln His Asn Ile Asn Ala Val Arg Thr Ser His Tyr Pro Asn Gln
    370                 375                 380

Thr Arg Trp Tyr Glu Leu Cys Asp Glu Tyr Gly Leu Tyr Val Ile Asp
385                 390                 395                 400

Glu Ala Asn Leu Glu Thr His Gly Thr Trp Gln Lys Leu Gly Leu Ser
                405                 410                 415

Glu Pro Ser Trp Asn Ile Pro Ala Ser Glu Pro Glu Trp Leu Pro Ala

420                 425                 430

Cys Leu Asp Arg Ala Asn Asn Met Phe Gln Arg Asp Lys Asn His Ala
        435                 440                 445

Ser Val Ile Ile Trp Ser Cys Gly Asn Glu Ser Tyr Ala Gly Lys Asp
    450                 455                 460

Ile Ala Asp Met Ala Asp Tyr Phe Arg Ser Val Asp Asn Thr Arg Pro
465                 470                 475                 480

Val His Tyr Glu Gly Val Thr Trp Cys Arg Glu Phe Asp Tyr Ile Thr
                485                 490                 495

Asp Ile Glu Ser Arg Met Tyr Ala Lys Pro Ala Asp Ile Glu Glu Tyr
            500                 505                 510

Leu Thr Asn Asn Pro Gln Lys Pro Tyr Ile Ser Cys Glu Tyr Met His
        515                 520                 525

Thr Met Gly Asn Ser Gly Gly Gly Leu Lys Leu Tyr Thr Asp Leu Glu
    530                 535                 540

Lys Tyr Pro Glu Tyr Gln Gly Gly Phe Ile Trp Asp Phe Ile Asp Gln
545                 550                 555                 560

Ala Ile Tyr Lys Pro Leu Pro Asn Gly Ser Glu Phe Leu Ser Tyr Gly
                565                 570                 575

Gly Asp Trp His Asp Arg Pro Ser Asp Tyr Glu Phe Cys Gly Asn Gly
            580                 585                 590

Ile Val Phe Ala Asp Arg Thr Leu Thr Pro Lys Leu Gln Thr Val Lys
        595                 600                 605

His Leu Tyr Ser Asn Ile Lys Ile Ala Val Asp Glu Lys Ser Val Thr
    610                 615                 620

Ile Lys Asn Asp Asn Val Phe Glu Asp Leu Ser Ala Tyr Thr Phe Leu
625                 630                 635                 640

Ala Arg Val Tyr Glu Asp Gly Arg Lys Val Ser Glu Ser Glu Tyr His
                645                 650                 655

Phe Asp Val Lys Pro Gly Glu Glu Ala Thr Phe Pro Val Glu Phe Ala
            660                 665                 670

Val Gly Ala Ser Asn Ala Glu Arg Ile Tyr Glu Val Ala Cys Val Gln
        675                 680                 685

Asn Glu Ala Thr Glu Trp Ala Pro Lys Gly His Glu Ile Val Arg Gly
    690                 695                 700

Gln Tyr Val Ala Glu Lys Ile Ser Thr Glu Thr Pro Val Lys Ala Pro
705                 710                 715                 720

Leu Asn Val Val Glu Gly Asp Phe Asn Ile Gly Ile Gln Gly Gln Asn
                725                 730                 735

Phe Ser Ile Leu Leu Ser Arg Ala Gln Asn Thr Leu Val Ser Ala Lys
            740                 745                 750

Tyr Asn Gly Val Glu Phe Ile Glu Lys Gly Pro Lys Leu Asn Phe Thr
        755                 760                 765

Arg Ala Tyr Thr Asp Asn Asp Arg Gly Ala Gly Tyr Pro Phe Glu Met
    770                 775                 780

Ala Gly Trp Lys Val Ala Gly Asn Tyr Ser Lys Val Thr Asp Thr Gln
785                 790                 795                 800

Ile Gln Ile Glu Asp Asp Ser Val Lys Val Thr Tyr Val His Glu Leu
                805                 810                 815

Pro Gly Phe Ser Asp Val Glu Val Lys Val Thr Tyr Gln Val Asp Tyr
            820                 825                 830

Lys Gly Arg Ile Phe Val Thr Thr Ser Tyr Asp Gly Lys Ala Gly Leu
        835                 840                 845

Pro Asn Phe Pro Glu Phe Gly Leu Glu Phe Ala Ile Gly Ser Gln Phe
    850                 855                 860

Thr Asn Leu Ser Tyr Tyr Gly Tyr Gly Ala Glu Glu Ser Tyr Leu Asp
865                 870                 875                 880

Lys Leu Pro Gly Ala Tyr Leu Gly Arg Tyr Glu Thr Ser Val Glu Lys
                885                 890                 895

Thr Phe Ala Pro Tyr Leu Met Pro Gln Glu Ser Gly Asn His Tyr Gly
            900                 905                 910

Thr Arg Glu Phe Thr Val Ser Asp Asp Asn His Asn Gly Leu Lys Phe
        915                 920                 925

Thr Ala Leu Asn Lys Ala Phe Glu Phe Ser Ala Leu Leu Asn Ser Thr
    930                 935                 940

Glu Gln Ile Glu Asn Ala Arg His Gln Tyr Glu Leu Gln Gln Ser Asp
945                 950                 955                 960

Ala Thr Trp Ile Lys Val Leu Ala Val Gln Met Gly Val Gly Gly Asp
                965                 970                 975

Asp Ser Trp Gly Ala Pro Val His Asp Glu Phe Leu Val Ser Ser Ala
            980                 985                 990

Asp Ser Tyr Gln Leu Ser Phe Val  Ile Glu Pro Leu Asn
        995                 1000                1005

<210>  13
<211>  945
<212>  DNA
<213>  Corynebacterium glutamicum strain ATCC 13032

<220>
<221>  CDS
<222>  (1)..(945)
<223>  ldh gene encoding L-lactate dehydrogenase

<300>
<308>  GenBank CP025533.1
<309>  2017-12-27
<313>  (3119670)..(3120614)

<400>  13
atg aaa gaa acc gtc ggt aac aag att gtc ctc att ggc gca gga gat      48
Met Lys Glu Thr Val Gly Asn Lys Ile Val Leu Ile Gly Ala Gly Asp
1               5                   10                  15

gtt gga gtt gca tac gca tac gca ctg atc aac cag ggc atg gca gat      96
Val Gly Val Ala Tyr Ala Tyr Ala Leu Ile Asn Gln Gly Met Ala Asp
            20                  25                  30

cac ctt gcg atc atc gac atc gat gaa aag aaa ctc gaa ggc aac gtc      144
His Leu Ala Ile Ile Asp Ile Asp Glu Lys Lys Leu Glu Gly Asn Val
        35                  40                  45

atg gac tta aac cat ggt gtt gtg tgg gcc gat tcc cgc acc cgc gtc      192
Met Asp Leu Asn His Gly Val Val Trp Ala Asp Ser Arg Thr Arg Val
    50                  55                  60

acc aag ggc acc tac gct gac tgc gaa gac gca gcc atg gtt gtc att      240
Thr Lys Gly Thr Tyr Ala Asp Cys Glu Asp Ala Ala Met Val Val Ile
65                  70                  75                  80

tgt gcc ggc gca gcc caa aag cca ggc gag acc cgc ctc cag ctg gtg      288
Cys Ala Gly Ala Ala Gln Lys Pro Gly Glu Thr Arg Leu Gln Leu Val
                85                  90                  95

gac aaa aac gtc aag att atg aaa tcc atc gtc ggc gat gtc atg gac      336

Asp Lys Asn Val Lys Ile Met Lys Ser Ile Val Gly Asp Val Met Asp
            100                 105                 110

agc gga ttc gac ggc atc ttc ctc gtg gcg tcc aac cca gtg gat atc 384
Ser Gly Phe Asp Gly Ile Phe Leu Val Ala Ser Asn Pro Val Asp Ile
        115                 120                 125

ctg acc tac gca gtg tgg aaa ttc tcc ggc ttg gaa tgg aac cgc gtg 432
Leu Thr Tyr Ala Val Trp Lys Phe Ser Gly Leu Glu Trp Asn Arg Val
    130                 135                 140

atc ggc tcc gga act gtc ctg gac tcc gct cga ttc cgc tac atg ctg 480
Ile Gly Ser Gly Thr Val Leu Asp Ser Ala Arg Phe Arg Tyr Met Leu
145                 150                 155                 160

ggc gaa ctc tac gaa gtg gca cca agc tcc gtc cac gcc tac atc atc 528
Gly Glu Leu Tyr Glu Val Ala Pro Ser Ser Val His Ala Tyr Ile Ile
                165                 170                 175

ggc gaa cac ggc gac act gaa ctt cca gtc ctg tcc tcc gcg acc atc 576
Gly Glu His Gly Asp Thr Glu Leu Pro Val Leu Ser Ser Ala Thr Ile
            180                 185                 190

gca ggc gta tcg ctt agc cga atg ctg gac aaa gac cca gag ctt gag 624
Ala Gly Val Ser Leu Ser Arg Met Leu Asp Lys Asp Pro Glu Leu Glu
        195                 200                 205

ggc cgt cta gag aaa att ttc gaa gac acc cgc gac gct gcc tat cac 672
Gly Arg Leu Glu Lys Ile Phe Glu Asp Thr Arg Asp Ala Ala Tyr His
    210                 215                 220

att atc gac gcc aag ggc tcc act tcc tac ggc atc ggc atg ggt ctt 720
Ile Ile Asp Ala Lys Gly Ser Thr Ser Tyr Gly Ile Gly Met Gly Leu
225                 230                 235                 240

gct cgc atc acc cgc gca atc ctg cag aac caa gac gtt gca gtc cca 768
Ala Arg Ile Thr Arg Ala Ile Leu Gln Asn Gln Asp Val Ala Val Pro
                245                 250                 255

gtc tct gca ctg ctc cac ggt gaa tac ggt gag gaa gac atc tac atc 816
Val Ser Ala Leu Leu His Gly Glu Tyr Gly Glu Glu Asp Ile Tyr Ile
            260                 265                 270

ggc acc cca gct gtg gtg aac cgc cga ggc atc cgc cgc gtt gtc gaa 864
Gly Thr Pro Ala Val Val Asn Arg Arg Gly Ile Arg Arg Val Val Glu
        275                 280                 285

cta gaa atc acc gac cac gag atg gaa cgc ttc aag cat tcc gca aat 912
Leu Glu Ile Thr Asp His Glu Met Glu Arg Phe Lys His Ser Ala Asn
    290                 295                 300

acc ctg cgc gaa att cag aag cag ttc ttc taa 945
Thr Leu Arg Glu Ile Gln Lys Gln Phe Phe
305                 310

<210> 14
<211> 314
<212> PRT
<213> Corynebacterium glutamicum strain ATCC 13032

<400> 14

Met Lys Glu Thr Val Gly Asn Lys Ile Val Leu Ile Gly Ala Gly Asp
1               5                   10                  15

Val Gly Val Ala Tyr Ala Tyr Ala Leu Ile Asn Gln Gly Met Ala Asp
            20                  25                  30

His Leu Ala Ile Ile Asp Ile Asp Glu Lys Lys Leu Glu Gly Asn Val
        35                  40                  45

Met Asp Leu Asn His Gly Val Val Trp Ala Asp Ser Arg Thr Arg Val
    50                  55                  60

Thr Lys Gly Thr Tyr Ala Asp Cys Glu Asp Ala Ala Met Val Val Ile
65                  70                  75                  80

Cys Ala Gly Ala Ala Gln Lys Pro Gly Glu Thr Arg Leu Gln Leu Val
                85                  90                  95

Asp Lys Asn Val Lys Ile Met Lys Ser Ile Val Gly Asp Val Met Asp
            100                 105                 110

Ser Gly Phe Asp Gly Ile Phe Leu Val Ala Ser Asn Pro Val Asp Ile
        115                 120                 125

Leu Thr Tyr Ala Val Trp Lys Phe Ser Gly Leu Glu Trp Asn Arg Val
    130                 135                 140

Ile Gly Ser Gly Thr Val Leu Asp Ser Ala Arg Phe Arg Tyr Met Leu
145                 150                 155                 160

Gly Glu Leu Tyr Glu Val Ala Pro Ser Ser Val His Ala Tyr Ile Ile
                165                 170                 175

Gly Glu His Gly Asp Thr Glu Leu Pro Val Leu Ser Ser Ala Thr Ile
            180                 185                 190

Ala Gly Val Ser Leu Ser Arg Met Leu Asp Lys Asp Pro Glu Leu Glu
        195                 200                 205

Gly Arg Leu Glu Lys Ile Phe Glu Asp Thr Arg Asp Ala Ala Tyr His
    210                 215                 220

Ile Ile Asp Ala Lys Gly Ser Thr Ser Tyr Gly Ile Gly Met Gly Leu
225                 230                 235                 240

Ala Arg Ile Thr Arg Ala Ile Leu Gln Asn Gln Asp Val Ala Val Pro
                245                 250                 255

Val Ser Ala Leu Leu His Gly Glu Tyr Gly Glu Glu Asp Ile Tyr Ile
            260                 265                 270

Gly Thr Pro Ala Val Val Asn Arg Arg Gly Ile Arg Arg Val Val Glu
        275                 280                 285

Leu Glu Ile Thr Asp His Glu Met Glu Arg Phe Lys His Ser Ala Asn
    290                 295                 300

Thr Leu Arg Glu Ile Gln Lys Gln Phe Phe
305                 310

<210>  15
<211>  948
<212>  DNA
<213>  Corynebacterium callunae DSM 20147

<220>
<221>  CDS
<222>  (1)..(948)
<223>  ldh gene encoding L-lactate-dehydrogenase

<300>
<308>  GenBank CP004354.1
<309>  2015-01-16
<313>  (2672590)..(2673537)

<400>  15
atg aaa aag acc gtc gga aat aag atc gtt ctc att ggt gca ggt gat      48
Met Lys Lys Thr Val Gly Asn Lys Ile Val Leu Ile Gly Ala Gly Asp
1               5                   10                  15

gtc gga gtt gct tat gct tat gcg ctg att aac caa gga acc tgc gat      96
Val Gly Val Ala Tyr Ala Tyr Ala Leu Ile Asn Gln Gly Thr Cys Asp
            20                  25                  30

cac cta gcc atc att gac att gat gaa aag aag ctc gaa ggc aat gtc      144
His Leu Ala Ile Ile Asp Ile Asp Glu Lys Lys Leu Glu Gly Asn Val
        35                  40                  45

atg gac cta aac cac ggt gtg gtg tgg tcg gaa tcc cgt act cgc gta      192
Met Asp Leu Asn His Gly Val Val Trp Ser Glu Ser Arg Thr Arg Val
    50                  55                  60

acc aag gga acc tac gca gac tgc gaa gac gct gcc atg gtg gtt att      240
Thr Lys Gly Thr Tyr Ala Asp Cys Glu Asp Ala Ala Met Val Val Ile
65                  70                  75                  80

tgc gca ggt gca gcc caa agg cca ggc gaa act cgc ctg cag ctg gtg      288
Cys Ala Gly Ala Ala Gln Arg Pro Gly Glu Thr Arg Leu Gln Leu Val
                85                  90                  95

gat aaa aac gtc aag atc atg aag tct att gtt ggc gag gtc atg gcc      336
Asp Lys Asn Val Lys Ile Met Lys Ser Ile Val Gly Glu Val Met Ala
            100                 105                 110

gcc ggc ttt gat gga att ttc ctg gta gcc agc aac ccc gta gac atc      384

Ala Gly Phe Asp Gly Ile Phe Leu Val Ala Ser Asn Pro Val Asp Ile
        115                 120                 125

ctc act tat gca gta tgg aag ttc tcc ggc ttg gac tgg cac cgc gta     432
Leu Thr Tyr Ala Val Trp Lys Phe Ser Gly Leu Asp Trp His Arg Val
    130                 135                 140

atc ggc tcg ggc acc gtc ttg gac tcc gca cgt ttc cgc tat atg ctg     480
Ile Gly Ser Gly Thr Val Leu Asp Ser Ala Arg Phe Arg Tyr Met Leu
145                 150                 155                 160

ggc gag ctt tat gag gta gcg cca agc tcc att cac tcc tac att att     528
Gly Glu Leu Tyr Glu Val Ala Pro Ser Ser Ile His Ser Tyr Ile Ile
                165                 170                 175

ggc gag cat ggc gat acc gaa ctc cca gtg ctt tct tcc gcc acc atc     576
Gly Glu His Gly Asp Thr Glu Leu Pro Val Leu Ser Ser Ala Thr Ile
            180                 185                 190

gcc ggt gtt tcc ctg cgc aag ctt ttg gaa aag gat cca gag cta gat     624
Ala Gly Val Ser Leu Arg Lys Leu Leu Glu Lys Asp Pro Glu Leu Asp
        195                 200                 205

ggc cgc ttg gaa aaa gtc ttt gaa gat act cgc gac gct gcc tat cac     672
Gly Arg Leu Glu Lys Val Phe Glu Asp Thr Arg Asp Ala Ala Tyr His
    210                 215                 220

atc atc gat gcc aag ggc tca acc tct tat ggc att ggc atg ggc ttg     720
Ile Ile Asp Ala Lys Gly Ser Thr Ser Tyr Gly Ile Gly Met Gly Leu
225                 230                 235                 240

gcg cgt atc acc cgc gcg gtt atc caa aac caa gac gtg gcg ctt ccc     768
Ala Arg Ile Thr Arg Ala Val Ile Gln Asn Gln Asp Val Ala Leu Pro
                245                 250                 255

gta tct gcg ctg ctc aat ggc gaa tat ggg gaa gaa gac att tat att     816
Val Ser Ala Leu Leu Asn Gly Glu Tyr Gly Glu Glu Asp Ile Tyr Ile
            260                 265                 270

ggt acc ccg gcc atc att aac cgc cgt ggt att cgc cgc gtg gta gaa     864
Gly Thr Pro Ala Ile Ile Asn Arg Arg Gly Ile Arg Arg Val Val Glu
        275                 280                 285

ctg gaa atc agc gag cat gag atg gag cgc ttc aag cac tcc gct aat     912
Leu Glu Ile Ser Glu His Glu Met Glu Arg Phe Lys His Ser Ala Asn
    290                 295                 300

act ttg cgt gct att cag gaa cag att ttc agc taa                     948
Thr Leu Arg Ala Ile Gln Glu Gln Ile Phe Ser
305                 310                 315


<210>  16
<211>  315
<212>  PRT
<213>  Corynebacterium callunae DSM 20147

<400>  16

Met Lys Lys Thr Val Gly Asn Lys Ile Val Leu Ile Gly Ala Gly Asp
1               5                   10                  15

Val Gly Val Ala Tyr Ala Tyr Ala Leu Ile Asn Gln Gly Thr Cys Asp
            20                  25                  30
His Leu Ala Ile Ile Asp Ile Asp Glu Lys Lys Leu Glu Gly Asn Val
        35                  40                  45
Met Asp Leu Asn His Gly Val Val Trp Ser Glu Ser Arg Thr Arg Val
    50                  55                  60
Thr Lys Gly Thr Tyr Ala Asp Cys Glu Asp Ala Ala Met Val Val Ile
65                  70                  75                  80
Cys Ala Gly Ala Ala Gln Arg Pro Gly Glu Thr Arg Leu Gln Leu Val
                85                  90                  95
Asp Lys Asn Val Lys Ile Met Lys Ser Ile Val Gly Glu Val Met Ala
            100                 105                 110
Ala Gly Phe Asp Gly Ile Phe Leu Val Ala Ser Asn Pro Val Asp Ile
        115                 120                 125
Leu Thr Tyr Ala Val Trp Lys Phe Ser Gly Leu Asp Trp His Arg Val
    130                 135                 140
Ile Gly Ser Gly Thr Val Leu Asp Ser Ala Arg Phe Arg Tyr Met Leu
145                 150                 155                 160
Gly Glu Leu Tyr Glu Val Ala Pro Ser Ser Ile His Ser Tyr Ile Ile
                165                 170                 175
Gly Glu His Gly Asp Thr Glu Leu Pro Val Leu Ser Ser Ala Thr Ile
            180                 185                 190
Ala Gly Val Ser Leu Arg Lys Leu Leu Glu Lys Asp Pro Glu Leu Asp
        195                 200                 205
Gly Arg Leu Glu Lys Val Phe Glu Asp Thr Arg Asp Ala Ala Tyr His
    210                 215                 220
Ile Ile Asp Ala Lys Gly Ser Thr Ser Tyr Gly Ile Gly Met Gly Leu
225                 230                 235                 240
Ala Arg Ile Thr Arg Ala Val Ile Gln Asn Gln Asp Val Ala Leu Pro
                245                 250                 255
Val Ser Ala Leu Leu Asn Gly Glu Tyr Gly Glu Glu Asp Ile Tyr Ile
            260                 265                 270

Gly Thr Pro Ala Ile Ile Asn Arg Arg Gly Ile Arg Arg Val Val Glu
        275                 280                 285

Leu Glu Ile Ser Glu His Glu Met Glu Arg Phe Lys His Ser Ala Asn
    290                 295                 300

Thr Leu Arg Ala Ile Gln Glu Gln Ile Phe Ser
305                 310                 315

<210>  17
<211>  954
<212>  DNA
<213>  Corynebacterium kutscheri strain DSM 20755

<220>
<221>  CDS
<222>  (1)..(954)
<223>  ldh gene encodingL-lactate-dehydrogenase

<300>
<308>  GenBank CP011312.1
<309>  2015-06-10
<313>  (2203817)..(2204770)

<400>  17
atg aag aaa aac gtc gga aat aaa gtc gtc ctt att ggt gct ggt gac      48
Met Lys Lys Asn Val Gly Asn Lys Val Val Leu Ile Gly Ala Gly Asp
1               5                   10                  15

gtc gga gtt gct tac gct tat gct ttg gta aac cag ggc act gca gac      96
Val Gly Val Ala Tyr Ala Tyr Ala Leu Val Asn Gln Gly Thr Ala Asp
            20                  25                  30

cac cta gcc att att gat atc gat gaa aag aag cta gaa ggc aac gtt      144
His Leu Ala Ile Ile Asp Ile Asp Glu Lys Lys Leu Glu Gly Asn Val
        35                  40                  45

aaa gac ctt aac cat ggt gtt gtc tgg tcc gga cat cgc acc acc gtt      192
Lys Asp Leu Asn His Gly Val Val Trp Ser Gly His Arg Thr Thr Val
    50                  55                  60

acc aag gga agc tat gcc gat tgc gct gat gca tcg atg gtt gtg atc      240
Thr Lys Gly Ser Tyr Ala Asp Cys Ala Asp Ala Ser Met Val Val Ile
65                  70                  75                  80

tgt gcc ggt gct gct cag aag cca ggc gag acg cgc ttg caa ctg gtg      288
Cys Ala Gly Ala Ala Gln Lys Pro Gly Glu Thr Arg Leu Gln Leu Val
                85                  90                  95

ggt aag aat atg ggc att atg aaa tcc atc gtc gat gag gtc atg gct      336
Gly Lys Asn Met Gly Ile Met Lys Ser Ile Val Asp Glu Val Met Ala
            100                 105                 110

aat ggt ttc gac gga att ttc ctt att gca tcc aac cca gta gat att      384
Asn Gly Phe Asp Gly Ile Phe Leu Ile Ala Ser Asn Pro Val Asp Ile
        115                 120                 125

ctt acc tac gca gta tgg cag tac tcc ggc ttg gac tgg cac cgt gtt      432

Leu Thr Tyr Ala Val Trp Gln Tyr Ser Gly Leu Asp Trp His Arg Val
    130                 135                 140

att gga tcc ggc acc gtt ttg gat tcg gca cgc ttc cgc tat atg ctc      480
Ile Gly Ser Gly Thr Val Leu Asp Ser Ala Arg Phe Arg Tyr Met Leu
145                 150                 155                 160

ggc gag cgc tac caa gtg aat cca tct tcg att cac gct tat att atc      528
Gly Glu Arg Tyr Gln Val Asn Pro Ser Ser Ile His Ala Tyr Ile Ile
                165                 170                 175

ggt gag cac ggc gat act gag cta cca gtg ctg tcc tca gcg acc att      576
Gly Glu His Gly Asp Thr Glu Leu Pro Val Leu Ser Ser Ala Thr Ile
            180                 185                 190

gct ggt gtg tct atg cgc aag cag ttg gag aag aac cca ggc ctg gaa      624
Ala Gly Val Ser Met Arg Lys Gln Leu Glu Lys Asn Pro Gly Leu Glu
        195                 200                 205

gca gaa cta gag aag atc ttc gaa gaa acc cgc gac gcc gct tac aac      672
Ala Glu Leu Glu Lys Ile Phe Glu Glu Thr Arg Asp Ala Ala Tyr Asn
    210                 215                 220

att att gag gcc aag gga tca act tcc tac ggt atc ggc atg ggg ctt      720
Ile Ile Glu Ala Lys Gly Ser Thr Ser Tyr Gly Ile Gly Met Gly Leu
225                 230                 235                 240

gct cgc att aca aaa gcg gtt ttg cat aat cag gat gtt gct ttg cca      768
Ala Arg Ile Thr Lys Ala Val Leu His Asn Gln Asp Val Ala Leu Pro
                245                 250                 255

tgc tcc gca ttg ttg gaa gga caa tat ggc cag gaa aac att tac att      816
Cys Ser Ala Leu Leu Glu Gly Gln Tyr Gly Gln Glu Asn Ile Tyr Ile
            260                 265                 270

ggt acc cca gct gtg att aat ggt ggt gga atc gag cgc gtt gtt gaa      864
Gly Thr Pro Ala Val Ile Asn Gly Gly Gly Ile Glu Arg Val Val Glu
        275                 280                 285

ctt gaa ctt acc gat cat gag cgg gag cgt ttc acc cac tca gct gat      912
Leu Glu Leu Thr Asp His Glu Arg Glu Arg Phe Thr His Ser Ala Asp
    290                 295                 300

acc ttg cgc tca atc cag gaa gaa ttc ttc cca gct aag taa              954
Thr Leu Arg Ser Ile Gln Glu Glu Phe Phe Pro Ala Lys
305                 310                 315


<210>  18
<211>  317
<212>  PRT
<213>  Corynebacterium kutscheri strain DSM 20755

<400>  18

Met Lys Lys Asn Val Gly Asn Lys Val Val Leu Ile Gly Ala Gly Asp
1               5                   10                  15


Val Gly Val Ala Tyr Ala Tyr Ala Leu Val Asn Gln Gly Thr Ala Asp
            20                  25                  30

His Leu Ala Ile Ile Asp Ile Asp Glu Lys Lys Leu Glu Gly Asn Val
        35                  40                  45

Lys Asp Leu Asn His Gly Val Val Trp Ser Gly His Arg Thr Thr Val
    50                  55                  60

Thr Lys Gly Ser Tyr Ala Asp Cys Ala Asp Ala Ser Met Val Val Ile
65                  70                  75                  80

Cys Ala Gly Ala Ala Gln Lys Pro Gly Glu Thr Arg Leu Gln Leu Val
                85                  90                  95

Gly Lys Asn Met Gly Ile Met Lys Ser Ile Val Asp Glu Val Met Ala
            100                 105                 110

Asn Gly Phe Asp Gly Ile Phe Leu Ile Ala Ser Asn Pro Val Asp Ile
        115                 120                 125

Leu Thr Tyr Ala Val Trp Gln Tyr Ser Gly Leu Asp Trp His Arg Val
    130                 135                 140

Ile Gly Ser Gly Thr Val Leu Asp Ser Ala Arg Phe Arg Tyr Met Leu
145                 150                 155                 160

Gly Glu Arg Tyr Gln Val Asn Pro Ser Ser Ile His Ala Tyr Ile Ile
                165                 170                 175

Gly Glu His Gly Asp Thr Glu Leu Pro Val Leu Ser Ser Ala Thr Ile
            180                 185                 190

Ala Gly Val Ser Met Arg Lys Gln Leu Glu Lys Asn Pro Gly Leu Glu
        195                 200                 205

Ala Glu Leu Glu Lys Ile Phe Glu Glu Thr Arg Asp Ala Ala Tyr Asn
    210                 215                 220

Ile Ile Glu Ala Lys Gly Ser Thr Ser Tyr Gly Ile Gly Met Gly Leu
225                 230                 235                 240

Ala Arg Ile Thr Lys Ala Val Leu His Asn Gln Asp Val Ala Leu Pro
                245                 250                 255

Cys Ser Ala Leu Leu Glu Gly Gln Tyr Gly Gln Glu Asn Ile Tyr Ile
            260                 265                 270

Gly Thr Pro Ala Val Ile Asn Gly Gly Gly Ile Glu Arg Val Val Glu
        275                 280                 285

Leu Glu Leu Thr Asp His Glu Arg Glu Arg Phe Thr His Ser Ala Asp
    290                 295                 300

Thr Leu Arg Ser Ile Gln Glu Glu Phe Phe Pro Ala Lys
305                 310                 315

<210>  19
<211>  1020
<212>  DNA
<213>  Lactococcus lactis subsp. cremoris NZ9000

<220>
<221>  CDS
<222>  (1)..(1020)
<223>  galM gene encoding aldose 1-epimerase

<300>
<308>  GenBank CP002094.1
<309>  2014-01-30
<313>  (2199168)..(2200187)

<400>  19
atg gaa att aca aca aaa gat ttt ggt tta gga agt agc cta att agt      48
Met Glu Ile Thr Thr Lys Asp Phe Gly Leu Gly Ser Ser Leu Ile Ser
1               5                   10                  15

cta acg aac aaa aat gat gtt aca att gct ttt aca aac tta gga gct      96
Leu Thr Asn Lys Asn Asp Val Thr Ile Ala Phe Thr Asn Leu Gly Ala
            20                  25                  30

cgc att gtt gat tgg caa aaa gac ggg aaa cat ttt att tta gga ttt      144
Arg Ile Val Asp Trp Gln Lys Asp Gly Lys His Phe Ile Leu Gly Phe
        35                  40                  45

gat tcc gcc caa gaa tat tta gaa aaa gat gct tat cca ggg gcc aca      192
Asp Ser Ala Gln Glu Tyr Leu Glu Lys Asp Ala Tyr Pro Gly Ala Thr
    50                  55                  60

gtt gga cgt aca gct ggc aga atc aaa gat ggt ttg gtt gat att tct      240
Val Gly Arg Thr Ala Gly Arg Ile Lys Asp Gly Leu Val Asp Ile Ser
65                  70                  75                  80

gga aaa aca tat cat tta aat caa aat gaa gct ccc caa acc tta cat      288
Gly Lys Thr Tyr His Leu Asn Gln Asn Glu Ala Pro Gln Thr Leu His
                85                  90                  95

ggt gga gaa gat agc att cat acc aaa ctt tgg act tat gaa att aat      336
Gly Gly Glu Asp Ser Ile His Thr Lys Leu Trp Thr Tyr Glu Ile Asn
            100                 105                 110

gat tta gga gat gaa gtt caa gtc aaa ttt agc tta gtt tcc aat gat      384
Asp Leu Gly Asp Glu Val Gln Val Lys Phe Ser Leu Val Ser Asn Asp
        115                 120                 125

gga gaa aat gga tat cct ggt aaa ata gag atg tcg gtg act cat tct      432
Gly Glu Asn Gly Tyr Pro Gly Lys Ile Glu Met Ser Val Thr His Ser
    130                 135                 140

ttt gat gaa gag aac aat tgg aaa att aag tat gaa gca att tct gac      480

Phe Asp Glu Glu Asn Asn Trp Lys Ile Lys Tyr Glu Ala Ile Ser Asp
145                 150                 155                 160

aaa gat acg gta ttt aat ccc aca gga cat gtc tat ttc aac tta aat      528
Lys Asp Thr Val Phe Asn Pro Thr Gly His Val Tyr Phe Asn Leu Asn
                165                 170                 175

ggg gat gct agc aag tct att gaa aat cat caa ctc aag ctt gct gct      576
Gly Asp Ala Ser Lys Ser Ile Glu Asn His Gln Leu Lys Leu Ala Ala
            180                 185                 190

tca aga ttt gta cct tta aaa gat caa aca gaa att gtc cgt ggc gat      624
Ser Arg Phe Val Pro Leu Lys Asp Gln Thr Glu Ile Val Arg Gly Asp
        195                 200                 205

atc gtt gat aca aaa aat act gac tta gac ttc cgt cag gaa aaa caa      672
Ile Val Asp Thr Lys Asn Thr Asp Leu Asp Phe Arg Gln Glu Lys Gln
    210                 215                 220

tta tca aaa gcc tta gag tct agc atg gaa caa gtt caa ctg gtt ggt      720
Leu Ser Lys Ala Leu Glu Ser Ser Met Glu Gln Val Gln Leu Val Gly
225                 230                 235                 240

gga att gac cac ccc ttc tta tta gat gaa cag agc ctt gag aaa gaa      768
Gly Ile Asp His Pro Phe Leu Leu Asp Glu Gln Ser Leu Glu Lys Glu
                245                 250                 255

caa gcg cgt tta tct tta gat gac ttg tca gtt tcc gtt tat act gac      816
Gln Ala Arg Leu Ser Leu Asp Asp Leu Ser Val Ser Val Tyr Thr Asp
            260                 265                 270

caa cca agt att gta att ttt aca gca aac ttt gga gat ttg ggg acc      864
Gln Pro Ser Ile Val Ile Phe Thr Ala Asn Phe Gly Asp Leu Gly Thr
        275                 280                 285

gtt tat cac ggg aaa aat caa gtt cat cat ggt ggt att acc ttt gaa      912
Val Tyr His Gly Lys Asn Gln Val His His Gly Gly Ile Thr Phe Glu
    290                 295                 300

tgt caa gtt tca ccc ggg tca caa cag att cca gaa ctt gga gac att      960
Cys Gln Val Ser Pro Gly Ser Gln Gln Ile Pro Glu Leu Gly Asp Ile
305                 310                 315                 320

agt tta aag gca gga gat gaa tat caa gcc acc acc att tat agt tta     1008
Ser Leu Lys Ala Gly Asp Glu Tyr Gln Ala Thr Thr Ile Tyr Ser Leu
                325                 330                 335

cat aca aat taa                                                     1020
His Thr Asn

<210>  20
<211>  339
<212>  PRT
<213>  Lactococcus lactis subsp. cremoris NZ9000

<400>  20

Met Glu Ile Thr Thr Lys Asp Phe Gly Leu Gly Ser Ser Leu Ile Ser
1               5                   10                  15

Leu Thr Asn Lys Asn Asp Val Thr Ile Ala Phe Thr Asn Leu Gly Ala
            20                  25                  30

Arg Ile Val Asp Trp Gln Lys Asp Gly Lys His Phe Ile Leu Gly Phe
        35                  40                  45

Asp Ser Ala Gln Glu Tyr Leu Glu Lys Asp Ala Tyr Pro Gly Ala Thr
    50                  55                  60

Val Gly Arg Thr Ala Gly Arg Ile Lys Asp Gly Leu Val Asp Ile Ser
65                  70                  75                  80

Gly Lys Thr Tyr His Leu Asn Gln Asn Glu Ala Pro Gln Thr Leu His
                85                  90                  95

Gly Gly Glu Asp Ser Ile His Thr Lys Leu Trp Thr Tyr Glu Ile Asn
            100                 105                 110

Asp Leu Gly Asp Glu Val Gln Val Lys Phe Ser Leu Val Ser Asn Asp
        115                 120                 125

Gly Glu Asn Gly Tyr Pro Gly Lys Ile Glu Met Ser Val Thr His Ser
    130                 135                 140

Phe Asp Glu Glu Asn Asn Trp Lys Ile Lys Tyr Glu Ala Ile Ser Asp
145                 150                 155                 160

Lys Asp Thr Val Phe Asn Pro Thr Gly His Val Tyr Phe Asn Leu Asn
                165                 170                 175

Gly Asp Ala Ser Lys Ser Ile Glu Asn His Gln Leu Lys Leu Ala Ala
            180                 185                 190

Ser Arg Phe Val Pro Leu Lys Asp Gln Thr Glu Ile Val Arg Gly Asp
        195                 200                 205

Ile Val Asp Thr Lys Asn Thr Asp Leu Asp Phe Arg Gln Glu Lys Gln
    210                 215                 220

Leu Ser Lys Ala Leu Glu Ser Ser Met Glu Gln Val Gln Leu Val Gly
225                 230                 235                 240

Gly Ile Asp His Pro Phe Leu Leu Asp Glu Gln Ser Leu Glu Lys Glu
                245                 250                 255

Gln Ala Arg Leu Ser Leu Asp Asp Leu Ser Val Ser Val Tyr Thr Asp
            260                 265                 270

Gln Pro Ser Ile Val Ile Phe Thr Ala Asn Phe Gly Asp Leu Gly Thr
        275                 280                 285

Val Tyr His Gly Lys Asn Gln Val His His Gly Gly Ile Thr Phe Glu
    290                 295                 300

Cys Gln Val Ser Pro Gly Ser Gln Gln Ile Pro Glu Leu Gly Asp Ile
305                 310                 315                 320

Ser Leu Lys Ala Gly Asp Glu Tyr Gln Ala Thr Thr Ile Tyr Ser Leu
                325                 330                 335

His Thr Asn

<210>  21
<211>  1020
<212>  DNA
<213>  Lactococcus lactis subsp. lactis NCDO 2118

<220>
<221>  CDS
<222>  (1)..(1020)
<223>  galM gene encoding Aldose 1-epimerase

<300>
<308>  GenBank CP009054.1
<309>  2014-10-21
<313>  (2244316)..(2245335)

<400>  21
atg gaa att aaa ata aga gat ttt ggc tta gga agt gac tta att agt      48
Met Glu Ile Lys Ile Arg Asp Phe Gly Leu Gly Ser Asp Leu Ile Ser
1               5                   10                  15

cta acc aat aaa gca ggt gtc aca att tct ttt aca aat ctg ggc gct      96
Leu Thr Asn Lys Ala Gly Val Thr Ile Ser Phe Thr Asn Leu Gly Ala
            20                  25                  30

cgg att gtt gac tgg caa aaa gat gga aag cat ctg att cta ggc ttt      144
Arg Ile Val Asp Trp Gln Lys Asp Gly Lys His Leu Ile Leu Gly Phe
        35                  40                  45

gat tca gct aaa gaa tat tta gaa aaa gat gcc tat cct ggt gca aca      192
Asp Ser Ala Lys Glu Tyr Leu Glu Lys Asp Ala Tyr Pro Gly Ala Thr
    50                  55                  60

gtt gga cct aca gca gga aga att aag gat ggt ttg gtc aaa att tct      240
Val Gly Pro Thr Ala Gly Arg Ile Lys Asp Gly Leu Val Lys Ile Ser
65                  70                  75                  80

ggg aaa gat tat atc tta aat caa aat gaa ggc cct caa acc tta cat      288
Gly Lys Asp Tyr Ile Leu Asn Gln Asn Glu Gly Pro Gln Thr Leu His
                85                  90                  95

ggt ggg gaa gaa agt att cat act aaa ctt tgg aca tat gaa gtt acc      336

Gly Gly Glu Glu Ser Ile His Thr Lys Leu Trp Thr Tyr Glu Val Thr
            100                 105                 110

gat tta gga gct gaa gtt caa gtc aaa ttt tcc tta gtt tct aat gac      384
Asp Leu Gly Ala Glu Val Gln Val Lys Phe Ser Leu Val Ser Asn Asp
        115                 120                 125

gga aca aat ggt tat cct ggt aaa ata gaa atg tca gta act cat tct      432
Gly Thr Asn Gly Tyr Pro Gly Lys Ile Glu Met Ser Val Thr His Ser
    130                 135                 140

ttt gat gag gac aat aag tgg aaa att cat tat gag gcc att tct gat      480
Phe Asp Glu Asp Asn Lys Trp Lys Ile His Tyr Glu Ala Ile Ser Asp
145                 150                 155                 160

aaa gac act gtg ttt aat cca act ggg cat gtc tat ttt aat ttg aat      528
Lys Asp Thr Val Phe Asn Pro Thr Gly His Val Tyr Phe Asn Leu Asn
                165                 170                 175

ggt gat gct agt gaa tct gtt gaa aat cat gga ctt cga ctg gcc gct      576
Gly Asp Ala Ser Glu Ser Val Glu Asn His Gly Leu Arg Leu Ala Ala
            180                 185                 190

tca aga ttt gtt cct tta aaa gac caa aca gaa att gtt cgt ggt gat      624
Ser Arg Phe Val Pro Leu Lys Asp Gln Thr Glu Ile Val Arg Gly Asp
        195                 200                 205

atc gtt gat ata aaa aat aca gat ttg gat ttc cgt cag gga aaa caa      672
Ile Val Asp Ile Lys Asn Thr Asp Leu Asp Phe Arg Gln Gly Lys Gln
    210                 215                 220

tta tca aat gcc ctc aat tct aat atg gaa caa gtt caa tta gtt aaa      720
Leu Ser Asn Ala Leu Asn Ser Asn Met Glu Gln Val Gln Leu Val Lys
225                 230                 235                 240

gga att gac cac cct ttc ttg tta gac cag tta gga ctt gat aaa gaa      768
Gly Ile Asp His Pro Phe Leu Leu Asp Gln Leu Gly Leu Asp Lys Glu
                245                 250                 255

caa gct cgt tta acg ctt gat gat act tca att tca gtc ttt act gac      816
Gln Ala Arg Leu Thr Leu Asp Asp Thr Ser Ile Ser Val Phe Thr Asp
            260                 265                 270

caa cca agt att gtg ata ttt aca gca aat ttt ggt gat tta gga act      864
Gln Pro Ser Ile Val Ile Phe Thr Ala Asn Phe Gly Asp Leu Gly Thr
        275                 280                 285

ctt tat cat ggg aaa aag caa gtg cac cat ggt gga att act ttt gaa      912
Leu Tyr His Gly Lys Lys Gln Val His His Gly Gly Ile Thr Phe Glu
    290                 295                 300

tgt caa gtt tca cct ggg tct gag caa att cca gaa ctt gga gat atc      960
Cys Gln Val Ser Pro Gly Ser Glu Gln Ile Pro Glu Leu Gly Asp Ile
305                 310                 315                 320

agc cta aaa gcg gga gaa aaa tat caa gca acc aca att tac agc ttg     1008
Ser Leu Lys Ala Gly Glu Lys Tyr Gln Ala Thr Thr Ile Tyr Ser Leu
                325                 330                 335

cat aca aaa taa                                                     1020
His Thr Lys

<210>  22
<211>  339
<212>  PRT
<213>  Lactococcus lactis subsp. lactis NCDO 2118

<400>  22

Met Glu Ile Lys Ile Arg Asp Phe Gly Leu Gly Ser Asp Leu Ile Ser
1               5                   10                  15

Leu Thr Asn Lys Ala Gly Val Thr Ile Ser Phe Thr Asn Leu Gly Ala
            20                  25                  30

Arg Ile Val Asp Trp Gln Lys Asp Gly Lys His Leu Ile Leu Gly Phe
        35                  40                  45

Asp Ser Ala Lys Glu Tyr Leu Glu Lys Asp Ala Tyr Pro Gly Ala Thr
    50                  55                  60

Val Gly Pro Thr Ala Gly Arg Ile Lys Asp Gly Leu Val Lys Ile Ser
65                  70                  75                  80

Gly Lys Asp Tyr Ile Leu Asn Gln Asn Glu Gly Pro Gln Thr Leu His
                85                  90                  95

Gly Gly Glu Glu Ser Ile His Thr Lys Leu Trp Thr Tyr Glu Val Thr
            100                 105                 110

Asp Leu Gly Ala Glu Val Gln Val Lys Phe Ser Leu Val Ser Asn Asp
        115                 120                 125

Gly Thr Asn Gly Tyr Pro Gly Lys Ile Glu Met Ser Val Thr His Ser
    130                 135                 140

Phe Asp Glu Asp Asn Lys Trp Lys Ile His Tyr Glu Ala Ile Ser Asp
145                 150                 155                 160

Lys Asp Thr Val Phe Asn Pro Thr Gly His Val Tyr Phe Asn Leu Asn
                165                 170                 175

Gly Asp Ala Ser Glu Ser Val Glu Asn His Gly Leu Arg Leu Ala Ala
            180                 185                 190

Ser Arg Phe Val Pro Leu Lys Asp Gln Thr Glu Ile Val Arg Gly Asp
        195                 200                 205

Ile Val Asp Ile Lys Asn Thr Asp Leu Asp Phe Arg Gln Gly Lys Gln
    210                 215                 220

Leu Ser Asn Ala Leu Asn Ser Asn Met Glu Gln Val Gln Leu Val Lys
225                 230                 235                 240

Gly Ile Asp His Pro Phe Leu Leu Asp Gln Leu Gly Leu Asp Lys Glu
                245                 250                 255

Gln Ala Arg Leu Thr Leu Asp Asp Thr Ser Ile Ser Val Phe Thr Asp
            260                 265                 270

Gln Pro Ser Ile Val Ile Phe Thr Ala Asn Phe Gly Asp Leu Gly Thr
        275                 280                 285

Leu Tyr His Gly Lys Lys Gln Val His His Gly Gly Ile Thr Phe Glu
    290                 295                 300

Cys Gln Val Ser Pro Gly Ser Glu Gln Ile Pro Glu Leu Gly Asp Ile
305                 310                 315                 320

Ser Leu Lys Ala Gly Glu Lys Tyr Gln Ala Thr Thr Ile Tyr Ser Leu
                325                 330                 335

His Thr Lys

<210>  23
<211>  1020
<212>  DNA
<213>  Lactococcus lactis subsp. cremoris strain JM1

<220>
<221>  CDS
<222>  (1)..(1020)
<223>  galM gene encoding Aldose 1-epimerase

<300>
<308>  GanBank CP015899.1
<309>  2017-04-04
<313>  (306262)..(307281)

<400>  23
atg gaa act aca aca aaa gat ttt ggt tta gga agt agc cta att agt      48
Met Glu Thr Thr Thr Lys Asp Phe Gly Leu Gly Ser Ser Leu Ile Ser
1               5                   10                  15

cta acg aac aaa aat gat gtt aca att gct ttt aca aac tta gga gct      96
Leu Thr Asn Lys Asn Asp Val Thr Ile Ala Phe Thr Asn Leu Gly Ala
            20                  25                  30

cgc att gtt gat tgg caa aaa gac ggg aaa cat ttt gtt tta gga ttt      144
Arg Ile Val Asp Trp Gln Lys Asp Gly Lys His Phe Val Leu Gly Phe
        35                  40                  45

gat tcc gcc caa gaa tac tta gaa aaa gat gct tat cca ggg gcc aca      192

Asp Ser Ala Gln Glu Tyr Leu Glu Lys Asp Ala Tyr Pro Gly Ala Thr
    50                  55                  60

gtt gga cgt aca gct ggc aga atc aaa gat ggt ttg gtt gat att tct      240
Val Gly Arg Thr Ala Gly Arg Ile Lys Asp Gly Leu Val Asp Ile Ser
65                  70                  75                  80

gga aaa aca tat cat tta aat caa aat gaa gct ccc caa acc tta cat      288
Gly Lys Thr Tyr His Leu Asn Gln Asn Glu Ala Pro Gln Thr Leu His
                85                  90                  95

ggt gga gaa gat agc att cat acc aaa ctt tgg act tat gaa att aat      336
Gly Gly Glu Asp Ser Ile His Thr Lys Leu Trp Thr Tyr Glu Ile Asn
            100                 105                 110

gat tta gga gat gaa gtc caa gta aaa ttt agc tta gtt tcc aat gat      384
Asp Leu Gly Asp Glu Val Gln Val Lys Phe Ser Leu Val Ser Asn Asp
        115                 120                 125

gga gaa aat gga tat cct ggt aaa ata gag atg tcg gtg act cat tct      432
Gly Glu Asn Gly Tyr Pro Gly Lys Ile Glu Met Ser Val Thr His Ser
    130                 135                 140

ttt gat gaa gag aac aat tgg aaa att aag tat gaa gca att tct gac      480
Phe Asp Glu Glu Asn Asn Trp Lys Ile Lys Tyr Glu Ala Ile Ser Asp
145                 150                 155                 160

aaa gat acg gta ttt aat ccc aca gga cat gtc tat ttc aac tta aat      528
Lys Asp Thr Val Phe Asn Pro Thr Gly His Val Tyr Phe Asn Leu Asn
                165                 170                 175

ggg gat gct agc aag tct gtt gaa aat cat caa ctc aag ctt gct gct      576
Gly Asp Ala Ser Lys Ser Val Glu Asn His Gln Leu Lys Leu Ala Ala
            180                 185                 190

tca aga ttt gta cct tta aaa gat caa aca gaa att gtc cgt ggc gat      624
Ser Arg Phe Val Pro Leu Lys Asp Gln Thr Glu Ile Val Arg Gly Asp
        195                 200                 205

atc gtt gat ata aaa aat act gac tta gac ttc cgt cag gaa aaa caa      672
Ile Val Asp Ile Lys Asn Thr Asp Leu Asp Phe Arg Gln Glu Lys Gln
    210                 215                 220

tta tca aaa gcc tta gag tct agc atg gaa caa gtt caa ctg gtt ggt      720
Leu Ser Lys Ala Leu Glu Ser Ser Met Glu Gln Val Gln Leu Val Gly
225                 230                 235                 240

gga att gac cac ccc ttc tta tta gat gaa cag agc ctt gag aaa gaa      768
Gly Ile Asp His Pro Phe Leu Leu Asp Glu Gln Ser Leu Glu Lys Glu
                245                 250                 255

caa gcg cgt tta tct tta aat gac ttg tca gtt tcc gtt tat act gac      816
Gln Ala Arg Leu Ser Leu Asn Asp Leu Ser Val Ser Val Tyr Thr Asp
            260                 265                 270

caa cca agt att gta att ttt aca gca aac ttt gga gat ttg ggg acc      864
Gln Pro Ser Ile Val Ile Phe Thr Ala Asn Phe Gly Asp Leu Gly Thr
        275                 280                 285

gtt tat cac ggg aaa aat caa gtt cat cat ggt ggt att acc ttt gaa      912
Val Tyr His Gly Lys Asn Gln Val His His Gly Gly Ile Thr Phe Glu
    290                 295                 300

tgt caa gtt tca ccc ggg tca caa cag att cca gaa ctt gga gac att     960
Cys Gln Val Ser Pro Gly Ser Gln Gln Ile Pro Glu Leu Gly Asp Ile
305                 310                 315                 320

agt tta aag gcg gga gat gaa tat caa gcc acc acc att tat agt tta     1008
Ser Leu Lys Ala Gly Asp Glu Tyr Gln Ala Thr Thr Ile Tyr Ser Leu
                325                 330                 335

cat aca aat taa                                                     1020
His Thr Asn

<210>  24
<211>  339
<212>  PRT
<213>  Lactococcus lactis subsp. cremoris strain JM1

<400>  24

Met Glu Thr Thr Thr Lys Asp Phe Gly Leu Gly Ser Ser Leu Ile Ser
1               5                   10                  15

Leu Thr Asn Lys Asn Asp Val Thr Ile Ala Phe Thr Asn Leu Gly Ala
            20                  25                  30

Arg Ile Val Asp Trp Gln Lys Asp Gly Lys His Phe Val Leu Gly Phe
        35                  40                  45

Asp Ser Ala Gln Glu Tyr Leu Glu Lys Asp Ala Tyr Pro Gly Ala Thr
    50                  55                  60

Val Gly Arg Thr Ala Gly Arg Ile Lys Asp Gly Leu Val Asp Ile Ser
65                  70                  75                  80

Gly Lys Thr Tyr His Leu Asn Gln Asn Glu Ala Pro Gln Thr Leu His
                85                  90                  95

Gly Gly Glu Asp Ser Ile His Thr Lys Leu Trp Thr Tyr Glu Ile Asn
            100                 105                 110

Asp Leu Gly Asp Glu Val Gln Val Lys Phe Ser Leu Val Ser Asn Asp
        115                 120                 125

Gly Glu Asn Gly Tyr Pro Gly Lys Ile Glu Met Ser Val Thr His Ser
    130                 135                 140

Phe Asp Glu Glu Asn Asn Trp Lys Ile Lys Tyr Glu Ala Ile Ser Asp
145                 150                 155                 160

Lys Asp Thr Val Phe Asn Pro Thr Gly His Val Tyr Phe Asn Leu Asn
                165                 170                 175

Gly Asp Ala Ser Lys Ser Val Glu Asn His Gln Leu Lys Leu Ala Ala
            180                 185                 190

Ser Arg Phe Val Pro Leu Lys Asp Gln Thr Glu Ile Val Arg Gly Asp
        195                 200                 205

Ile Val Asp Ile Lys Asn Thr Asp Leu Asp Phe Arg Gln Glu Lys Gln
    210                 215                 220

Leu Ser Lys Ala Leu Glu Ser Ser Met Glu Gln Val Gln Leu Val Gly
225                 230                 235                 240

Gly Ile Asp His Pro Phe Leu Leu Asp Glu Gln Ser Leu Glu Lys Glu
                245                 250                 255

Gln Ala Arg Leu Ser Leu Asn Asp Leu Ser Val Ser Val Tyr Thr Asp
            260                 265                 270

Gln Pro Ser Ile Val Ile Phe Thr Ala Asn Phe Gly Asp Leu Gly Thr
        275                 280                 285

Val Tyr His Gly Lys Asn Gln Val His His Gly Gly Ile Thr Phe Glu
    290                 295                 300

Cys Gln Val Ser Pro Gly Ser Gln Gln Ile Pro Glu Leu Gly Asp Ile
305                 310                 315                 320

Ser Leu Lys Ala Gly Asp Glu Tyr Gln Ala Thr Thr Ile Tyr Ser Leu
                325                 330                 335

His Thr Asn

<210>  25
<211>  1200
<212>  DNA
<213>  Lactococcus lactis subsp. cremoris NZ9000

<220>
<221>  CDS
<222>  (1)..(1200)
<223>  galK gene encoding galactokinase

<300>
<308>  GenBank CP002094.1
<309>  2014-01-30
<313>  (2197942)..(2199141)

<400>  25
atg tca ata gtt gtc gaa aat agt act gta tta tca gcg tta aca gaa        48

Met Ser Ile Val Val Glu Asn Ser Thr Val Leu Ser Ala Leu Thr Glu
1               5                   10                  15

aaa ttt gca gaa gtt ttt gga gat aca aaa aaa gtt gaa tac ttt ttc      96
Lys Phe Ala Glu Val Phe Gly Asp Thr Lys Lys Val Glu Tyr Phe Phe
            20                  25                  30

agt cca gga cgg att aat ctg att ggt gaa cat act gat tac aat ggg      144
Ser Pro Gly Arg Ile Asn Leu Ile Gly Glu His Thr Asp Tyr Asn Gly
        35                  40                  45

gga tat gtt ttc cca gca tca att aca atc gga acg aca gga ctt gct      192
Gly Tyr Val Phe Pro Ala Ser Ile Thr Ile Gly Thr Thr Gly Leu Ala
    50                  55                  60

cgt ttg cga gaa gat aat aaa gtc aaa ctt tac tca gaa aat ttt cct      240
Arg Leu Arg Glu Asp Asn Lys Val Lys Leu Tyr Ser Glu Asn Phe Pro
65                  70                  75                  80

aaa ttg ggc gtc atc gaa ttt gac tta gat gat gtt gaa aat aag gat      288
Lys Leu Gly Val Ile Glu Phe Asp Leu Asp Asp Val Glu Asn Lys Asp
                85                  90                  95

ggt gaa ctt tgg tct aac tat gtc aaa gga atg att gtc atg ctc aaa      336
Gly Glu Leu Trp Ser Asn Tyr Val Lys Gly Met Ile Val Met Leu Lys
            100                 105                 110

gga gca gga tat gaa att gac aaa ggt ttt gaa ctg ttg att aaa gga      384
Gly Ala Gly Tyr Glu Ile Asp Lys Gly Phe Glu Leu Leu Ile Lys Gly
        115                 120                 125

gaa att cct aca gca tca ggc ctt tcg tca tca gct tca ctt gag ctt      432
Glu Ile Pro Thr Ala Ser Gly Leu Ser Ser Ser Ala Ser Leu Glu Leu
    130                 135                 140

tta gtg ggt gtt gtt ttg gat gat tta ttt aag cta agt gtt cct cgt      480
Leu Val Gly Val Val Leu Asp Asp Leu Phe Lys Leu Ser Val Pro Arg
145                 150                 155                 160

ctt gaa ctg gtt caa ttg gga caa aaa aca gaa aat gat tat att ggt      528
Leu Glu Leu Val Gln Leu Gly Gln Lys Thr Glu Asn Asp Tyr Ile Gly
                165                 170                 175

gtc aac tcc gga att ttg gac caa ttc gcc att ggt ttt ggt gaa gtc      576
Val Asn Ser Gly Ile Leu Asp Gln Phe Ala Ile Gly Phe Gly Glu Val
            180                 185                 190

aaa aag gca atc ttg ttg gat tgc aac act tta aaa tat gaa atg gtt      624
Lys Lys Ala Ile Leu Leu Asp Cys Asn Thr Leu Lys Tyr Glu Met Val
        195                 200                 205

cct gtt gaa ctt cgt gat tat gat att gtt atc atg aac act aac aaa      672
Pro Val Glu Leu Arg Asp Tyr Asp Ile Val Ile Met Asn Thr Asn Lys
    210                 215                 220

ccg cgg gca ttg act gaa tca aaa tac aac gaa cgt ttt gct gaa aca      720
Pro Arg Ala Leu Thr Glu Ser Lys Tyr Asn Glu Arg Phe Ala Glu Thr
225                 230                 235                 240

cgt gaa gca ctc aaa aga atg caa act aaa ttg gac att caa tca ctt      768
Arg Glu Ala Leu Lys Arg Met Gln Thr Lys Leu Asp Ile Gln Ser Leu
                245                 250                 255

gga gaa cta tca aat gaa gaa ttt gat gcc aat acc gat ttg ata ggt 816
Gly Glu Leu Ser Asn Glu Glu Phe Asp Ala Asn Thr Asp Leu Ile Gly
            260                 265                 270

gat gaa aca tta att aaa cgt gca cgt cat gcc gtt tat gaa aat aat 864
Asp Glu Thr Leu Ile Lys Arg Ala Arg His Ala Val Tyr Glu Asn Asn
        275                 280                 285

cgg acg aaa att gcc caa aag gca ttt gtt gct gga aat cta aca aaa 912
Arg Thr Lys Ile Ala Gln Lys Ala Phe Val Ala Gly Asn Leu Thr Lys
    290                 295                 300

ttt gga gaa ctt ctt aat gct tca cac gct tct tta aaa gat gat tac 960
Phe Gly Glu Leu Leu Asn Ala Ser His Ala Ser Leu Lys Asp Asp Tyr
305                 310                 315                 320

gaa gtc act gga ctt gaa ctt gat acc tta gca gaa act gct caa aaa 1008
Glu Val Thr Gly Leu Glu Leu Asp Thr Leu Ala Glu Thr Ala Gln Lys
                325                 330                 335

caa gcc ggc gta tta ggg gct cga atg act gga gct gga ttt ggt ggt 1056
Gln Ala Gly Val Leu Gly Ala Arg Met Thr Gly Ala Gly Phe Gly Gly
            340                 345                 350

tgt gca atc gct ctt gtt gct cat gac aat gtt tca gcc ttt gaa aaa 1104
Cys Ala Ile Ala Leu Val Ala His Asp Asn Val Ser Ala Phe Glu Lys
        355                 360                 365

gct gtc ggt gaa gtt tac gaa gaa gtt gtc ggt tat cca gca agc ttc 1152
Ala Val Gly Glu Val Tyr Glu Glu Val Val Gly Tyr Pro Ala Ser Phe
    370                 375                 380

tat gtt gct caa att ggt tct ggc tca aca aag cta gat gtt gaa taa 1200
Tyr Val Ala Gln Ile Gly Ser Gly Ser Thr Lys Leu Asp Val Glu
385                 390                 395

<210> 26
<211> 399
<212> PRT
<213> Lactococcus lactis subsp. cremoris NZ9000

<400> 26

Met Ser Ile Val Val Glu Asn Ser Thr Val Leu Ser Ala Leu Thr Glu
1               5                   10                  15

Lys Phe Ala Glu Val Phe Gly Asp Thr Lys Lys Val Glu Tyr Phe Phe
            20                  25                  30

Ser Pro Gly Arg Ile Asn Leu Ile Gly Glu His Thr Asp Tyr Asn Gly
        35                  40                  45

Gly Tyr Val Phe Pro Ala Ser Ile Thr Ile Gly Thr Thr Gly Leu Ala
    50                  55                  60

Arg Leu Arg Glu Asp Asn Lys Val Lys Leu Tyr Ser Glu Asn Phe Pro
65                  70                  75                  80

Lys Leu Gly Val Ile Glu Phe Asp Leu Asp Asp Val Glu Asn Lys Asp
                85                  90                  95

Gly Glu Leu Trp Ser Asn Tyr Val Lys Gly Met Ile Val Met Leu Lys
            100                 105                 110

Gly Ala Gly Tyr Glu Ile Asp Lys Gly Phe Glu Leu Leu Ile Lys Gly
        115                 120                 125

Glu Ile Pro Thr Ala Ser Gly Leu Ser Ser Ser Ala Ser Leu Glu Leu
    130                 135                 140

Leu Val Gly Val Val Leu Asp Asp Leu Phe Lys Leu Ser Val Pro Arg
145                 150                 155                 160

Leu Glu Leu Val Gln Leu Gly Gln Lys Thr Glu Asn Asp Tyr Ile Gly
                165                 170                 175

Val Asn Ser Gly Ile Leu Asp Gln Phe Ala Ile Gly Phe Gly Glu Val
            180                 185                 190

Lys Lys Ala Ile Leu Leu Asp Cys Asn Thr Leu Lys Tyr Glu Met Val
        195                 200                 205

Pro Val Glu Leu Arg Asp Tyr Asp Ile Val Ile Met Asn Thr Asn Lys
    210                 215                 220

Pro Arg Ala Leu Thr Glu Ser Lys Tyr Asn Glu Arg Phe Ala Glu Thr
225                 230                 235                 240

Arg Glu Ala Leu Lys Arg Met Gln Thr Lys Leu Asp Ile Gln Ser Leu
                245                 250                 255

Gly Glu Leu Ser Asn Glu Glu Phe Asp Ala Asn Thr Asp Leu Ile Gly
            260                 265                 270

Asp Glu Thr Leu Ile Lys Arg Ala Arg His Ala Val Tyr Glu Asn Asn
        275                 280                 285

Arg Thr Lys Ile Ala Gln Lys Ala Phe Val Ala Gly Asn Leu Thr Lys
    290                 295                 300

Phe Gly Glu Leu Leu Asn Ala Ser His Ala Ser Leu Lys Asp Asp Tyr
305                 310                 315                 320

Glu Val Thr Gly Leu Glu Leu Asp Thr Leu Ala Glu Thr Ala Gln Lys
                325                 330                 335

Gln Ala Gly Val Leu Gly Ala Arg Met Thr Gly Ala Gly Phe Gly Gly
            340                 345                 350

Cys Ala Ile Ala Leu Val Ala His Asp Asn Val Ser Ala Phe Glu Lys
        355                 360                 365

Ala Val Gly Glu Val Tyr Glu Glu Val Val Gly Tyr Pro Ala Ser Phe
    370                 375                 380

Tyr Val Ala Gln Ile Gly Ser Gly Ser Thr Lys Leu Asp Val Glu
385                 390                 395

<210>  27
<211>  1200
<212>  DNA
<213>  Lactococcus lactis subsp. lactis strain UC063

<220>
<221>  CDS
<222>  (1)..(1200)
<223>  galK gene encoding galactokinase

<300>
<308>  GenBank CP015905.1
<309>  2017-04-04
<313>  (2008730)..(2009929)

<400>  27
atg tct ata gtt gta gaa aat agt act gta tta tca gca tta aca gaa      48
Met Ser Ile Val Val Glu Asn Ser Thr Val Leu Ser Ala Leu Thr Glu
1               5                   10                  15

aaa ttt gca gaa gtt ttt gga gat aca aaa gaa gtt gaa tac ttt ttt      96
Lys Phe Ala Glu Val Phe Gly Asp Thr Lys Glu Val Glu Tyr Phe Phe
            20                  25                  30

agc cca gga cga att aat ttg att ggt gaa cat acc gat tac aat ggg      144
Ser Pro Gly Arg Ile Asn Leu Ile Gly Glu His Thr Asp Tyr Asn Gly
        35                  40                  45

gga tat gtt ttc cca gca tca att ata att gga aca aca gga cta gct      192
Gly Tyr Val Phe Pro Ala Ser Ile Ile Ile Gly Thr Thr Gly Leu Ala
    50                  55                  60

cgt ctg aga gaa gat aaa aaa gtc aaa ctt tac tca gaa aat ttt cct      240
Arg Leu Arg Glu Asp Lys Lys Val Lys Leu Tyr Ser Glu Asn Phe Pro
65                  70                  75                  80

aaa tta ggc gtc att gaa ttt gat tta gat gaa gtt gaa aag aaa gat      288
Lys Leu Gly Val Ile Glu Phe Asp Leu Asp Glu Val Glu Lys Lys Asp
                85                  90                  95

ggt gag ctt tgg tct aat tat gtt aaa gga atg att gtc atg ctt aaa      336
Gly Glu Leu Trp Ser Asn Tyr Val Lys Gly Met Ile Val Met Leu Lys
            100                 105                 110

gga gca gga tat gaa att gac aaa ggt ttt gaa ttg ttg ata aaa gga 384
Gly Ala Gly Tyr Glu Ile Asp Lys Gly Phe Glu Leu Leu Ile Lys Gly
        115                 120                 125

gaa att cct aca gca tct ggc ctt tca tca tca gca tca ctt gag ctt 432
Glu Ile Pro Thr Ala Ser Gly Leu Ser Ser Ser Ala Ser Leu Glu Leu
    130                 135                 140

tta gtc ggc gtt gtt ttg gat gat tta ttt aac tta aat gtt cca cgt 480
Leu Val Gly Val Val Leu Asp Asp Leu Phe Asn Leu Asn Val Pro Arg
145                 150                 155                 160

ctt gaa ttg gtt caa tta gga caa aaa aca gaa aat gat tat att ggt 528
Leu Glu Leu Val Gln Leu Gly Gln Lys Thr Glu Asn Asp Tyr Ile Gly
                165                 170                 175

gtt aat tct gga att tta gac caa ttc gct att ggt ttt ggt gaa gtc 576
Val Asn Ser Gly Ile Leu Asp Gln Phe Ala Ile Gly Phe Gly Glu Val
            180                 185                 190

aaa aaa gca atc cta ttg gac tgt aac act cta aaa tat gag atg gtt 624
Lys Lys Ala Ile Leu Leu Asp Cys Asn Thr Leu Lys Tyr Glu Met Val
        195                 200                 205

cct gtt gaa ctt cgt gat tat gat att gtt atc atg aat act aac aaa 672
Pro Val Glu Leu Arg Asp Tyr Asp Ile Val Ile Met Asn Thr Asn Lys
    210                 215                 220

ccg cgg gct ttg aca gaa tca aaa tac aac gaa cgt ttt gct gaa aca 720
Pro Arg Ala Leu Thr Glu Ser Lys Tyr Asn Glu Arg Phe Ala Glu Thr
225                 230                 235                 240

cgt gag gcg ctc aaa aga atg caa acg aga ttg gac att caa tca ctt 768
Arg Glu Ala Leu Lys Arg Met Gln Thr Arg Leu Asp Ile Gln Ser Leu
                245                 250                 255

gga gaa tta tca aat gaa gaa ttt gat gcc aat act gat ttg att ggg 816
Gly Glu Leu Ser Asn Glu Glu Phe Asp Ala Asn Thr Asp Leu Ile Gly
            260                 265                 270

gat gaa aca tta att aaa cgt gca cgt cat gcc gtt tat gaa aat aat 864
Asp Glu Thr Leu Ile Lys Arg Ala Arg His Ala Val Tyr Glu Asn Asn
        275                 280                 285

cga act aaa att gct caa aaa gca ttt gtt gca ggt aat ttg aca aaa 912
Arg Thr Lys Ile Ala Gln Lys Ala Phe Val Ala Gly Asn Leu Thr Lys
    290                 295                 300

ttt gga gaa ctt ctt aat gct tca cat gct tct ttg aaa gat gat tat 960
Phe Gly Glu Leu Leu Asn Ala Ser His Ala Ser Leu Lys Asp Asp Tyr
305                 310                 315                 320

gaa gtc aca gga ctt gaa ctt gat acc tta gca gag act gct caa aaa 1008
Glu Val Thr Gly Leu Glu Leu Asp Thr Leu Ala Glu Thr Ala Gln Lys
                325                 330                 335

caa gcc ggt gtc ttg ggg gcc aga atg act gga gcc gga ttt ggc ggt 1056
Gln Ala Gly Val Leu Gly Ala Arg Met Thr Gly Ala Gly Phe Gly Gly
            340                 345                 350

tgt gca atc gct ctt gtt gct cat gac aat gtt tca gcc ttt gaa aaa 1104
Cys Ala Ile Ala Leu Val Ala His Asp Asn Val Ser Ala Phe Glu Lys
        355                 360                 365

gcc gtc ggc caa gtt tac gaa gaa gtt gtc ggt tat cca gca agc ttt 1152
Ala Val Gly Gln Val Tyr Glu Glu Val Val Gly Tyr Pro Ala Ser Phe
    370                 375                 380

tac gtc gct caa att ggt tct ggt tca aca aag ttg gat gtt gaa taa 1200
Tyr Val Ala Gln Ile Gly Ser Gly Ser Thr Lys Leu Asp Val Glu
385                 390                 395

<210> 28
<211> 399
<212> PRT
<213> Lactococcus lactis subsp. lactis strain UC063

<400> 28

Met Ser Ile Val Val Glu Asn Ser Thr Val Leu Ser Ala Leu Thr Glu
1               5                   10                  15

Lys Phe Ala Glu Val Phe Gly Asp Thr Lys Glu Val Glu Tyr Phe Phe
            20                  25                  30

Ser Pro Gly Arg Ile Asn Leu Ile Gly Glu His Thr Asp Tyr Asn Gly
        35                  40                  45

Gly Tyr Val Phe Pro Ala Ser Ile Ile Ile Gly Thr Thr Gly Leu Ala
    50                  55                  60

Arg Leu Arg Glu Asp Lys Lys Val Lys Leu Tyr Ser Glu Asn Phe Pro
65                  70                  75                  80

Lys Leu Gly Val Ile Glu Phe Asp Leu Asp Glu Val Glu Lys Lys Asp
                85                  90                  95

Gly Glu Leu Trp Ser Asn Tyr Val Lys Gly Met Ile Val Met Leu Lys
            100                 105                 110

Gly Ala Gly Tyr Glu Ile Asp Lys Gly Phe Glu Leu Leu Ile Lys Gly
        115                 120                 125

Glu Ile Pro Thr Ala Ser Gly Leu Ser Ser Ser Ala Ser Leu Glu Leu
    130                 135                 140

Leu Val Gly Val Val Leu Asp Asp Leu Phe Asn Leu Asn Val Pro Arg
145                 150                 155                 160

Leu Glu Leu Val Gln Leu Gly Gln Lys Thr Glu Asn Asp Tyr Ile Gly
                165                 170                 175

Val Asn Ser Gly Ile Leu Asp Gln Phe Ala Ile Gly Phe Gly Glu Val
            180                 185                 190

Lys Lys Ala Ile Leu Leu Asp Cys Asn Thr Leu Lys Tyr Glu Met Val
        195                 200                 205

Pro Val Glu Leu Arg Asp Tyr Asp Ile Val Ile Met Asn Thr Asn Lys
    210                 215                 220

Pro Arg Ala Leu Thr Glu Ser Lys Tyr Asn Glu Arg Phe Ala Glu Thr
225                 230                 235                 240

Arg Glu Ala Leu Lys Arg Met Gln Thr Arg Leu Asp Ile Gln Ser Leu
                245                 250                 255

Gly Glu Leu Ser Asn Glu Glu Phe Asp Ala Asn Thr Asp Leu Ile Gly
            260                 265                 270

Asp Glu Thr Leu Ile Lys Arg Ala Arg His Ala Val Tyr Glu Asn Asn
        275                 280                 285

Arg Thr Lys Ile Ala Gln Lys Ala Phe Val Ala Gly Asn Leu Thr Lys
    290                 295                 300

Phe Gly Glu Leu Leu Asn Ala Ser His Ala Ser Leu Lys Asp Asp Tyr
305                 310                 315                 320

Glu Val Thr Gly Leu Glu Leu Asp Thr Leu Ala Glu Thr Ala Gln Lys
                325                 330                 335

Gln Ala Gly Val Leu Gly Ala Arg Met Thr Gly Ala Gly Phe Gly Gly
            340                 345                 350

Cys Ala Ile Ala Leu Val Ala His Asp Asn Val Ser Ala Phe Glu Lys
        355                 360                 365

Ala Val Gly Gln Val Tyr Glu Glu Val Val Gly Tyr Pro Ala Ser Phe
    370                 375                 380

Tyr Val Ala Gln Ile Gly Ser Gly Ser Thr Lys Leu Asp Val Glu
385                 390                 395

<210>  29
<211>  1200
<212>  DNA
<213>  Lactococcus garvieae Lg2

<220>
<221>  CDS
<222>  (1)..(1200)

<223>  galK gene encoding galactokinase

<300>
<308>  GenBank AP009333.1
<309>  2016-10-07
<313>  (1694398 )..(1695597)

<400>  29
atg aca aca atc gta gaa aac agt aaa gtt tta aca gca tta aca aac      48
Met Thr Thr Ile Val Glu Asn Ser Lys Val Leu Thr Ala Leu Thr Asn
1               5                   10                  15

agt ttt gaa aaa gtc ttt gga gat aca gaa aat gtg gaa tat ttc ttt      96
Ser Phe Glu Lys Val Phe Gly Asp Thr Glu Asn Val Glu Tyr Phe Phe
            20                  25                  30

tcg cca gga cgt att aac ttg att ggt gaa cat aca gac tat aat ggc      144
Ser Pro Gly Arg Ile Asn Leu Ile Gly Glu His Thr Asp Tyr Asn Gly
        35                  40                  45

ggc tac gtc ttc ccc gct tca atc aca att ggg aca aca ggt ctg gcc      192
Gly Tyr Val Phe Pro Ala Ser Ile Thr Ile Gly Thr Thr Gly Leu Ala
    50                  55                  60

cgt tta cgt gag gat aca aaa atc aga ctt ttt tca caa aac ttt ccg      240
Arg Leu Arg Glu Asp Thr Lys Ile Arg Leu Phe Ser Gln Asn Phe Pro
65                  70                  75                  80

gaa gca ggt ctt atc gaa ttt gat tta act gag gtc aag gaa aaa gag      288
Glu Ala Gly Leu Ile Glu Phe Asp Leu Thr Glu Val Lys Glu Lys Glu
                85                  90                  95

gat gac agt tgg tca aat tat gtt aaa gga atg att gtg atg ctt caa      336
Asp Asp Ser Trp Ser Asn Tyr Val Lys Gly Met Ile Val Met Leu Gln
            100                 105                 110

ggc gca ggc tat acc atc gat aaa ggt ttt gaa ctt ctt att aat ggt      384
Gly Ala Gly Tyr Thr Ile Asp Lys Gly Phe Glu Leu Leu Ile Asn Gly
        115                 120                 125

gaa att cca aca gct tct ggt tta tca tct tca gct tct tta gag ctt      432
Glu Ile Pro Thr Ala Ser Gly Leu Ser Ser Ser Ala Ser Leu Glu Leu
    130                 135                 140

tta gtc ggt gta gtt tta gac gac ttg ttt aac tta caa gtc cca cgt      480
Leu Val Gly Val Val Leu Asp Asp Leu Phe Asn Leu Gln Val Pro Arg
145                 150                 155                 160

ttg gag ctt gtg caa ctc gga caa aaa act gaa aat gaa ttc att ggt      528
Leu Glu Leu Val Gln Leu Gly Gln Lys Thr Glu Asn Glu Phe Ile Gly
                165                 170                 175

gtc aac tct ggt att ttg gac caa ttt gcg att ggt ttt ggt gaa gtg      576
Val Asn Ser Gly Ile Leu Asp Gln Phe Ala Ile Gly Phe Gly Glu Val
            180                 185                 190

aaa aaa gca att ttg ttg gac tgt aat act ctt aaa tat gaa atg gta      624
Lys Lys Ala Ile Leu Leu Asp Cys Asn Thr Leu Lys Tyr Glu Met Val
        195                 200                 205

ccc gta gag tta cgt gat tat gat att gtc atc atg aat aca aat aaa      672
Pro Val Glu Leu Arg Asp Tyr Asp Ile Val Ile Met Asn Thr Asn Lys
    210                 215                 220

cca cgt gct tta aca gaa tca aaa tat aat gag cgt ttt gct gaa aca     720
Pro Arg Ala Leu Thr Glu Ser Lys Tyr Asn Glu Arg Phe Ala Glu Thr
225                 230                 235                 240

cgt gaa gca ttg aaa cgt atg caa agt aag tta gct atc gaa tca ctg     768
Arg Glu Ala Leu Lys Arg Met Gln Ser Lys Leu Ala Ile Glu Ser Leu
                245                 250                 255

ggg gaa ctt tct aat gag gaa ttt gac gcc aac aca gac ttg att ggc     816
Gly Glu Leu Ser Asn Glu Glu Phe Asp Ala Asn Thr Asp Leu Ile Gly
            260                 265                 270

gat gaa acc ttg att aaa cgt gca cgc cat gcc gta tat gaa aat aac     864
Asp Glu Thr Leu Ile Lys Arg Ala Arg His Ala Val Tyr Glu Asn Asn
        275                 280                 285

cgt act aaa ata gct caa aaa gct ttt gtt gcg ggt aat ttg acg aaa     912
Arg Thr Lys Ile Ala Gln Lys Ala Phe Val Ala Gly Asn Leu Thr Lys
    290                 295                 300

ttt ggt gaa tta ctt aat gct tca cat gct tcc ctc aaa gat gat tat     960
Phe Gly Glu Leu Leu Asn Ala Ser His Ala Ser Leu Lys Asp Asp Tyr
305                 310                 315                 320

gaa gtg acg ggg att gag ctt gat acg ctg gct gaa acc gca caa aaa     1008
Glu Val Thr Gly Ile Glu Leu Asp Thr Leu Ala Glu Thr Ala Gln Lys
                325                 330                 335

caa gca ggg gtg tta ggt gca cgt atg aca ggt gca ggc ttt gga ggt     1056
Gln Ala Gly Val Leu Gly Ala Arg Met Thr Gly Ala Gly Phe Gly Gly
            340                 345                 350

tgt gca att gct tta gtt gca cat gat aaa gtc gct gat ttt gaa gaa     1104
Cys Ala Ile Ala Leu Val Ala His Asp Lys Val Ala Asp Phe Glu Glu
        355                 360                 365

gca gtt ggt gcg gaa tat gaa aaa gta gtt ggc tac cca gca agc ttc     1152
Ala Val Gly Ala Glu Tyr Glu Lys Val Val Gly Tyr Pro Ala Ser Phe
    370                 375                 380

tat gtc gcc caa atc ggt tca gga tca aca aaa ctg gat gtg gag tag     1200
Tyr Val Ala Gln Ile Gly Ser Gly Ser Thr Lys Leu Asp Val Glu
385                 390                 395


<210>  30
<211>  399
<212>  PRT
<213>  Lactococcus garvieae Lg2

<400>  30

Met Thr Thr Ile Val Glu Asn Ser Lys Val Leu Thr Ala Leu Thr Asn
1               5                   10                  15


Ser Phe Glu Lys Val Phe Gly Asp Thr Glu Asn Val Glu Tyr Phe Phe
            20                  25                  30


Ser Pro Gly Arg Ile Asn Leu Ile Gly Glu His Thr Asp Tyr Asn Gly
        35                  40                  45

Gly Tyr Val Phe Pro Ala Ser Ile Thr Ile Gly Thr Thr Gly Leu Ala
    50                  55                  60

Arg Leu Arg Glu Asp Thr Lys Ile Arg Leu Phe Ser Gln Asn Phe Pro
65                  70                  75                  80

Glu Ala Gly Leu Ile Glu Phe Asp Leu Thr Glu Val Lys Glu Lys Glu
                85                  90                  95

Asp Asp Ser Trp Ser Asn Tyr Val Lys Gly Met Ile Val Met Leu Gln
            100                 105                 110

Gly Ala Gly Tyr Thr Ile Asp Lys Gly Phe Glu Leu Leu Ile Asn Gly
        115                 120                 125

Glu Ile Pro Thr Ala Ser Gly Leu Ser Ser Ser Ala Ser Leu Glu Leu
    130                 135                 140

Leu Val Gly Val Val Leu Asp Asp Leu Phe Asn Leu Gln Val Pro Arg
145                 150                 155                 160

Leu Glu Leu Val Gln Leu Gly Gln Lys Thr Glu Asn Glu Phe Ile Gly
                165                 170                 175

Val Asn Ser Gly Ile Leu Asp Gln Phe Ala Ile Gly Phe Gly Glu Val
            180                 185                 190

Lys Lys Ala Ile Leu Leu Asp Cys Asn Thr Leu Lys Tyr Glu Met Val
        195                 200                 205

Pro Val Glu Leu Arg Asp Tyr Asp Ile Val Ile Met Asn Thr Asn Lys
    210                 215                 220

Pro Arg Ala Leu Thr Glu Ser Lys Tyr Asn Glu Arg Phe Ala Glu Thr
225                 230                 235                 240

Arg Glu Ala Leu Lys Arg Met Gln Ser Lys Leu Ala Ile Glu Ser Leu
                245                 250                 255

Gly Glu Leu Ser Asn Glu Glu Phe Asp Ala Asn Thr Asp Leu Ile Gly
            260                 265                 270

Asp Glu Thr Leu Ile Lys Arg Ala Arg His Ala Val Tyr Glu Asn Asn
        275                 280                 285

Arg Thr Lys Ile Ala Gln Lys Ala Phe Val Ala Gly Asn Leu Thr Lys

290                 295                 300

Phe Gly Glu Leu Leu Asn Ala Ser His Ala Ser Leu Lys Asp Asp Tyr
305                 310                 315                 320

Glu Val Thr Gly Ile Glu Leu Asp Thr Leu Ala Glu Thr Ala Gln Lys
                325                 330                 335

Gln Ala Gly Val Leu Gly Ala Arg Met Thr Gly Ala Gly Phe Gly Gly
            340                 345                 350

Cys Ala Ile Ala Leu Val Ala His Asp Lys Val Ala Asp Phe Glu Glu
        355                 360                 365

Ala Val Gly Ala Glu Tyr Glu Lys Val Val Gly Tyr Pro Ala Ser Phe
    370                 375                 380

Tyr Val Ala Gln Ile Gly Ser Gly Ser Thr Lys Leu Asp Val Glu
385                 390                 395

<210>  31
<211>  1482
<212>  DNA
<213>  Lactococcus lactis subsp. cremoris NZ9000

<220>
<221>  CDS
<222>  (1)..(1482)
<223>  galT gene encoding galactose-1-phosphate uridylyltransferase

<300>
<308>  GenBank CP002094.1
<309>  2014-01-30
<313>  (2196288)..(2197769)

<400>  31
atg tca att tat caa tca att caa gat ttc att agt ctt gct tta caa      48
Met Ser Ile Tyr Gln Ser Ile Gln Asp Phe Ile Ser Leu Ala Leu Gln
1               5                   10                  15

aat gga acg att gag cca ctt gat gag ctc tat cat aga aat caa ctt      96
Asn Gly Thr Ile Glu Pro Leu Asp Glu Leu Tyr His Arg Asn Gln Leu
            20                  25                  30

ctt cat ttt ctt ggt cta aat gat tgg gca gaa gtt gat aaa gag gtt      144
Leu His Phe Leu Gly Leu Asn Asp Trp Ala Glu Val Asp Lys Glu Val
        35                  40                  45

cat gaa act aat tca tta att ttg atg gac caa ctt ctg gcg att gca      192
His Glu Thr Asn Ser Leu Ile Leu Met Asp Gln Leu Leu Ala Ile Ala
    50                  55                  60

aat gaa aat aat gtg att gcg aaa ggg caa gat gaa ttt tat gaa gcc      240
Asn Glu Asn Asn Val Ile Ala Lys Gly Gln Asp Glu Phe Tyr Glu Ala
65                  70                  75                  80

gct ctg atg aat ttt atg aca cca cgt cca agt aaa att aat cat gat 288
Ala Leu Met Asn Phe Met Thr Pro Arg Pro Ser Lys Ile Asn His Asp
                85                  90                  95

ttc tgg gaa aaa tat cag gct tct cca gat gat gca act caa tat ttt 336
Phe Trp Glu Lys Tyr Gln Ala Ser Pro Asp Asp Ala Thr Gln Tyr Phe
            100                 105                 110

tat gaa tta gca cag caa gtg aat caa gta aaa act cgg gat att gca 384
Tyr Glu Leu Ala Gln Gln Val Asn Gln Val Lys Thr Arg Asp Ile Ala
        115                 120                 125

cgc aat att gct ttt agt cat tta aca aaa tat gga aaa cta gaa ata 432
Arg Asn Ile Ala Phe Ser His Leu Thr Lys Tyr Gly Lys Leu Glu Ile
    130                 135                 140

aca att aat ctc tca aaa cca gaa aaa gac ccc aaa gcg att gca gca 480
Thr Ile Asn Leu Ser Lys Pro Glu Lys Asp Pro Lys Ala Ile Ala Ala
145                 150                 155                 160

gcc aaa tta gta aag gct tcc tct tat ccg gct tgt caa ctt tgc ttg 528
Ala Lys Leu Val Lys Ala Ser Ser Tyr Pro Ala Cys Gln Leu Cys Leu
                165                 170                 175

gaa aat gaa gga ttt tat ggc cta gga aat aag cct gct cgt tcc aat 576
Glu Asn Glu Gly Phe Tyr Gly Leu Gly Asn Lys Pro Ala Arg Ser Asn
            180                 185                 190

cat cga att att caa gtt tca att aac ggc gag gac tgg ggc ttt caa 624
His Arg Ile Ile Gln Val Ser Ile Asn Gly Glu Asp Trp Gly Phe Gln
        195                 200                 205

tat tct cca tac gcc tat ttt aat gaa cac tct att tta tta aat gct 672
Tyr Ser Pro Tyr Ala Tyr Phe Asn Glu His Ser Ile Leu Leu Asn Ala
    210                 215                 220

aaa cat caa cca atg gag att aat aaa cga gct ttt gat aat ctt cta 720
Lys His Gln Pro Met Glu Ile Asn Lys Arg Ala Phe Asp Asn Leu Leu
225                 230                 235                 240

gga ttc ttg gat aaa ttt cct aac tat atg att ggt tca aat gca gat 768
Gly Phe Leu Asp Lys Phe Pro Asn Tyr Met Ile Gly Ser Asn Ala Asp
                245                 250                 255

tta cca att gtg ggt ggt tca att ttg aca cat gat cat tat caa gca 816
Leu Pro Ile Val Gly Gly Ser Ile Leu Thr His Asp His Tyr Gln Ala
            260                 265                 270

ggt cgt cat gat ttt cca atg gca aag gct gaa ctt cgt gaa act att 864
Gly Arg His Asp Phe Pro Met Ala Lys Ala Glu Leu Arg Glu Thr Ile
        275                 280                 285

gaa ctc gct cat ttt cca gaa gtt tct tgt ggg ata gtt aat tgg cca 912
Glu Leu Ala His Phe Pro Glu Val Ser Cys Gly Ile Val Asn Trp Pro
    290                 295                 300

atg tca gtc cta aga tta gca agt gaa aat caa gta gaa ctt tca aaa 960
Met Ser Val Leu Arg Leu Ala Ser Glu Asn Gln Val Glu Leu Ser Lys
305                 310                 315                 320

gct gcg gat gac ttt tta aaa aaa tgg caa gtt tat agt gat gaa agt 1008
Ala Ala Asp Asp Phe Leu Lys Lys Trp Gln Val Tyr Ser Asp Glu Ser

325                 330                 335

ctt cag att aaa gca aaa agt aca gat gga aca cct cat cat acc att      1056
Leu Gln Ile Lys Ala Lys Ser Thr Asp Gly Thr Pro His His Thr Ile
            340                 345                 350

acg ccg att gct cga att cga gat gga aaa tat gaa tta gat ttg gtc      1104
Thr Pro Ile Ala Arg Ile Arg Asp Gly Lys Tyr Glu Leu Asp Leu Val
        355                 360                 365

tta cgc gat aat aat act aat gaa aaa tat cca gat ggt att ttt cat      1152
Leu Arg Asp Asn Asn Thr Asn Glu Lys Tyr Pro Asp Gly Ile Phe His
    370                 375                 380

cca cat cca gcc cta cac cat att aaa aaa gaa aat att ggt tta att      1200
Pro His Pro Ala Leu His His Ile Lys Lys Glu Asn Ile Gly Leu Ile
385                 390                 395                 400

gaa gtg atg gga ctt gca att ttg cct gcc cgt ttg gga acg gaa ctt      1248
Glu Val Met Gly Leu Ala Ile Leu Pro Ala Arg Leu Gly Thr Glu Leu
                405                 410                 415

ttg gag gtt gaa aag tat ctt ctt aat cag gac aac caa atg gac gaa      1296
Leu Glu Val Glu Lys Tyr Leu Leu Asn Gln Asp Asn Gln Met Asp Glu
            420                 425                 430

att cat aaa gct tgg gct gaa cag ctt aaa aat gag gaa cat ttt act      1344
Ile His Lys Ala Trp Ala Glu Gln Leu Lys Asn Glu Glu His Phe Thr
        435                 440                 445

aga gaa acg gtt cat gcc act gtt caa ggg gca gta gga gaa gtg ttt      1392
Arg Glu Thr Val His Ala Thr Val Gln Gly Ala Val Gly Glu Val Phe
    450                 455                 460

gaa gaa gtc cta aaa gac gca ggt gta ttt aag gat act caa gaa gga      1440
Glu Glu Val Leu Lys Asp Ala Gly Val Phe Lys Asp Thr Gln Glu Gly
465                 470                 475                 480

cat gaa ggt ttt cgt aaa ttt att gat ttt gtg aat caa taa              1482
His Glu Gly Phe Arg Lys Phe Ile Asp Phe Val Asn Gln
                485                 490

<210>  32
<211>  493
<212>  PRT
<213>  Lactococcus lactis subsp. cremoris NZ9000

<400>  32

Met Ser Ile Tyr Gln Ser Ile Gln Asp Phe Ile Ser Leu Ala Leu Gln
1               5                   10                  15

Asn Gly Thr Ile Glu Pro Leu Asp Glu Leu Tyr His Arg Asn Gln Leu
            20                  25                  30

Leu His Phe Leu Gly Leu Asn Asp Trp Ala Glu Val Asp Lys Glu Val
        35                  40                  45

His Glu Thr Asn Ser Leu Ile Leu Met Asp Gln Leu Leu Ala Ile Ala

50                  55                  60

Asn Glu Asn Asn Val Ile Ala Lys Gly Gln Asp Glu Phe Tyr Glu Ala
65                  70                  75                  80

Ala Leu Met Asn Phe Met Thr Pro Arg Pro Ser Lys Ile Asn His Asp
                85                  90                  95

Phe Trp Glu Lys Tyr Gln Ala Ser Pro Asp Asp Ala Thr Gln Tyr Phe
            100                 105                 110

Tyr Glu Leu Ala Gln Gln Val Asn Gln Val Lys Thr Arg Asp Ile Ala
        115                 120                 125

Arg Asn Ile Ala Phe Ser His Leu Thr Lys Tyr Gly Lys Leu Glu Ile
    130                 135                 140

Thr Ile Asn Leu Ser Lys Pro Glu Lys Asp Pro Lys Ala Ile Ala Ala
145                 150                 155                 160

Ala Lys Leu Val Lys Ala Ser Ser Tyr Pro Ala Cys Gln Leu Cys Leu
                165                 170                 175

Glu Asn Glu Gly Phe Tyr Gly Leu Gly Asn Lys Pro Ala Arg Ser Asn
            180                 185                 190

His Arg Ile Ile Gln Val Ser Ile Asn Gly Glu Asp Trp Gly Phe Gln
        195                 200                 205

Tyr Ser Pro Tyr Ala Tyr Phe Asn Glu His Ser Ile Leu Leu Asn Ala
    210                 215                 220

Lys His Gln Pro Met Glu Ile Asn Lys Arg Ala Phe Asp Asn Leu Leu
225                 230                 235                 240

Gly Phe Leu Asp Lys Phe Pro Asn Tyr Met Ile Gly Ser Asn Ala Asp
                245                 250                 255

Leu Pro Ile Val Gly Gly Ser Ile Leu Thr His Asp His Tyr Gln Ala
            260                 265                 270

Gly Arg His Asp Phe Pro Met Ala Lys Ala Glu Leu Arg Glu Thr Ile
        275                 280                 285

Glu Leu Ala His Phe Pro Glu Val Ser Cys Gly Ile Val Asn Trp Pro
    290                 295                 300

Met Ser Val Leu Arg Leu Ala Ser Glu Asn Gln Val Glu Leu Ser Lys
305                 310                 315                 320

Ala Ala Asp Asp Phe Leu Lys Lys Trp Gln Val Tyr Ser Asp Glu Ser
                325                 330                 335

Leu Gln Ile Lys Ala Lys Ser Thr Asp Gly Thr Pro His His Thr Ile
            340                 345                 350

Thr Pro Ile Ala Arg Ile Arg Asp Gly Lys Tyr Glu Leu Asp Leu Val
        355                 360                 365

Leu Arg Asp Asn Asn Thr Asn Glu Lys Tyr Pro Asp Gly Ile Phe His
    370                 375                 380

Pro His Pro Ala Leu His His Ile Lys Lys Glu Asn Ile Gly Leu Ile
385                 390                 395                 400

Glu Val Met Gly Leu Ala Ile Leu Pro Ala Arg Leu Gly Thr Glu Leu
                405                 410                 415

Leu Glu Val Glu Lys Tyr Leu Leu Asn Gln Asp Asn Gln Met Asp Glu
            420                 425                 430

Ile His Lys Ala Trp Ala Glu Gln Leu Lys Asn Glu Glu His Phe Thr
        435                 440                 445

Arg Glu Thr Val His Ala Thr Val Gln Gly Ala Val Gly Glu Val Phe
    450                 455                 460

Glu Glu Val Leu Lys Asp Ala Gly Val Phe Lys Asp Thr Gln Glu Gly
465                 470                 475                 480

His Glu Gly Phe Arg Lys Phe Ile Asp Phe Val Asn Gln
                485                 490

<210>  33
<211>  1482
<212>  DNA
<213>  Lactococcus lactis subsp. cremoris KW2

<220>
<221>  CDS
<222>  (1)..(1482)
<223>  galT gene enocding galactose-1-phosphate uridylyltransferase

<300>
<308>  GenBank CP004884.1
<309>  2014-01-30
<313>  (2114080)..(2115561)

<400> 33
atg tca att tat caa tca att caa gat ttc att agt ctt gct tta caa 48
Met Ser Ile Tyr Gln Ser Ile Gln Asp Phe Ile Ser Leu Ala Leu Gln
1               5                   10                  15

aat gga acg att gag cca ctt gat gag ctc tat cat aga aat caa ctt 96
Asn Gly Thr Ile Glu Pro Leu Asp Glu Leu Tyr His Arg Asn Gln Leu
            20                  25                  30

ctt cat ttt ctt ggt cta aat gat tgg gca gaa gtt gat aaa gag gct 144
Leu His Phe Leu Gly Leu Asn Asp Trp Ala Glu Val Asp Lys Glu Ala
        35                  40                  45

cat gaa act aat tca tta att ttg atg gac caa ctt ctg gtg att gca 192
His Glu Thr Asn Ser Leu Ile Leu Met Asp Gln Leu Leu Val Ile Ala
    50                  55                  60

aat gaa aat aat gtg att gcg aaa ggg caa gat gag ttt tat gaa gcc 240
Asn Glu Asn Asn Val Ile Ala Lys Gly Gln Asp Glu Phe Tyr Glu Ala
65                  70                  75                  80

gct ctg atg aat ttt atg aca cca cgt cca agt aaa att aat cat gat 288
Ala Leu Met Asn Phe Met Thr Pro Arg Pro Ser Lys Ile Asn His Asp
                85                  90                  95

ttc tgg gaa aaa tat cag gct tct cca gat gct gca act caa tat ttt 336
Phe Trp Glu Lys Tyr Gln Ala Ser Pro Asp Ala Ala Thr Gln Tyr Phe
            100                 105                 110

tat gaa ttg gca cag caa gtg aat caa gta aaa act cgg gat att gca 384
Tyr Glu Leu Ala Gln Gln Val Asn Gln Val Lys Thr Arg Asp Ile Ala
        115                 120                 125

cgc aat ata gct ttt agt cat ttg aca aaa tat gga aaa ctt gaa ata 432
Arg Asn Ile Ala Phe Ser His Leu Thr Lys Tyr Gly Lys Leu Glu Ile
    130                 135                 140

aca att aat ctc tca aaa cca gaa aaa gac ccc aaa gcg att gca gca 480
Thr Ile Asn Leu Ser Lys Pro Glu Lys Asp Pro Lys Ala Ile Ala Ala
145                 150                 155                 160

gcc aaa tta gta aag gct tcc tct tat ccg gtt tgt caa ctt tgt ctg 528
Ala Lys Leu Val Lys Ala Ser Ser Tyr Pro Val Cys Gln Leu Cys Leu
                165                 170                 175

gaa aat gaa gga ttt tat ggc cta gga aat aag cct gct cgt tcc aat 576
Glu Asn Glu Gly Phe Tyr Gly Leu Gly Asn Lys Pro Ala Arg Ser Asn
            180                 185                 190

cat cga att att caa gtt tca att aac ggc gag gac tgg ggc ttt caa 624
His Arg Ile Ile Gln Val Ser Ile Asn Gly Glu Asp Trp Gly Phe Gln
        195                 200                 205

tat tct cca tac gcc tat ttt aat gaa cac tct att tta tta aat gct 672
Tyr Ser Pro Tyr Ala Tyr Phe Asn Glu His Ser Ile Leu Leu Asn Ala
    210                 215                 220

aaa cat caa cca atg gag att aat aaa cga gct ttt gat aat ctt cta 720
Lys His Gln Pro Met Glu Ile Asn Lys Arg Ala Phe Asp Asn Leu Leu
225                 230                 235                 240

gga ttc ttg gat aaa ttt cct aac tat atg att ggt tca aat gca gat 768

Gly Phe Leu Asp Lys Phe Pro Asn Tyr Met Ile Gly Ser Asn Ala Asp
                245                 250                 255

tta cca att gtg ggt ggt tca att ttg aca cat gat cat tat caa gca 816
Leu Pro Ile Val Gly Gly Ser Ile Leu Thr His Asp His Tyr Gln Ala
            260                 265                 270

ggt cgt cat gat ttt cca atg gca aag gct gaa ctt cgt gaa act att 864
Gly Arg His Asp Phe Pro Met Ala Lys Ala Glu Leu Arg Glu Thr Ile
        275                 280                 285

gaa ctc gct cat ttt cca gaa gtt tct tgt ggg ata gtt aat tgg cca 912
Glu Leu Ala His Phe Pro Glu Val Ser Cys Gly Ile Val Asn Trp Pro
    290                 295                 300

atg tca gtc cta aga tta gca agt gaa aat caa gta gaa ctt tca aaa 960
Met Ser Val Leu Arg Leu Ala Ser Glu Asn Gln Val Glu Leu Ser Lys
305                 310                 315                 320

gct gcg gat gac ttt tta aaa aaa tgg caa gtt tac agt gat gaa agt 1008
Ala Ala Asp Asp Phe Leu Lys Lys Trp Gln Val Tyr Ser Asp Glu Ser
                325                 330                 335

ctt cag att aaa gca aaa agt aca gat gga aca cct cat cat acc att 1056
Leu Gln Ile Lys Ala Lys Ser Thr Asp Gly Thr Pro His His Thr Ile
            340                 345                 350

acg ccg att gct cga att cga gat gga aaa tat gaa tta gat ttg gtc 1104
Thr Pro Ile Ala Arg Ile Arg Asp Gly Lys Tyr Glu Leu Asp Leu Val
        355                 360                 365

tta cgc gat aat aat act aat gaa aaa tat cca gat ggt att ttt cat 1152
Leu Arg Asp Asn Asn Thr Asn Glu Lys Tyr Pro Asp Gly Ile Phe His
    370                 375                 380

cca cat cca gcc cta cac cat att aaa aaa gaa aat att ggt tta att 1200
Pro His Pro Ala Leu His His Ile Lys Lys Glu Asn Ile Gly Leu Ile
385                 390                 395                 400

gaa gtg atg gga ctt gca att ttg cct gcc cgt ttg gaa acg gaa ctt 1248
Glu Val Met Gly Leu Ala Ile Leu Pro Ala Arg Leu Glu Thr Glu Leu
                405                 410                 415

ttg gag gtt gaa aag tat ctt ctt aat cag gac aac caa atg gac gaa 1296
Leu Glu Val Glu Lys Tyr Leu Leu Asn Gln Asp Asn Gln Met Asp Glu
            420                 425                 430

att cat aaa gct tgg gct gaa cag ctt aaa aat gag gaa cat ttt act 1344
Ile His Lys Ala Trp Ala Glu Gln Leu Lys Asn Glu Glu His Phe Thr
        435                 440                 445

aaa gaa acg gtt cat gcc act gtt caa ggg gca gta gga gaa gtg ttt 1392
Lys Glu Thr Val His Ala Thr Val Gln Gly Ala Val Gly Glu Val Phe
    450                 455                 460

gaa gaa gtc cta aaa gac gca ggt gta ttt aag gat act caa gaa gga 1440
Glu Glu Val Leu Lys Asp Ala Gly Val Phe Lys Asp Thr Gln Glu Gly
465                 470                 475                 480

cat gaa ggt ttt cgt aaa ttt att gat ttt gtg aat caa taa 1482
His Glu Gly Phe Arg Lys Phe Ile Asp Phe Val Asn Gln
                485                 490

<210>  34
<211>  493
<212>  PRT
<213>  Lactococcus lactis subsp. cremoris KW2

<400>  34

Met Ser Ile Tyr Gln Ser Ile Gln Asp Phe Ile Ser Leu Ala Leu Gln
1               5                   10                  15

Asn Gly Thr Ile Glu Pro Leu Asp Glu Leu Tyr His Arg Asn Gln Leu
            20                  25                  30

Leu His Phe Leu Gly Leu Asn Asp Trp Ala Glu Val Asp Lys Glu Ala
        35                  40                  45

His Glu Thr Asn Ser Leu Ile Leu Met Asp Gln Leu Leu Val Ile Ala
    50                  55                  60

Asn Glu Asn Asn Val Ile Ala Lys Gly Gln Asp Glu Phe Tyr Glu Ala
65                  70                  75                  80

Ala Leu Met Asn Phe Met Thr Pro Arg Pro Ser Lys Ile Asn His Asp
                85                  90                  95

Phe Trp Glu Lys Tyr Gln Ala Ser Pro Asp Ala Ala Thr Gln Tyr Phe
            100                 105                 110

Tyr Glu Leu Ala Gln Gln Val Asn Gln Val Lys Thr Arg Asp Ile Ala
        115                 120                 125

Arg Asn Ile Ala Phe Ser His Leu Thr Lys Tyr Gly Lys Leu Glu Ile
    130                 135                 140

Thr Ile Asn Leu Ser Lys Pro Glu Lys Asp Pro Lys Ala Ile Ala Ala
145                 150                 155                 160

Ala Lys Leu Val Lys Ala Ser Ser Tyr Pro Val Cys Gln Leu Cys Leu
                165                 170                 175

Glu Asn Glu Gly Phe Tyr Gly Leu Gly Asn Lys Pro Ala Arg Ser Asn
            180                 185                 190

His Arg Ile Ile Gln Val Ser Ile Asn Gly Glu Asp Trp Gly Phe Gln
        195                 200                 205

Tyr Ser Pro Tyr Ala Tyr Phe Asn Glu His Ser Ile Leu Leu Asn Ala
    210                 215                 220

Lys His Gln Pro Met Glu Ile Asn Lys Arg Ala Phe Asp Asn Leu Leu
225                 230                 235                 240

Gly Phe Leu Asp Lys Phe Pro Asn Tyr Met Ile Gly Ser Asn Ala Asp
                245                 250                 255

Leu Pro Ile Val Gly Gly Ser Ile Leu Thr His Asp His Tyr Gln Ala
            260                 265                 270

Gly Arg His Asp Phe Pro Met Ala Lys Ala Glu Leu Arg Glu Thr Ile
        275                 280                 285

Glu Leu Ala His Phe Pro Glu Val Ser Cys Gly Ile Val Asn Trp Pro
    290                 295                 300

Met Ser Val Leu Arg Leu Ala Ser Glu Asn Gln Val Glu Leu Ser Lys
305                 310                 315                 320

Ala Ala Asp Asp Phe Leu Lys Lys Trp Gln Val Tyr Ser Asp Glu Ser
                325                 330                 335

Leu Gln Ile Lys Ala Lys Ser Thr Asp Gly Thr Pro His His Thr Ile
            340                 345                 350

Thr Pro Ile Ala Arg Ile Arg Asp Gly Lys Tyr Glu Leu Asp Leu Val
        355                 360                 365

Leu Arg Asp Asn Asn Thr Asn Glu Lys Tyr Pro Asp Gly Ile Phe His
    370                 375                 380

Pro His Pro Ala Leu His His Ile Lys Lys Glu Asn Ile Gly Leu Ile
385                 390                 395                 400

Glu Val Met Gly Leu Ala Ile Leu Pro Ala Arg Leu Glu Thr Glu Leu
                405                 410                 415

Leu Glu Val Glu Lys Tyr Leu Leu Asn Gln Asp Asn Gln Met Asp Glu
            420                 425                 430

Ile His Lys Ala Trp Ala Glu Gln Leu Lys Asn Glu Glu His Phe Thr
        435                 440                 445

Lys Glu Thr Val His Ala Thr Val Gln Gly Ala Val Gly Glu Val Phe
    450                 455                 460

Glu Glu Val Leu Lys Asp Ala Gly Val Phe Lys Asp Thr Gln Glu Gly
465                 470                 475                 480

His Glu Gly Phe Arg Lys Phe Ile Asp Phe Val Asn Gln
                485                 490

<210>  35
<211>  1482
<212>  DNA
<213>  Lactococcus lactis subsp. lactis strain UC77

<220>
<221>  CDS
<222>  (1)..(1482)
<223>  galT gene enocding galactose-1-phosphate uridylyltransferase

<300>
<308>  GenBank CP015906.1
<309>  2017-04-04
<313>  (2215131)..(2216612)

<400>  35
atg tca att tat caa tca att caa gat ttt att agt ctt gct tta caa      48
Met Ser Ile Tyr Gln Ser Ile Gln Asp Phe Ile Ser Leu Ala Leu Gln
1               5                   10                  15

aat gga acg att gag cca ctt gat gag ctc tat cac aga aat caa ctt      96
Asn Gly Thr Ile Glu Pro Leu Asp Glu Leu Tyr His Arg Asn Gln Leu
            20                  25                  30

ctt cat ttt ctt ggt cta aat gat tgg gca gaa gtc gat aaa gaa gct      144
Leu His Phe Leu Gly Leu Asn Asp Trp Ala Glu Val Asp Lys Glu Ala
        35                  40                  45

cac gag aaa gat tcc ttg att tta atg gac caa ctt tta gcg att gca      192
His Glu Lys Asp Ser Leu Ile Leu Met Asp Gln Leu Leu Ala Ile Ala
    50                  55                  60

aat gaa aat aat gtg att gaa aaa gga caa gat gaa ttt tat gaa gcc      240
Asn Glu Asn Asn Val Ile Glu Lys Gly Gln Asp Glu Phe Tyr Glu Ala
65                  70                  75                  80

gct ctg atg aat ttt att aca cca cgc cca agt aaa atc aat caa gat      288
Ala Leu Met Asn Phe Ile Thr Pro Arg Pro Ser Lys Ile Asn Gln Asp
                85                  90                  95

ttc tgg gaa aaa tat aag act tct cca gat gca gca act caa tat ttt      336
Phe Trp Glu Lys Tyr Lys Thr Ser Pro Asp Ala Ala Thr Gln Tyr Phe
            100                 105                 110

tat gaa ctg gca cag caa gtg aac caa gta aaa act cga gat att aca      384
Tyr Glu Leu Ala Gln Gln Val Asn Gln Val Lys Thr Arg Asp Ile Thr
        115                 120                 125

cgt aat att gcc ttt agt cat ttg aca aaa tat gga aaa ctt gaa ata      432
Arg Asn Ile Ala Phe Ser His Leu Thr Lys Tyr Gly Lys Leu Glu Ile
    130                 135                 140

acg att aat ctc tca aaa cca gaa aaa gac ccc aaa gcc att gcg gca      480
Thr Ile Asn Leu Ser Lys Pro Glu Lys Asp Pro Lys Ala Ile Ala Ala
145                 150                 155                 160

gct aaa tta gta aag gct tcc tct tat cca gcc tgt caa ctt tgt ttg 528
Ala Lys Leu Val Lys Ala Ser Ser Tyr Pro Ala Cys Gln Leu Cys Leu
                165                 170                 175

gaa aac gaa gga ttt tat ggc ata gga aat aaa cct gct cgt tct aat 576
Glu Asn Glu Gly Phe Tyr Gly Ile Gly Asn Lys Pro Ala Arg Ser Asn
            180                 185                 190

cat cga atc att caa gtc tca att aac ggc gaa gac tgg ggc ttc caa 624
His Arg Ile Ile Gln Val Ser Ile Asn Gly Glu Asp Trp Gly Phe Gln
        195                 200                 205

tat tct cct tat gcc tat ttt aat gaa cac tcg att tta cta aat gct 672
Tyr Ser Pro Tyr Ala Tyr Phe Asn Glu His Ser Ile Leu Leu Asn Ala
    210                 215                 220

aaa cac caa cca atg gaa att aat aaa cgt gct ttt gat aat tta ttg 720
Lys His Gln Pro Met Glu Ile Asn Lys Arg Ala Phe Asp Asn Leu Leu
225                 230                 235                 240

gga ttt cta gat caa ttt cct gat tat atg att ggt tct aat gca gat 768
Gly Phe Leu Asp Gln Phe Pro Asp Tyr Met Ile Gly Ser Asn Ala Asp
                245                 250                 255

tta cca att gta gga ggg tca att ttg act cat gac cat tat caa gct 816
Leu Pro Ile Val Gly Gly Ser Ile Leu Thr His Asp His Tyr Gln Ala
            260                 265                 270

gga cgt tat gaa ttt ccg atg gca aaa gct gaa ctt cga gaa gaa att 864
Gly Arg Tyr Glu Phe Pro Met Ala Lys Ala Glu Leu Arg Glu Glu Ile
        275                 280                 285

aaa ctt caa caa ttt cca gaa gtt tct tgc gga att gtg aat tgg cca 912
Lys Leu Gln Gln Phe Pro Glu Val Ser Cys Gly Ile Val Asn Trp Pro
    290                 295                 300

atg tct gtt cta aga tta tca agt aaa aac caa gta gaa ctt tca aaa 960
Met Ser Val Leu Arg Leu Ser Ser Lys Asn Gln Val Glu Leu Ser Lys
305                 310                 315                 320

gct gca gat gaa ctt tta aaa aaa tgg caa gtt tat agt gat gaa agt 1008
Ala Ala Asp Glu Leu Leu Lys Lys Trp Gln Val Tyr Ser Asp Glu Ser
                325                 330                 335

ctt caa atc aaa gct aaa agt gct gac gga aag ctt cat cat aca att 1056
Leu Gln Ile Lys Ala Lys Ser Ala Asp Gly Lys Leu His His Thr Ile
            340                 345                 350

act ccg att gct cga att cga gag ggg aaa tat gaa tta gat tta gtt 1104
Thr Pro Ile Ala Arg Ile Arg Glu Gly Lys Tyr Glu Leu Asp Leu Val
        355                 360                 365

tta cga gat aat aat aca agt gaa att tat cca gat gga att ttt cat 1152
Leu Arg Asp Asn Asn Thr Ser Glu Ile Tyr Pro Asp Gly Ile Phe His
    370                 375                 380

cca cat cca gcc tta cac cat att aaa aaa gaa aat att gga ttg att 1200
Pro His Pro Ala Leu His His Ile Lys Lys Glu Asn Ile Gly Leu Ile
385                 390                 395                 400

gaa gtg atg gga ctt gca att tta cca gcc agg tta aag aca gaa ctt 1248
Glu Val Met Gly Leu Ala Ile Leu Pro Ala Arg Leu Lys Thr Glu Leu
                405                 410                 415

ttg gaa gtt gaa aaa tat ctc ttg aat caa gaa aat gag atg tca gaa 1296
Leu Glu Val Glu Lys Tyr Leu Leu Asn Gln Glu Asn Glu Met Ser Glu
            420                 425                 430

att cac aaa cca tgg gct gaa gaa ttg aaa gaa aca gaa tat ttt tca 1344
Ile His Lys Pro Trp Ala Glu Glu Leu Lys Glu Thr Glu Tyr Phe Ser
        435                 440                 445

aaa gaa aca gtt cat gaa aca gtt cag gaa gca gtt gga gaa gta ttt 1392
Lys Glu Thr Val His Glu Thr Val Gln Glu Ala Val Gly Glu Val Phe
    450                 455                 460

gaa cag gtg tta aaa gat gcc gga gta ttc aaa gat aat gaa gta gga 1440
Glu Gln Val Leu Lys Asp Ala Gly Val Phe Lys Asp Asn Glu Val Gly
465                 470                 475                 480

caa aaa ggt ttt tat gaa ttt ata aat ttc gta aat aat taa 1482
Gln Lys Gly Phe Tyr Glu Phe Ile Asn Phe Val Asn Asn
                485                 490

<210> 36
<211> 493
<212> PRT
<213> Lactococcus lactis subsp. lactis strain UC77

<400> 36

Met Ser Ile Tyr Gln Ser Ile Gln Asp Phe Ile Ser Leu Ala Leu Gln
1               5                   10                  15

Asn Gly Thr Ile Glu Pro Leu Asp Glu Leu Tyr His Arg Asn Gln Leu
            20                  25                  30

Leu His Phe Leu Gly Leu Asn Asp Trp Ala Glu Val Asp Lys Glu Ala
        35                  40                  45

His Glu Lys Asp Ser Leu Ile Leu Met Asp Gln Leu Leu Ala Ile Ala
    50                  55                  60

Asn Glu Asn Asn Val Ile Glu Lys Gly Gln Asp Glu Phe Tyr Glu Ala
65                  70                  75                  80

Ala Leu Met Asn Phe Ile Thr Pro Arg Pro Ser Lys Ile Asn Gln Asp
                85                  90                  95

Phe Trp Glu Lys Tyr Lys Thr Ser Pro Asp Ala Ala Thr Gln Tyr Phe
            100                 105                 110

Tyr Glu Leu Ala Gln Gln Val Asn Gln Val Lys Thr Arg Asp Ile Thr
        115                 120                 125

Arg Asn Ile Ala Phe Ser His Leu Thr Lys Tyr Gly Lys Leu Glu Ile
    130                 135                 140

Thr Ile Asn Leu Ser Lys Pro Glu Lys Asp Pro Lys Ala Ile Ala Ala
145                 150                 155                 160

Ala Lys Leu Val Lys Ala Ser Ser Tyr Pro Ala Cys Gln Leu Cys Leu
                165                 170                 175

Glu Asn Glu Gly Phe Tyr Gly Ile Gly Asn Lys Pro Ala Arg Ser Asn
            180                 185                 190

His Arg Ile Ile Gln Val Ser Ile Asn Gly Glu Asp Trp Gly Phe Gln
        195                 200                 205

Tyr Ser Pro Tyr Ala Tyr Phe Asn Glu His Ser Ile Leu Leu Asn Ala
    210                 215                 220

Lys His Gln Pro Met Glu Ile Asn Lys Arg Ala Phe Asp Asn Leu Leu
225                 230                 235                 240

Gly Phe Leu Asp Gln Phe Pro Asp Tyr Met Ile Gly Ser Asn Ala Asp
                245                 250                 255

Leu Pro Ile Val Gly Gly Ser Ile Leu Thr His Asp His Tyr Gln Ala
            260                 265                 270

Gly Arg Tyr Glu Phe Pro Met Ala Lys Ala Glu Leu Arg Glu Glu Ile
        275                 280                 285

Lys Leu Gln Gln Phe Pro Glu Val Ser Cys Gly Ile Val Asn Trp Pro
    290                 295                 300

Met Ser Val Leu Arg Leu Ser Ser Lys Asn Gln Val Glu Leu Ser Lys
305                 310                 315                 320

Ala Ala Asp Glu Leu Leu Lys Lys Trp Gln Val Tyr Ser Asp Glu Ser
                325                 330                 335

Leu Gln Ile Lys Ala Lys Ser Ala Asp Gly Lys Leu His His Thr Ile
            340                 345                 350

Thr Pro Ile Ala Arg Ile Arg Glu Gly Lys Tyr Glu Leu Asp Leu Val
        355                 360                 365

Leu Arg Asp Asn Asn Thr Ser Glu Ile Tyr Pro Asp Gly Ile Phe His
    370                 375                 380

Pro His Pro Ala Leu His His Ile Lys Lys Glu Asn Ile Gly Leu Ile

385                 390                 395                 400

Glu Val Met Gly Leu Ala Ile Leu Pro Ala Arg Leu Lys Thr Glu Leu
                405                 410                 415

Leu Glu Val Glu Lys Tyr Leu Leu Asn Gln Glu Asn Glu Met Ser Glu
            420                 425                 430

Ile His Lys Pro Trp Ala Glu Glu Leu Lys Glu Thr Glu Tyr Phe Ser
        435                 440                 445

Lys Glu Thr Val His Glu Thr Val Gln Glu Ala Val Gly Glu Val Phe
    450                 455                 460

Glu Gln Val Leu Lys Asp Ala Gly Val Phe Lys Asp Asn Glu Val Gly
465                 470                 475                 480

Gln Lys Gly Phe Tyr Glu Phe Ile Asn Phe Val Asn Asn
                485                 490

<210>  37
<211>  981
<212>  DNA
<213>  Lactococcus lactis subsp. cremoris NZ9000

<220>
<221>  CDS
<222>  (1)..(981)
<223>  GalE gene encoding UDP-glucose 4-epimerase

<300>
<308>  GenBank CP002094.1
<309>  2014-01-30
<313>  (2195191)..(2196171)

<400>  37
atg aca gtt tta gta ctt ggt gga gca gga tat gta gga agc cat gcg        48
Met Thr Val Leu Val Leu Gly Gly Ala Gly Tyr Val Gly Ser His Ala
1               5                   10                  15

gta gat atg ctt gtt aat cgt ggt tac gat gtg gca gtt gtt gat aat        96
Val Asp Met Leu Val Asn Arg Gly Tyr Asp Val Ala Val Val Asp Asn
            20                  25                  30

tta gta aca ggt cat cgc gag gct gtg tca gaa aaa gta cgt ttt tat       144
Leu Val Thr Gly His Arg Glu Ala Val Ser Glu Lys Val Arg Phe Tyr
        35                  40                  45

gag gga gat gta cgg gac cat gct ttc tta gct agt gtt ttt gaa aag       192
Glu Gly Asp Val Arg Asp His Ala Phe Leu Ala Ser Val Phe Glu Lys
    50                  55                  60

gaa aat att gaa gga att atg cat ttt tgc gct tat tca tta gtg ggt       240
Glu Asn Ile Glu Gly Ile Met His Phe Cys Ala Tyr Ser Leu Val Gly
65                  70                  75                  80

gag tca atg caa aaa cca ttg atg tat ttc aat aac aat gtc ggc gga 288
Glu Ser Met Gln Lys Pro Leu Met Tyr Phe Asn Asn Asn Val Gly Gly
                85                  90                  95

gct cag gtg att cta gaa aca atg gaa gaa ttt ggt gtt aag cat att 336
Ala Gln Val Ile Leu Glu Thr Met Glu Glu Phe Gly Val Lys His Ile
            100                 105                 110

gtt ttc tca agt acc gca gct act ttt ggt att cct gag gaa agt cca 384
Val Phe Ser Ser Thr Ala Ala Thr Phe Gly Ile Pro Glu Glu Ser Pro
        115                 120                 125

att tct gaa aaa aca cca caa aaa cca att aat cct tac ggc gaa agt 432
Ile Ser Glu Lys Thr Pro Gln Lys Pro Ile Asn Pro Tyr Gly Glu Ser
    130                 135                 140

aaa tta atc atg gaa aaa atg atg aaa tgg caa tcg caa gcc aca gat 480
Lys Leu Ile Met Glu Lys Met Met Lys Trp Gln Ser Gln Ala Thr Asp
145                 150                 155                 160

atg act tat gtt gct ctg cgc tat ttc aat gtt gct ggt gcc aaa gat 528
Met Thr Tyr Val Ala Leu Arg Tyr Phe Asn Val Ala Gly Ala Lys Asp
                165                 170                 175

gat ggg tca att ggt gaa gcc cat aaa aat gaa aca cat tta att cca 576
Asp Gly Ser Ile Gly Glu Ala His Lys Asn Glu Thr His Leu Ile Pro
            180                 185                 190

att atc cta caa act gca ctt gga caa aga gaa ttt att acc att tat 624
Ile Ile Leu Gln Thr Ala Leu Gly Gln Arg Glu Phe Ile Thr Ile Tyr
        195                 200                 205

ggt gat gat tat cag act cct gat gga aca tgt atc cgt gat tat att 672
Gly Asp Asp Tyr Gln Thr Pro Asp Gly Thr Cys Ile Arg Asp Tyr Ile
    210                 215                 220

gat atg gaa gat ttg att gaa gca cat atc aaa gca ctt gaa tat ctt 720
Asp Met Glu Asp Leu Ile Glu Ala His Ile Lys Ala Leu Glu Tyr Leu
225                 230                 235                 240

aaa tct ggt ggg aaa tct gac caa ttt aat ctt ggc tca agt aaa ggt 768
Lys Ser Gly Gly Lys Ser Asp Gln Phe Asn Leu Gly Ser Ser Lys Gly
                245                 250                 255

tat tca aat ctt gaa gtg ctt gaa aca gca cga aaa gta aca gga aaa 816
Tyr Ser Asn Leu Glu Val Leu Glu Thr Ala Arg Lys Val Thr Gly Lys
            260                 265                 270

gaa att cca agt caa atg ggt gaa cgt cgt cct ggc gat cca gat gaa 864
Glu Ile Pro Ser Gln Met Gly Glu Arg Arg Pro Gly Asp Pro Asp Glu
        275                 280                 285

ctt gta gcg gac tca aca aaa gcc ggc gaa att tta gga tgg aaa gta 912
Leu Val Ala Asp Ser Thr Lys Ala Gly Glu Ile Leu Gly Trp Lys Val
    290                 295                 300

aaa aat gat ttg gaa cac atc att aca aat gct tgg gaa tgg cat caa 960
Lys Asn Asp Leu Glu His Ile Ile Thr Asn Ala Trp Glu Trp His Gln
305                 310                 315                 320

agt cat cca aaa ggc tac tga 981
Ser His Pro Lys Gly Tyr

                325

<210>  38
<211>  326
<212>  PRT
<213>  Lactococcus lactis subsp. cremoris NZ9000

<400>  38

Met Thr Val Leu Val Leu Gly Gly Ala Gly Tyr Val Gly Ser His Ala
1               5                   10                  15

Val Asp Met Leu Val Asn Arg Gly Tyr Asp Val Ala Val Val Asp Asn
            20                  25                  30

Leu Val Thr Gly His Arg Glu Ala Val Ser Glu Lys Val Arg Phe Tyr
        35                  40                  45

Glu Gly Asp Val Arg Asp His Ala Phe Leu Ala Ser Val Phe Glu Lys
    50                  55                  60

Glu Asn Ile Glu Gly Ile Met His Phe Cys Ala Tyr Ser Leu Val Gly
65                  70                  75                  80

Glu Ser Met Gln Lys Pro Leu Met Tyr Phe Asn Asn Asn Val Gly Gly
                85                  90                  95

Ala Gln Val Ile Leu Glu Thr Met Glu Glu Phe Gly Val Lys His Ile
            100                 105                 110

Val Phe Ser Ser Thr Ala Ala Thr Phe Gly Ile Pro Glu Glu Ser Pro
        115                 120                 125

Ile Ser Glu Lys Thr Pro Gln Lys Pro Ile Asn Pro Tyr Gly Glu Ser
    130                 135                 140

Lys Leu Ile Met Glu Lys Met Met Lys Trp Gln Ser Gln Ala Thr Asp
145                 150                 155                 160

Met Thr Tyr Val Ala Leu Arg Tyr Phe Asn Val Ala Gly Ala Lys Asp
                165                 170                 175

Asp Gly Ser Ile Gly Glu Ala His Lys Asn Glu Thr His Leu Ile Pro
            180                 185                 190

Ile Ile Leu Gln Thr Ala Leu Gly Gln Arg Glu Phe Ile Thr Ile Tyr
        195                 200                 205

Gly Asp Asp Tyr Gln Thr Pro Asp Gly Thr Cys Ile Arg Asp Tyr Ile

210                 215                 220

Asp Met Glu Asp Leu Ile Glu Ala His Ile Lys Ala Leu Glu Tyr Leu
225                 230                 235                 240

Lys Ser Gly Gly Lys Ser Asp Gln Phe Asn Leu Gly Ser Ser Lys Gly
                245                 250                 255

Tyr Ser Asn Leu Glu Val Leu Glu Thr Ala Arg Lys Val Thr Gly Lys
            260                 265                 270

Glu Ile Pro Ser Gln Met Gly Glu Arg Arg Pro Gly Asp Pro Asp Glu
        275                 280                 285

Leu Val Ala Asp Ser Thr Lys Ala Gly Glu Ile Leu Gly Trp Lys Val
    290                 295                 300

Lys Asn Asp Leu Glu His Ile Ile Thr Asn Ala Trp Glu Trp His Gln
305                 310                 315                 320

Ser His Pro Lys Gly Tyr
                325

<210>  39
<211>  981
<212>  DNA
<213>  Lactococcus lactis strain AI06

<220>
<221>  CDS
<222>  (1)..(981)
<223>  galE gene encoding UDP-glucose 4-epimerase

<300>
<308>  GenBank CP009472.1
<309>  2014-12-10
<313>  (2097681)..(2098661)

<400>  39
atg acg gtt tta gtt ctt ggt gga gca gga tat gtg gga agc cat gcg       48
Met Thr Val Leu Val Leu Gly Gly Ala Gly Tyr Val Gly Ser His Ala
1               5                   10                  15

gta gat atg ctt ctt aaa cgt ggt tac gat gtg gca gtg gtg gat aat       96
Val Asp Met Leu Leu Lys Arg Gly Tyr Asp Val Ala Val Val Asp Asn
            20                  25                  30

tta gtg aca gga cac cgt gaa tct gtt cca gca aat gta cgc ttt tac      144
Leu Val Thr Gly His Arg Glu Ser Val Pro Ala Asn Val Arg Phe Tyr
        35                  40                  45

gag ggt gat gtg cgt gat cat gct ttc tta gct agt gtt ttt gaa aaa      192
Glu Gly Asp Val Arg Asp His Ala Phe Leu Ala Ser Val Phe Glu Lys
    50                  55                  60

gaa aat att gaa gga atc atg cat ttt tgt gct tac tct tta gtg ggt 240
Glu Asn Ile Glu Gly Ile Met His Phe Cys Ala Tyr Ser Leu Val Gly
65                  70                  75                  80

gag tca atg caa aaa cct tta atg tat ttt aat aat aat gtc ggt ggt 288
Glu Ser Met Gln Lys Pro Leu Met Tyr Phe Asn Asn Asn Val Gly Gly
                85                  90                  95

gct caa gta att cta gaa acg atg gaa gaa ttc gga gta aaa cat att 336
Ala Gln Val Ile Leu Glu Thr Met Glu Glu Phe Gly Val Lys His Ile
            100                 105                 110

gtt ttc tca agt aca gca gct aca ttt gga att cca gaa aaa agt cca 384
Val Phe Ser Ser Thr Ala Ala Thr Phe Gly Ile Pro Glu Lys Ser Pro
        115                 120                 125

att tca gaa aaa aca cct caa aaa cca att aac cct tat ggt gaa agc 432
Ile Ser Glu Lys Thr Pro Gln Lys Pro Ile Asn Pro Tyr Gly Glu Ser
    130                 135                 140

aaa tta atc atg gaa aaa atg atg aaa tgg caa tcg caa gca act gat 480
Lys Leu Ile Met Glu Lys Met Met Lys Trp Gln Ser Gln Ala Thr Asp
145                 150                 155                 160

atg act tat gtt gct ttg cgt tat ttc aat gtt gct ggg gct aaa gat 528
Met Thr Tyr Val Ala Leu Arg Tyr Phe Asn Val Ala Gly Ala Lys Asp
                165                 170                 175

gat ggt tca att ggt gaa gct cat aaa aat gaa aca cac ttg att cca 576
Asp Gly Ser Ile Gly Glu Ala His Lys Asn Glu Thr His Leu Ile Pro
            180                 185                 190

att att tta caa aca gca ctt ggt caa agg gaa ttg att act att tat 624
Ile Ile Leu Gln Thr Ala Leu Gly Gln Arg Glu Leu Ile Thr Ile Tyr
        195                 200                 205

ggt gat gac tat cac acg cct gat gga act tgt att cgt gat tat att 672
Gly Asp Asp Tyr His Thr Pro Asp Gly Thr Cys Ile Arg Asp Tyr Ile
    210                 215                 220

gat atg gaa gat ttg att gaa gca cac atc aaa gca ctg gag tat ctt 720
Asp Met Glu Asp Leu Ile Glu Ala His Ile Lys Ala Leu Glu Tyr Leu
225                 230                 235                 240

aaa tcc ggt ggt cag tct gac caa ttt aat ctc ggt tta agt aag ggt 768
Lys Ser Gly Gly Gln Ser Asp Gln Phe Asn Leu Gly Leu Ser Lys Gly
                245                 250                 255

tat tca aat ctt gaa gtg ctt gaa acc gca cga aaa gta aca ggt aaa 816
Tyr Ser Asn Leu Glu Val Leu Glu Thr Ala Arg Lys Val Thr Gly Lys
            260                 265                 270

gaa att cca agc caa atg ggt gaa cgt cgt gcg gga gac cca gat gaa 864
Glu Ile Pro Ser Gln Met Gly Glu Arg Arg Ala Gly Asp Pro Asp Glu
        275                 280                 285

ctc gta gca gat tca aca aaa gct ggt gaa att ttg ggt tgg aaa gca 912
Leu Val Ala Asp Ser Thr Lys Ala Gly Glu Ile Leu Gly Trp Lys Ala
    290                 295                 300

caa aat gat tta gag cac att att acg aac gct tgg aaa tgg cac caa 960
Gln Asn Asp Leu Glu His Ile Ile Thr Asn Ala Trp Lys Trp His Gln

305                 310                 315                 320

agt cat ccg aaa ggt tat tga                                         981
Ser His Pro Lys Gly Tyr
                325

<210>  40
<211>  326
<212>  PRT
<213>  Lactococcus lactis strain AI06

<400>  40

Met Thr Val Leu Val Leu Gly Gly Ala Gly Tyr Val Gly Ser His Ala
1               5                   10                  15

Val Asp Met Leu Leu Lys Arg Gly Tyr Asp Val Ala Val Val Asp Asn
            20                  25                  30

Leu Val Thr Gly His Arg Glu Ser Val Pro Ala Asn Val Arg Phe Tyr
        35                  40                  45

Glu Gly Asp Val Arg Asp His Ala Phe Leu Ala Ser Val Phe Glu Lys
    50                  55                  60

Glu Asn Ile Glu Gly Ile Met His Phe Cys Ala Tyr Ser Leu Val Gly
65                  70                  75                  80

Glu Ser Met Gln Lys Pro Leu Met Tyr Phe Asn Asn Asn Val Gly Gly
                85                  90                  95

Ala Gln Val Ile Leu Glu Thr Met Glu Glu Phe Gly Val Lys His Ile
            100                 105                 110

Val Phe Ser Ser Thr Ala Ala Thr Phe Gly Ile Pro Glu Lys Ser Pro
        115                 120                 125

Ile Ser Glu Lys Thr Pro Gln Lys Pro Ile Asn Pro Tyr Gly Glu Ser
    130                 135                 140

Lys Leu Ile Met Glu Lys Met Met Lys Trp Gln Ser Gln Ala Thr Asp
145                 150                 155                 160

Met Thr Tyr Val Ala Leu Arg Tyr Phe Asn Val Ala Gly Ala Lys Asp
                165                 170                 175

Asp Gly Ser Ile Gly Glu Ala His Lys Asn Glu Thr His Leu Ile Pro
            180                 185                 190

Ile Ile Leu Gln Thr Ala Leu Gly Gln Arg Glu Leu Ile Thr Ile Tyr

195                 200                 205

Gly Asp Asp Tyr His Thr Pro Asp Gly Thr Cys Ile Arg Asp Tyr Ile
    210                 215                 220

Asp Met Glu Asp Leu Ile Glu Ala His Ile Lys Ala Leu Glu Tyr Leu
225                 230                 235                 240

Lys Ser Gly Gly Gln Ser Asp Gln Phe Asn Leu Gly Leu Ser Lys Gly
                245                 250                 255

Tyr Ser Asn Leu Glu Val Leu Glu Thr Ala Arg Lys Val Thr Gly Lys
            260                 265                 270

Glu Ile Pro Ser Gln Met Gly Glu Arg Arg Ala Gly Asp Pro Asp Glu
        275                 280                 285

Leu Val Ala Asp Ser Thr Lys Ala Gly Glu Ile Leu Gly Trp Lys Ala
    290                 295                 300

Gln Asn Asp Leu Glu His Ile Ile Thr Asn Ala Trp Lys Trp His Gln
305                 310                 315                 320

Ser His Pro Lys Gly Tyr
                325

<210>  41
<211>  984
<212>  DNA
<213>  Lactococcus garvieae Lg2

<220>
<221>  CDS
<222>  (1)..(984)
<223>  galE gene encoding UDP-glucose 4-epimerase

<300>
<308>  GenBank AP009333.1
<309>  2016-10-07
<313>  (1691853)..(1692836)

<400>  41
atg aca gtc tta gta ctt gga ggc gca ggc tat gta gga agc cat gct      48
Met Thr Val Leu Val Leu Gly Gly Ala Gly Tyr Val Gly Ser His Ala
1               5                   10                  15

gtt gat att cta gtg gca cgt ggt tat gat gtg gcc gtt gta gat aat      96
Val Asp Ile Leu Val Ala Arg Gly Tyr Asp Val Ala Val Val Asp Asn
            20                  25                  30

ctt gtg aca gga cat cga gaa gct gtc cca gca gat gta cgc ttc tat      144
Leu Val Thr Gly His Arg Glu Ala Val Pro Ala Asp Val Arg Phe Tyr
        35                  40                  45

gaa gga gat gtg cgc gat caa gct ttt ttg gct gat gtt ttt aca aaa 192
Glu Gly Asp Val Arg Asp Gln Ala Phe Leu Ala Asp Val Phe Thr Lys
    50                  55                  60

gaa aaa gat att gaa ggc tta atg cat ttt tgt gcc tat tct ttg gtg 240
Glu Lys Asp Ile Glu Gly Leu Met His Phe Cys Ala Tyr Ser Leu Val
65                  70                  75                  80

ggt gaa tca atg caa aaa cca ctg atg tat ttt aat aat aac gtt ggt 288
Gly Glu Ser Met Gln Lys Pro Leu Met Tyr Phe Asn Asn Asn Val Gly
                85                  90                  95

ggg gca cag gtt atc ctt gaa acc atg gaa aaa ttt ggt atc aag cat 336
Gly Ala Gln Val Ile Leu Glu Thr Met Glu Lys Phe Gly Ile Lys His
            100                 105                 110

att gtc ttt tca agt acg gct gca act ttt ggg ata cca gaa gaa agt 384
Ile Val Phe Ser Ser Thr Ala Ala Thr Phe Gly Ile Pro Glu Glu Ser
        115                 120                 125

cct att tct gaa aaa act cca caa aaa cca att aat cct tac ggg gaa 432
Pro Ile Ser Glu Lys Thr Pro Gln Lys Pro Ile Asn Pro Tyr Gly Glu
    130                 135                 140

agc aaa ctc atc atg gaa aaa atg atg aag tgg caa tca gag gca act 480
Ser Lys Leu Ile Met Glu Lys Met Met Lys Trp Gln Ser Glu Ala Thr
145                 150                 155                 160

gat atg act tat gtg gcc ctg cgc tac ttc aat gtt gca ggt gcg aaa 528
Asp Met Thr Tyr Val Ala Leu Arg Tyr Phe Asn Val Ala Gly Ala Lys
                165                 170                 175

cat gat ggc agt att ggt gag gca cat aaa aat gaa aca cat ctg att 576
His Asp Gly Ser Ile Gly Glu Ala His Lys Asn Glu Thr His Leu Ile
            180                 185                 190

cca ctc att tta caa acg gct tta gga caa cgt gag ttt atc act atc 624
Pro Leu Ile Leu Gln Thr Ala Leu Gly Gln Arg Glu Phe Ile Thr Ile
        195                 200                 205

tat ggg gat gac tac aat acc cct gac ggt aca tgt gta cgt gac tat 672
Tyr Gly Asp Asp Tyr Asn Thr Pro Asp Gly Thr Cys Val Arg Asp Tyr
    210                 215                 220

att gac atg gaa gat ttg att gaa gca cat atc aaa gcc tta gaa tat 720
Ile Asp Met Glu Asp Leu Ile Glu Ala His Ile Lys Ala Leu Glu Tyr
225                 230                 235                 240

ctt aaa gca ggc ggt gct tcg gac cag ttt aac ctt ggc tct tca aaa 768
Leu Lys Ala Gly Gly Ala Ser Asp Gln Phe Asn Leu Gly Ser Ser Lys
                245                 250                 255

ggc tac tct aac ctt gaa gtc ttg gaa act gca cgc cgt gtt acc ggt 816
Gly Tyr Ser Asn Leu Glu Val Leu Glu Thr Ala Arg Arg Val Thr Gly
            260                 265                 270

aaa gaa att cca tca caa atg gga gaa cgt cgc cct ggt gac cca gat 864
Lys Glu Ile Pro Ser Gln Met Gly Glu Arg Arg Pro Gly Asp Pro Asp
        275                 280                 285

atg ctt gtt gca gac tcg acg aaa gct gga gaa atc ttg gat tgg cac 912
Met Leu Val Ala Asp Ser Thr Lys Ala Gly Glu Ile Leu Asp Trp His

290                 295                 300

gtg aag aat aat tta gaa tat att atc gag aat gct tgg aaa tgg cat         960
Val Lys Asn Asn Leu Glu Tyr Ile Ile Glu Asn Ala Trp Lys Trp His
305                 310                 315                 320

agc tct cat cca aat gga tat taa                                         984
Ser Ser His Pro Asn Gly Tyr
                325

<210>  42
<211>  327
<212>  PRT
<213>  Lactococcus garvieae Lg2

<400>  42

Met Thr Val Leu Val Leu Gly Gly Ala Gly Tyr Val Gly Ser His Ala
1               5                   10                  15

Val Asp Ile Leu Val Ala Arg Gly Tyr Asp Val Ala Val Val Asp Asn
            20                  25                  30

Leu Val Thr Gly His Arg Glu Ala Val Pro Ala Asp Val Arg Phe Tyr
        35                  40                  45

Glu Gly Asp Val Arg Asp Gln Ala Phe Leu Ala Asp Val Phe Thr Lys
    50                  55                  60

Glu Lys Asp Ile Glu Gly Leu Met His Phe Cys Ala Tyr Ser Leu Val
65                  70                  75                  80

Gly Glu Ser Met Gln Lys Pro Leu Met Tyr Phe Asn Asn Asn Val Gly
                85                  90                  95

Gly Ala Gln Val Ile Leu Glu Thr Met Glu Lys Phe Gly Ile Lys His
            100                 105                 110

Ile Val Phe Ser Ser Thr Ala Ala Thr Phe Gly Ile Pro Glu Glu Ser
        115                 120                 125

Pro Ile Ser Glu Lys Thr Pro Gln Lys Pro Ile Asn Pro Tyr Gly Glu
    130                 135                 140

Ser Lys Leu Ile Met Glu Lys Met Met Lys Trp Gln Ser Glu Ala Thr
145                 150                 155                 160

Asp Met Thr Tyr Val Ala Leu Arg Tyr Phe Asn Val Ala Gly Ala Lys
                165                 170                 175

His Asp Gly Ser Ile Gly Glu Ala His Lys Asn Glu Thr His Leu Ile

180                 185                 190

Pro Leu Ile Leu Gln Thr Ala Leu Gly Gln Arg Glu Phe Ile Thr Ile
        195                 200                 205

Tyr Gly Asp Asp Tyr Asn Thr Pro Asp Gly Thr Cys Val Arg Asp Tyr
    210                 215                 220

Ile Asp Met Glu Asp Leu Ile Glu Ala His Ile Lys Ala Leu Glu Tyr
225                 230                 235                 240

Leu Lys Ala Gly Gly Ala Ser Asp Gln Phe Asn Leu Gly Ser Ser Lys
                245                 250                 255

Gly Tyr Ser Asn Leu Glu Val Leu Glu Thr Ala Arg Arg Val Thr Gly
            260                 265                 270

Lys Glu Ile Pro Ser Gln Met Gly Glu Arg Arg Pro Gly Asp Pro Asp
        275                 280                 285

Met Leu Val Ala Asp Ser Thr Lys Ala Gly Glu Ile Leu Asp Trp His
    290                 295                 300

Val Lys Asn Asn Leu Glu Tyr Ile Ile Glu Asn Ala Trp Lys Trp His
305                 310                 315                 320

Ser Ser His Pro Asn Gly Tyr
                325

<210>  43
<211>  1188
<212>  DNA
<213>  Synthetic

<220>
<221>  CDS
<222>  (1)..(1188)
<223>  codon optimised xylA gene encoding xylose isomerase (originating
       from Arthrobacter sp.)

<400>  43
atg tcc gtg cag cca acc cca gct gat cac ttc acc ttc ggc ctg tgg      48
Met Ser Val Gln Pro Thr Pro Ala Asp His Phe Thr Phe Gly Leu Trp
1               5                   10                  15

acc gtg ggt tgg acc ggt gct gat cca ttc ggc gtc gca acc cgc aag      96
Thr Val Gly Trp Thr Gly Ala Asp Pro Phe Gly Val Ala Thr Arg Lys
            20                  25                  30

aac ctc gac cca gtg gag gca gtc cac aag ctc gca gaa ctg ggc gcc      144
Asn Leu Asp Pro Val Glu Ala Val His Lys Leu Ala Glu Leu Gly Ala
        35                  40                  45

tac ggc atc acc ttc cac gat aac gac ctg atc cca ttc gat gct acc 192
Tyr Gly Ile Thr Phe His Asp Asn Asp Leu Ile Pro Phe Asp Ala Thr
    50                  55                  60

gaa gca gag cgc gaa aag atc ctc ggc gat ttc aac cag gcc ctc aag 240
Glu Ala Glu Arg Glu Lys Ile Leu Gly Asp Phe Asn Gln Ala Leu Lys
65                  70                  75                  80

gac acc ggc ctg aag gtg cca atg gtc acc acc aac ctg ttc tcc cac 288
Asp Thr Gly Leu Lys Val Pro Met Val Thr Thr Asn Leu Phe Ser His
                85                  90                  95

cca gtg ttc aag gat ggt ggc ttc acc tcc aac gac cgc tcc atc cgt 336
Pro Val Phe Lys Asp Gly Gly Phe Thr Ser Asn Asp Arg Ser Ile Arg
            100                 105                 110

cgc ttc gca ctc gcc aag gtg ctg cac aac atc gat ctc gca gcc gag 384
Arg Phe Ala Leu Ala Lys Val Leu His Asn Ile Asp Leu Ala Ala Glu
        115                 120                 125

atg ggc gca gaa acc ttc gtc atg tgg ggc ggc cgt gag ggt tcc gaa 432
Met Gly Ala Glu Thr Phe Val Met Trp Gly Gly Arg Glu Gly Ser Glu
    130                 135                 140

tac gat ggc tcc aag gac ctc gca gca gca ctg gat cgc atg cgc gaa 480
Tyr Asp Gly Ser Lys Asp Leu Ala Ala Ala Leu Asp Arg Met Arg Glu
145                 150                 155                 160

ggc gtc gac acc gca gca ggc tac atc aag gat aag ggc tac aac ctc 528
Gly Val Asp Thr Ala Ala Gly Tyr Ile Lys Asp Lys Gly Tyr Asn Leu
                165                 170                 175

cgc atc gca ctg gag cca aag cca aac gaa cca cgc ggc gac atc ttc 576
Arg Ile Ala Leu Glu Pro Lys Pro Asn Glu Pro Arg Gly Asp Ile Phe
            180                 185                 190

ctg cca acc gtg ggt cac ggc ctg gca ttc atc gag cag ctc gaa cac 624
Leu Pro Thr Val Gly His Gly Leu Ala Phe Ile Glu Gln Leu Glu His
        195                 200                 205

ggc gat atc gtc ggc ctg aac cca gaa acc ggc cac gaa cag atg gct 672
Gly Asp Ile Val Gly Leu Asn Pro Glu Thr Gly His Glu Gln Met Ala
    210                 215                 220

ggc ctc aac ttc acc cac ggc atc gct cag gca ctc tgg gca gag aag 720
Gly Leu Asn Phe Thr His Gly Ile Ala Gln Ala Leu Trp Ala Glu Lys
225                 230                 235                 240

ctg ttc cac atc gac ctc aac ggc cag cgc ggc atc aag tac gat cag 768
Leu Phe His Ile Asp Leu Asn Gly Gln Arg Gly Ile Lys Tyr Asp Gln
                245                 250                 255

gac ctg gtg ttc ggc cac ggc gat ctc acc tcc gca ttc ttc acc gtc 816
Asp Leu Val Phe Gly His Gly Asp Leu Thr Ser Ala Phe Phe Thr Val
            260                 265                 270

gac ctg ctc gaa aac ggc ttc cca aac ggt ggc cca aag tac acc ggc 864
Asp Leu Leu Glu Asn Gly Phe Pro Asn Gly Gly Pro Lys Tyr Thr Gly
        275                 280                 285

cca cgc cac ttc gat tac aag cca tcc cgc acc gat ggt tac gat ggt 912
Pro Arg His Phe Asp Tyr Lys Pro Ser Arg Thr Asp Gly Tyr Asp Gly

290                 295                 300

gtg tgg gac tcc gcc aag gct aac atg tcc atg tac ctg ctg ttg aag         960
Val Trp Asp Ser Ala Lys Ala Asn Met Ser Met Tyr Leu Leu Leu Lys
305                 310                 315                 320

gag cgt gca ctg gca ttc cgt gct gat cca gag gtg cag gaa gct atg         1008
Glu Arg Ala Leu Ala Phe Arg Ala Asp Pro Glu Val Gln Glu Ala Met
                325                 330                 335

aag acc tcc ggc gtc ttc gag ctg ggc gaa acc acc ctc aac gct ggt         1056
Lys Thr Ser Gly Val Phe Glu Leu Gly Glu Thr Thr Leu Asn Ala Gly
            340                 345                 350

gaa tcc gcc gct gat ctg atg aac gac tcc gcc tcc ttc gct ggc ttc         1104
Glu Ser Ala Ala Asp Leu Met Asn Asp Ser Ala Ser Phe Ala Gly Phe
        355                 360                 365

gac gct gag gca gcc gct gaa cgc aac ttc gca ttc atc cgc ctc aac         1152
Asp Ala Glu Ala Ala Ala Glu Arg Asn Phe Ala Phe Ile Arg Leu Asn
    370                 375                 380

cag ctg gcc atc gaa cac ctc ctg ggc tcc cgc taa                         1188
Gln Leu Ala Ile Glu His Leu Leu Gly Ser Arg
385                 390                 395

<210>  44
<211>  395
<212>  PRT
<213>  Synthetic

<400>  44

Met Ser Val Gln Pro Thr Pro Ala Asp His Phe Thr Phe Gly Leu Trp
1               5                   10                  15

Thr Val Gly Trp Thr Gly Ala Asp Pro Phe Gly Val Ala Thr Arg Lys
            20                  25                  30

Asn Leu Asp Pro Val Glu Ala Val His Lys Leu Ala Glu Leu Gly Ala
        35                  40                  45

Tyr Gly Ile Thr Phe His Asp Asn Asp Leu Ile Pro Phe Asp Ala Thr
    50                  55                  60

Glu Ala Glu Arg Glu Lys Ile Leu Gly Asp Phe Asn Gln Ala Leu Lys
65                  70                  75                  80

Asp Thr Gly Leu Lys Val Pro Met Val Thr Thr Asn Leu Phe Ser His
                85                  90                  95

Pro Val Phe Lys Asp Gly Gly Phe Thr Ser Asn Asp Arg Ser Ile Arg
            100                 105                 110

Arg Phe Ala Leu Ala Lys Val Leu His Asn Ile Asp Leu Ala Ala Glu

115                 120                 125

Met Gly Ala Glu Thr Phe Val Met Trp Gly Gly Arg Glu Gly Ser Glu
    130                 135                 140

Tyr Asp Gly Ser Lys Asp Leu Ala Ala Ala Leu Asp Arg Met Arg Glu
145                 150                 155                 160

Gly Val Asp Thr Ala Ala Gly Tyr Ile Lys Asp Lys Gly Tyr Asn Leu
                165                 170                 175

Arg Ile Ala Leu Glu Pro Lys Pro Asn Glu Pro Arg Gly Asp Ile Phe
            180                 185                 190

Leu Pro Thr Val Gly His Gly Leu Ala Phe Ile Glu Gln Leu Glu His
        195                 200                 205

Gly Asp Ile Val Gly Leu Asn Pro Glu Thr Gly His Glu Gln Met Ala
    210                 215                 220

Gly Leu Asn Phe Thr His Gly Ile Ala Gln Ala Leu Trp Ala Glu Lys
225                 230                 235                 240

Leu Phe His Ile Asp Leu Asn Gly Gln Arg Gly Ile Lys Tyr Asp Gln
                245                 250                 255

Asp Leu Val Phe Gly His Gly Asp Leu Thr Ser Ala Phe Phe Thr Val
            260                 265                 270

Asp Leu Leu Glu Asn Gly Phe Pro Asn Gly Gly Pro Lys Tyr Thr Gly
        275                 280                 285

Pro Arg His Phe Asp Tyr Lys Pro Ser Arg Thr Asp Gly Tyr Asp Gly
    290                 295                 300

Val Trp Asp Ser Ala Lys Ala Asn Met Ser Met Tyr Leu Leu Leu Lys
305                 310                 315                 320

Glu Arg Ala Leu Ala Phe Arg Ala Asp Pro Glu Val Gln Glu Ala Met
                325                 330                 335

Lys Thr Ser Gly Val Phe Glu Leu Gly Glu Thr Thr Leu Asn Ala Gly
            340                 345                 350

Glu Ser Ala Ala Asp Leu Met Asn Asp Ser Ala Ser Phe Ala Gly Phe
        355                 360                 365

Asp Ala Glu Ala Ala Ala Glu Arg Asn Phe Ala Phe Ile Arg Leu Asn
    370                 375                 380

Gln Leu Ala Ile Glu His Leu Leu Gly Ser Arg
385                 390                 395

<210>  45
<211>  1188
<212>  DNA
<213>  Arthrobacter Sp. N.R.R.L. B3728

<220>
<221>  CDS
<222>  (1)..(1188)
<223>  xylA gene encoding D-xylose isomerase

<300>
<308>  GenBank X59466.1
<309>  2016-07-26
<313>  (396)..(1578)

<400>  45
atg agc gtt cag ccg acc cct gca gac cac ttc acc ttt ggc ctc tgg        48
Met Ser Val Gln Pro Thr Pro Ala Asp His Phe Thr Phe Gly Leu Trp
1               5                   10                  15

acc gta gga tgg acc ggc gcc gac cca ttc ggt gtc gcc acc cgc aag        96
Thr Val Gly Trp Thr Gly Ala Asp Pro Phe Gly Val Ala Thr Arg Lys
            20                  25                  30

aac ctg gac ccg gta gaa gcc gtc cac aag ctg gcc gag ctc ggc gcc       144
Asn Leu Asp Pro Val Glu Ala Val His Lys Leu Ala Glu Leu Gly Ala
        35                  40                  45

tac ggc atc acc ttc cac gac aat gac ctg att cct ttt gac gcc acc       192
Tyr Gly Ile Thr Phe His Asp Asn Asp Leu Ile Pro Phe Asp Ala Thr
    50                  55                  60

gag gca gag cgc gaa aag atc ctt ggt gac ttc aac cag gcg ctg aag       240
Glu Ala Glu Arg Glu Lys Ile Leu Gly Asp Phe Asn Gln Ala Leu Lys
65                  70                  75                  80

gac acc ggc ctg aag gtc cca atg gtg acc acc aac ctg ttc agc cac       288
Asp Thr Gly Leu Lys Val Pro Met Val Thr Thr Asn Leu Phe Ser His
                85                  90                  95

ccg gtc ttc aag gac ggc ggc ttc acc tct aac gac cgc tcg atc cgt       336
Pro Val Phe Lys Asp Gly Gly Phe Thr Ser Asn Asp Arg Ser Ile Arg
            100                 105                 110

cgt ttt gca ctg gct aag gtc ctg cac aac atc gac ttg gca gcc gag       384
Arg Phe Ala Leu Ala Lys Val Leu His Asn Ile Asp Leu Ala Ala Glu
        115                 120                 125

atg ggc gcc gaa acc ttc gtc atg tgg ggc ggg cgc gaa ggc agc gaa       432
Met Gly Ala Glu Thr Phe Val Met Trp Gly Gly Arg Glu Gly Ser Glu
    130                 135                 140

tac gac ggt tcc aag gac ctg gcc gca gca ctt gat cgc atg cgc gaa       480
Tyr Asp Gly Ser Lys Asp Leu Ala Ala Ala Leu Asp Arg Met Arg Glu

145                 150                 155                 160

ggc gtg gac acg gca gct ggc tac atc aag gac aag ggt tac aac ctg      528
Gly Val Asp Thr Ala Ala Gly Tyr Ile Lys Asp Lys Gly Tyr Asn Leu
                165                 170                 175

cgc atc gcg ctg gag cca aag cca aat gaa cca cgc ggc gac atc ttc      576
Arg Ile Ala Leu Glu Pro Lys Pro Asn Glu Pro Arg Gly Asp Ile Phe
            180                 185                 190

ctg cct acc gtc ggc cac ggc ctg gcc ttc atc gag cag ctg gag cac      624
Leu Pro Thr Val Gly His Gly Leu Ala Phe Ile Glu Gln Leu Glu His
        195                 200                 205

ggc gac atc gtc ggc ctg aac cca gaa acc ggc cac gag cag atg gcc      672
Gly Asp Ile Val Gly Leu Asn Pro Glu Thr Gly His Glu Gln Met Ala
    210                 215                 220

ggc ctg aac ttc acc cac ggc atc gct cag gca ctg tgg gcc gag aag      720
Gly Leu Asn Phe Thr His Gly Ile Ala Gln Ala Leu Trp Ala Glu Lys
225                 230                 235                 240

ctg ttc cac att gac ctc aac ggc cag cgc ggc atc aag tac gac cag      768
Leu Phe His Ile Asp Leu Asn Gly Gln Arg Gly Ile Lys Tyr Asp Gln
                245                 250                 255

gac ctg gtc ttc ggc cac ggc gat ctg acc agc gcg ttc ttc acc gta      816
Asp Leu Val Phe Gly His Gly Asp Leu Thr Ser Ala Phe Phe Thr Val
            260                 265                 270

gac ctg ctg gaa aac ggc ttc cct aac ggc gga cca aag tac acc ggc      864
Asp Leu Leu Glu Asn Gly Phe Pro Asn Gly Gly Pro Lys Tyr Thr Gly
        275                 280                 285

cca cgc cac ttc gac tac aag cca tcg cgc acc gac ggc tac gac ggc      912
Pro Arg His Phe Asp Tyr Lys Pro Ser Arg Thr Asp Gly Tyr Asp Gly
    290                 295                 300

gtg tgg gac tcg gcc aag gcc aac atg tcc atg tac ctg ctg ctc aag      960
Val Trp Asp Ser Ala Lys Ala Asn Met Ser Met Tyr Leu Leu Leu Lys
305                 310                 315                 320

gaa cgt gcc ctg gcc ttc cgt gcg gat cca gag gta cag gaa gcc atg     1008
Glu Arg Ala Leu Ala Phe Arg Ala Asp Pro Glu Val Gln Glu Ala Met
                325                 330                 335

aag acc tcg ggc gtc ttc gaa ctg ggc gaa acc acc ctg aac gcc ggg     1056
Lys Thr Ser Gly Val Phe Glu Leu Gly Glu Thr Thr Leu Asn Ala Gly
            340                 345                 350

gaa agc gca gcg gat ctg atg aat gat tcc gcg agc ttc gca ggc ttt     1104
Glu Ser Ala Ala Asp Leu Met Asn Asp Ser Ala Ser Phe Ala Gly Phe
        355                 360                 365

gac gcc gag gcc gcc gca gag cgc aac ttc gcg ttc atc cgc ctg aac     1152
Asp Ala Glu Ala Ala Ala Glu Arg Asn Phe Ala Phe Ile Arg Leu Asn
    370                 375                 380

cag ctg gcc atc gag cac ctg ctc ggc tcc cgc taa                     1188
Gln Leu Ala Ile Glu His Leu Leu Gly Ser Arg
385                 390                 395

<210>  46
<211>  395
<212>  PRT
<213>  Arthrobacter Sp. N.R.R.L. B3728

<400>  46

Met Ser Val Gln Pro Thr Pro Ala Asp His Phe Thr Phe Gly Leu Trp
1               5                   10                  15

Thr Val Gly Trp Thr Gly Ala Asp Pro Phe Gly Val Ala Thr Arg Lys
            20                  25                  30

Asn Leu Asp Pro Val Glu Ala Val His Lys Leu Ala Glu Leu Gly Ala
        35                  40                  45

Tyr Gly Ile Thr Phe His Asp Asn Asp Leu Ile Pro Phe Asp Ala Thr
    50                  55                  60

Glu Ala Glu Arg Glu Lys Ile Leu Gly Asp Phe Asn Gln Ala Leu Lys
65                  70                  75                  80

Asp Thr Gly Leu Lys Val Pro Met Val Thr Thr Asn Leu Phe Ser His
                85                  90                  95

Pro Val Phe Lys Asp Gly Gly Phe Thr Ser Asn Asp Arg Ser Ile Arg
            100                 105                 110

Arg Phe Ala Leu Ala Lys Val Leu His Asn Ile Asp Leu Ala Ala Glu
        115                 120                 125

Met Gly Ala Glu Thr Phe Val Met Trp Gly Gly Arg Glu Gly Ser Glu
    130                 135                 140

Tyr Asp Gly Ser Lys Asp Leu Ala Ala Ala Leu Asp Arg Met Arg Glu
145                 150                 155                 160

Gly Val Asp Thr Ala Ala Gly Tyr Ile Lys Asp Lys Gly Tyr Asn Leu
                165                 170                 175

Arg Ile Ala Leu Glu Pro Lys Pro Asn Glu Pro Arg Gly Asp Ile Phe
            180                 185                 190

Leu Pro Thr Val Gly His Gly Leu Ala Phe Ile Glu Gln Leu Glu His
        195                 200                 205

Gly Asp Ile Val Gly Leu Asn Pro Glu Thr Gly His Glu Gln Met Ala
    210                 215                 220

Gly Leu Asn Phe Thr His Gly Ile Ala Gln Ala Leu Trp Ala Glu Lys
225                 230                 235                 240

Leu Phe His Ile Asp Leu Asn Gly Gln Arg Gly Ile Lys Tyr Asp Gln
                245                 250                 255

Asp Leu Val Phe Gly His Gly Asp Leu Thr Ser Ala Phe Phe Thr Val
            260                 265                 270

Asp Leu Leu Glu Asn Gly Phe Pro Asn Gly Gly Pro Lys Tyr Thr Gly
        275                 280                 285

Pro Arg His Phe Asp Tyr Lys Pro Ser Arg Thr Asp Gly Tyr Asp Gly
    290                 295                 300

Val Trp Asp Ser Ala Lys Ala Asn Met Ser Met Tyr Leu Leu Leu Lys
305                 310                 315                 320

Glu Arg Ala Leu Ala Phe Arg Ala Asp Pro Glu Val Gln Glu Ala Met
                325                 330                 335

Lys Thr Ser Gly Val Phe Glu Leu Gly Glu Thr Thr Leu Asn Ala Gly
            340                 345                 350

Glu Ser Ala Ala Asp Leu Met Asn Asp Ser Ala Ser Phe Ala Gly Phe
        355                 360                 365

Asp Ala Glu Ala Ala Ala Glu Arg Asn Phe Ala Phe Ile Arg Leu Asn
    370                 375                 380

Gln Leu Ala Ile Glu His Leu Leu Gly Ser Arg
385                 390                 395

<210>  47
<211>  1188
<212>  DNA
<213>  Arthrobacter sp. ZXY-2

<220>
<221>  CDS
<222>  (1)..(1188)
<223>  xylA gene encoding D-xylose isomerase

<300>
<308>  CP017421.1
<309>  2016-10-26
<313>  (58776)..(59963)

<400>  47
atg tcc ccg caa ccc aca ccc cag gac cgt ttc acc ttt ggc ctc tgg          48
Met Ser Pro Gln Pro Thr Pro Gln Asp Arg Phe Thr Phe Gly Leu Trp

1               5                   10                  15

act gtt ggc tgg acc ggc aat gat ccc ttc ggt gtt ccc acc cgc gag          96
Thr Val Gly Trp Thr Gly Asn Asp Pro Phe Gly Val Pro Thr Arg Glu
            20                  25                  30

gcg ctg gac ccg gtg gaa gcc gtc cac aag ctg agt gag ctt ggc gcc         144
Ala Leu Asp Pro Val Glu Ala Val His Lys Leu Ser Glu Leu Gly Ala
        35                  40                  45

tat ggc att acg ttc cat gac aac gat ctg gtc ccg ttc gac gcc acc         192
Tyr Gly Ile Thr Phe His Asp Asn Asp Leu Val Pro Phe Asp Ala Thr
    50                  55                  60

gcg tcc gaa cgc gac ctc atc ctc aag aac ttc aag ggc gcc ctc gca         240
Ala Ser Glu Arg Asp Leu Ile Leu Lys Asn Phe Lys Gly Ala Leu Ala
65                  70                  75                  80

gaa acg ggc ctc aag gtg ccc atg gtc acc acc aac ctg ttc agc cac         288
Glu Thr Gly Leu Lys Val Pro Met Val Thr Thr Asn Leu Phe Ser His
                85                  90                  95

ccg gtc ttc aag gac ggc ggc ttc acg tcc aac gac cga tcc atc cgc         336
Pro Val Phe Lys Asp Gly Gly Phe Thr Ser Asn Asp Arg Ser Ile Arg
            100                 105                 110

cgc tac gcc ctg agc aag gtc ctg agg aac atc gat ctt gcc gct gag         384
Arg Tyr Ala Leu Ser Lys Val Leu Arg Asn Ile Asp Leu Ala Ala Glu
        115                 120                 125

ctc ggc gca gaa acg ttc gtc atg tgg ggc ggc cgc gaa ggc agc gag         432
Leu Gly Ala Glu Thr Phe Val Met Trp Gly Gly Arg Glu Gly Ser Glu
    130                 135                 140

tat gac ggc tca aag gac ctt tcc gca gca ctg gac cgc atg aag gaa         480
Tyr Asp Gly Ser Lys Asp Leu Ser Ala Ala Leu Asp Arg Met Lys Glu
145                 150                 155                 160

ggc gtc gac acc gct gcg ggc tac atc aag gag aag ggc tat ggc ctg         528
Gly Val Asp Thr Ala Ala Gly Tyr Ile Lys Glu Lys Gly Tyr Gly Leu
                165                 170                 175

cgc atc gca ctg gag ccc aag ccg aac gag ccc agg ggc gac att ttc         576
Arg Ile Ala Leu Glu Pro Lys Pro Asn Glu Pro Arg Gly Asp Ile Phe
            180                 185                 190

ctc ccg acc gta ggc cac gcc ctg gca ttt att gcc cag ctg gaa cac         624
Leu Pro Thr Val Gly His Ala Leu Ala Phe Ile Ala Gln Leu Glu His
        195                 200                 205

ggc gac att gtc ggc ctc aac ccg gag acc ggc cac gag caa atg gca         672
Gly Asp Ile Val Gly Leu Asn Pro Glu Thr Gly His Glu Gln Met Ala
    210                 215                 220

ggc ctg aac ttc acc cac gga atc gcc cag gca ctg tgg gcc ggc aag         720
Gly Leu Asn Phe Thr His Gly Ile Ala Gln Ala Leu Trp Ala Gly Lys
225                 230                 235                 240

ctc ttc cac att gat ctc aac ggc cag cgc ggc atc aag tac gac cag         768
Leu Phe His Ile Asp Leu Asn Gly Gln Arg Gly Ile Lys Tyr Asp Gln
                245                 250                 255

gac ctg gtg ttc ggc cac ggc gac ctc acc agc gcc ttc ttc acc gtc         816

Asp Leu Val Phe Gly His Gly Asp Leu Thr Ser Ala Phe Phe Thr Val
            260                 265                 270

gac ctc ctg gag aac ggc ttc ccg aac ggc gga ccc aag tac gac ggt      864
Asp Leu Leu Glu Asn Gly Phe Pro Asn Gly Gly Pro Lys Tyr Asp Gly
        275                 280                 285

ccg cgc cac ttc gac tac aag ccc tca cgc acc gac ggt tac gac ggc      912
Pro Arg His Phe Asp Tyr Lys Pro Ser Arg Thr Asp Gly Tyr Asp Gly
    290                 295                 300

gtc tgg gag tcg gcc aag tcc aac atg gcc atg tac ctg ctc ctc aag      960
Val Trp Glu Ser Ala Lys Ser Asn Met Ala Met Tyr Leu Leu Leu Lys
305                 310                 315                 320

gaa cgt gcc gtt gcc ttc cgc gcg gac ccc gaa gtc cag gaa gca ctc     1008
Glu Arg Ala Val Ala Phe Arg Ala Asp Pro Glu Val Gln Glu Ala Leu
                325                 330                 335

gca acc tcc ggc gtg ttc gaa ctc ggc gaa tcc acc ctg gcc gcc ggc     1056
Ala Thr Ser Gly Val Phe Glu Leu Gly Glu Ser Thr Leu Ala Ala Gly
            340                 345                 350

gag acc acc gcg gac ctc ctg gct gac gca tca gcc ttc gag tcg ttc     1104
Glu Thr Thr Ala Asp Leu Leu Ala Asp Ala Ser Ala Phe Glu Ser Phe
        355                 360                 365

gac gcc aac aag gcc gcg gag cgc tcc ttc gcc ttc atc agg ctg aac     1152
Asp Ala Asn Lys Ala Ala Glu Arg Ser Phe Ala Phe Ile Arg Leu Asn
    370                 375                 380

cag ctg gcc atc gag cac ctc ctc ggc gcc cgc tga                     1188
Gln Leu Ala Ile Glu His Leu Leu Gly Ala Arg
385                 390                 395


<210>  48
<211>  395
<212>  PRT
<213>  Arthrobacter sp. ZXY-2

<400>  48

Met Ser Pro Gln Pro Thr Pro Gln Asp Arg Phe Thr Phe Gly Leu Trp
1               5                   10                  15


Thr Val Gly Trp Thr Gly Asn Asp Pro Phe Gly Val Pro Thr Arg Glu
            20                  25                  30


Ala Leu Asp Pro Val Glu Ala Val His Lys Leu Ser Glu Leu Gly Ala
        35                  40                  45


Tyr Gly Ile Thr Phe His Asp Asn Asp Leu Val Pro Phe Asp Ala Thr
    50                  55                  60


Ala Ser Glu Arg Asp Leu Ile Leu Lys Asn Phe Lys Gly Ala Leu Ala
65                  70                  75                  80

Glu Thr Gly Leu Lys Val Pro Met Val Thr Thr Asn Leu Phe Ser His
                85                  90                  95

Pro Val Phe Lys Asp Gly Gly Phe Thr Ser Asn Asp Arg Ser Ile Arg
            100                 105                 110

Arg Tyr Ala Leu Ser Lys Val Leu Arg Asn Ile Asp Leu Ala Ala Glu
        115                 120                 125

Leu Gly Ala Glu Thr Phe Val Met Trp Gly Gly Arg Glu Gly Ser Glu
    130                 135                 140

Tyr Asp Gly Ser Lys Asp Leu Ser Ala Ala Leu Asp Arg Met Lys Glu
145                 150                 155                 160

Gly Val Asp Thr Ala Ala Gly Tyr Ile Lys Glu Lys Gly Tyr Gly Leu
                165                 170                 175

Arg Ile Ala Leu Glu Pro Lys Pro Asn Glu Pro Arg Gly Asp Ile Phe
            180                 185                 190

Leu Pro Thr Val Gly His Ala Leu Ala Phe Ile Ala Gln Leu Glu His
        195                 200                 205

Gly Asp Ile Val Gly Leu Asn Pro Glu Thr Gly His Glu Gln Met Ala
    210                 215                 220

Gly Leu Asn Phe Thr His Gly Ile Ala Gln Ala Leu Trp Ala Gly Lys
225                 230                 235                 240

Leu Phe His Ile Asp Leu Asn Gly Gln Arg Gly Ile Lys Tyr Asp Gln
                245                 250                 255

Asp Leu Val Phe Gly His Gly Asp Leu Thr Ser Ala Phe Phe Thr Val
            260                 265                 270

Asp Leu Leu Glu Asn Gly Phe Pro Asn Gly Gly Pro Lys Tyr Asp Gly
        275                 280                 285

Pro Arg His Phe Asp Tyr Lys Pro Ser Arg Thr Asp Gly Tyr Asp Gly
    290                 295                 300

Val Trp Glu Ser Ala Lys Ser Asn Met Ala Met Tyr Leu Leu Leu Lys
305                 310                 315                 320

Glu Arg Ala Val Ala Phe Arg Ala Asp Pro Glu Val Gln Glu Ala Leu
                325                 330                 335

Ala Thr Ser Gly Val Phe Glu Leu Gly Glu Ser Thr Leu Ala Ala Gly
            340                 345                 350

Glu Thr Thr Ala Asp Leu Leu Ala Asp Ala Ser Ala Phe Glu Ser Phe
        355                 360                 365

Asp Ala Asn Lys Ala Ala Glu Arg Ser Phe Ala Phe Ile Arg Leu Asn
    370                 375                 380

Gln Leu Ala Ile Glu His Leu Leu Gly Ala Arg
385                 390                 395

<210>  49
<211>  1422
<212>  DNA
<213>  Synthetic

<220>
<221>  CDS
<222>  (1)..(1422)
<223>  codon optimized araA gene encoding arabinose isomerase
       (originating from B. coagulans)

<400>  49
atg ctg aag atc aag gat tac gaa ttc tgg ttc atc gtg ggc tcc cag      48
Met Leu Lys Ile Lys Asp Tyr Glu Phe Trp Phe Ile Val Gly Ser Gln
1               5                   10                  15

cac ctg tac ggc aag gaa acc ctg gat gca gtc aag gaa gac gcc gag      96
His Leu Tyr Gly Lys Glu Thr Leu Asp Ala Val Lys Glu Asp Ala Glu
            20                  25                  30

aag atc atc aac gaa atc aac gca tcc ggc aag ctg cca tac cca atc      144
Lys Ile Ile Asn Glu Ile Asn Ala Ser Gly Lys Leu Pro Tyr Pro Ile
        35                  40                  45

gtg ttc aag acc gtc gca acc acc gcc gat tcc atc acc cag gtg atg      192
Val Phe Lys Thr Val Ala Thr Thr Ala Asp Ser Ile Thr Gln Val Met
    50                  55                  60

aag gag gtc aac tac aac gac aac gtg gcc ggc gtc atc acc tgg atg      240
Lys Glu Val Asn Tyr Asn Asp Asn Val Ala Gly Val Ile Thr Trp Met
65                  70                  75                  80

cac acc ttc tcc cca gct aag aac tgg atc cgc ggc acc aag ctg ctc      288
His Thr Phe Ser Pro Ala Lys Asn Trp Ile Arg Gly Thr Lys Leu Leu
                85                  90                  95

cag aag cca ctg ctc cac ctg gcc acc cag ttc ctc gat cac atc cca      336
Gln Lys Pro Leu Leu His Leu Ala Thr Gln Phe Leu Asp His Ile Pro
            100                 105                 110

tgg gaa acc atc gat atg gac tac atg aac ctg cac cag tcc gca cac      384
Trp Glu Thr Ile Asp Met Asp Tyr Met Asn Leu His Gln Ser Ala His
        115                 120                 125

ggc gac cgc gag tac ggc ttc atc aac gcc cgc ctc aac aag aac aac      432

Gly Asp Arg Glu Tyr Gly Phe Ile Asn Ala Arg Leu Asn Lys Asn Asn
    130                 135                 140

aag atc gtg gtc ggc cac tgg aag gat gaa aag gtg cag acc cag atc     480
Lys Ile Val Val Gly His Trp Lys Asp Glu Lys Val Gln Thr Gln Ile
145                 150                 155                 160

ggc cag tgg atg gac gtg gcc gtc gct ttc aac gag tcc ttc aac atc     528
Gly Gln Trp Met Asp Val Ala Val Ala Phe Asn Glu Ser Phe Asn Ile
                165                 170                 175

aag gtc gca cgc ttc ggc gat aac atg cgc aac gtg gct gtc acc gat     576
Lys Val Ala Arg Phe Gly Asp Asn Met Arg Asn Val Ala Val Thr Asp
            180                 185                 190

ggc gac aag atc gaa gca cag atc cag ttc ggc tgg acc gtg gat tac     624
Gly Asp Lys Ile Glu Ala Gln Ile Gln Phe Gly Trp Thr Val Asp Tyr
        195                 200                 205

tac ggc atc ggc gac ctg gtg gct gaa atg aag gag gtc acc gac cag     672
Tyr Gly Ile Gly Asp Leu Val Ala Glu Met Lys Glu Val Thr Asp Gln
    210                 215                 220

gaa atc gag gct gtc tgc gca gaa tgc cag gag aag tac gaa ctc gtg     720
Glu Ile Glu Ala Val Cys Ala Glu Cys Gln Glu Lys Tyr Glu Leu Val
225                 230                 235                 240

gtc ggc aac aac gat cca gca tac ttc gag gac cac gtg aag gaa cag     768
Val Gly Asn Asn Asp Pro Ala Tyr Phe Glu Asp His Val Lys Glu Gln
                245                 250                 255

atc cgc atc gag atc gca ctg cgc aag ttc ctc gat cgc ggc ggc tac     816
Ile Arg Ile Glu Ile Ala Leu Arg Lys Phe Leu Asp Arg Gly Gly Tyr
            260                 265                 270

acc gcc ttc acc acc aac ttc gaa gac ctg tgg ggc atg aag cag ctc     864
Thr Ala Phe Thr Thr Asn Phe Glu Asp Leu Trp Gly Met Lys Gln Leu
        275                 280                 285

cca ggc atg gct gtg cag cgc ctg aac gca gaa ggt tac ggt ttc gca     912
Pro Gly Met Ala Val Gln Arg Leu Asn Ala Glu Gly Tyr Gly Phe Ala
    290                 295                 300

ggc gag ggt gat tgg aag acc gca gcc ctg gac cgc ctc gtg aag atc     960
Gly Glu Gly Asp Trp Lys Thr Ala Ala Leu Asp Arg Leu Val Lys Ile
305                 310                 315                 320

atg gcc cac aac gaa aag acc ggc ttc atg gag gat tac acc tac aac    1008
Met Ala His Asn Glu Lys Thr Gly Phe Met Glu Asp Tyr Thr Tyr Asn
                325                 330                 335

ctg gtg aag ggc cac gaa gag atc ctg ggc tcc cac atg ctc gaa gtc    1056
Leu Val Lys Gly His Glu Glu Ile Leu Gly Ser His Met Leu Glu Val
            340                 345                 350

gac cca acc ctc gct tcc ggc aag atc cgc gtg gaa gtc cac cca ctg    1104
Asp Pro Thr Leu Ala Ser Gly Lys Ile Arg Val Glu Val His Pro Leu
        355                 360                 365

ggt atc ggc gat cgc gag gac cca gct cgt ctc gtg ttc gat ggc acc    1152
Gly Ile Gly Asp Arg Glu Asp Pro Ala Arg Leu Val Phe Asp Gly Thr
    370                 375                 380

gat ggc gac gca gtg aac ctg acc gtc tcc gat ttc ggc gac cag ttc 1200
Asp Gly Asp Ala Val Asn Leu Thr Val Ser Asp Phe Gly Asp Gln Phe
385                 390                 395                 400

aag ctc gtg atg tac gaa gtc gac ggc aag aag cca gcc gag gct gca 1248
Lys Leu Val Met Tyr Glu Val Asp Gly Lys Lys Pro Ala Glu Ala Ala
                405                 410                 415

cca aag ctg cca gtg gct cgt cag ctc tgg acc cca aag cca ggc ttc 1296
Pro Lys Leu Pro Val Ala Arg Gln Leu Trp Thr Pro Lys Pro Gly Phe
            420                 425                 430

tac gaa ggc gtc cag aag tgg atc gag aac ggt ggt ggt cac cac acc 1344
Tyr Glu Gly Val Gln Lys Trp Ile Glu Asn Gly Gly Gly His His Thr
        435                 440                 445

gtg ctg tcc ttc gcc gtc acc gct gag cag atc gaa gat ttc gca aag 1392
Val Leu Ser Phe Ala Val Thr Ala Glu Gln Ile Glu Asp Phe Ala Lys
    450                 455                 460

atg gtg ggc ctc aag acc gtc aag atc taa 1422
Met Val Gly Leu Lys Thr Val Lys Ile
465                 470

<210> 50
<211> 473
<212> PRT
<213> Synthetic

<400> 50

Met Leu Lys Ile Lys Asp Tyr Glu Phe Trp Phe Ile Val Gly Ser Gln
1               5                   10                  15

His Leu Tyr Gly Lys Glu Thr Leu Asp Ala Val Lys Glu Asp Ala Glu
            20                  25                  30

Lys Ile Ile Asn Glu Ile Asn Ala Ser Gly Lys Leu Pro Tyr Pro Ile
        35                  40                  45

Val Phe Lys Thr Val Ala Thr Thr Ala Asp Ser Ile Thr Gln Val Met
    50                  55                  60

Lys Glu Val Asn Tyr Asn Asp Asn Val Ala Gly Val Ile Thr Trp Met
65                  70                  75                  80

His Thr Phe Ser Pro Ala Lys Asn Trp Ile Arg Gly Thr Lys Leu Leu
                85                  90                  95

Gln Lys Pro Leu Leu His Leu Ala Thr Gln Phe Leu Asp His Ile Pro
            100                 105                 110

Trp Glu Thr Ile Asp Met Asp Tyr Met Asn Leu His Gln Ser Ala His
        115                 120                 125

Gly Asp Arg Glu Tyr Gly Phe Ile Asn Ala Arg Leu Asn Lys Asn Asn
    130                 135                 140

Lys Ile Val Val Gly His Trp Lys Asp Glu Lys Val Gln Thr Gln Ile
145                 150                 155                 160

Gly Gln Trp Met Asp Val Ala Val Ala Phe Asn Glu Ser Phe Asn Ile
                165                 170                 175

Lys Val Ala Arg Phe Gly Asp Asn Met Arg Asn Val Ala Val Thr Asp
            180                 185                 190

Gly Asp Lys Ile Glu Ala Gln Ile Gln Phe Gly Trp Thr Val Asp Tyr
        195                 200                 205

Tyr Gly Ile Gly Asp Leu Val Ala Glu Met Lys Glu Val Thr Asp Gln
    210                 215                 220

Glu Ile Glu Ala Val Cys Ala Glu Cys Gln Glu Lys Tyr Glu Leu Val
225                 230                 235                 240

Val Gly Asn Asn Asp Pro Ala Tyr Phe Glu Asp His Val Lys Glu Gln
                245                 250                 255

Ile Arg Ile Glu Ile Ala Leu Arg Lys Phe Leu Asp Arg Gly Gly Tyr
            260                 265                 270

Thr Ala Phe Thr Thr Asn Phe Glu Asp Leu Trp Gly Met Lys Gln Leu
        275                 280                 285

Pro Gly Met Ala Val Gln Arg Leu Asn Ala Glu Gly Tyr Gly Phe Ala
    290                 295                 300

Gly Glu Gly Asp Trp Lys Thr Ala Ala Leu Asp Arg Leu Val Lys Ile
305                 310                 315                 320

Met Ala His Asn Glu Lys Thr Gly Phe Met Glu Asp Tyr Thr Tyr Asn
                325                 330                 335

Leu Val Lys Gly His Glu Glu Ile Leu Gly Ser His Met Leu Glu Val
            340                 345                 350

Asp Pro Thr Leu Ala Ser Gly Lys Ile Arg Val Glu Val His Pro Leu
        355                 360                 365

Gly Ile Gly Asp Arg Glu Asp Pro Ala Arg Leu Val Phe Asp Gly Thr
    370                 375                 380

Asp Gly Asp Ala Val Asn Leu Thr Val Ser Asp Phe Gly Asp Gln Phe
385                 390                 395                 400

Lys Leu Val Met Tyr Glu Val Asp Gly Lys Lys Pro Ala Glu Ala Ala
                405                 410                 415

Pro Lys Leu Pro Val Ala Arg Gln Leu Trp Thr Pro Lys Pro Gly Phe
            420                 425                 430

Tyr Glu Gly Val Gln Lys Trp Ile Glu Asn Gly Gly Gly His His Thr
        435                 440                 445

Val Leu Ser Phe Ala Val Thr Ala Glu Gln Ile Glu Asp Phe Ala Lys
    450                 455                 460

Met Val Gly Leu Lys Thr Val Lys Ile
465                 470

<210>  51
<211>  77
<212>  DNA
<213>  Synthetic

<220>
<221>  promoter
<222>  (1)..(77)
<223>  trp promoter core fragment

<400>  51
gagctgttga caattaatca tccggctcgt ataatgtgtg gaattgtgag cggataacaa      60

tttcacacag gaaacag                                                     77

## Description

### FIELD OF THE INVENTION

The present invention provides a genetically modified Corynebacterium capable of hydrolyzing lactose. Further, the invention provides methods for hydrolyzing lactose and producing value-added compounds using the Corynebacterium of the invention. And even further, the invention provides a gene encoding a modified lactose transporter.

### BACKGROUND OF THE INVENTION

The potential that lies in using microorganisms for converting various substrates into value-added compounds has already been recognized, and intense research is being carried out to establish robust and economically feasible processes for their production.

One cheap abundant substrate is cheese whey and its various processed forms, such as whey permeate or whey powder. Whey represents about 85-95% of the milk volume and retains 55% of milk nutrients; and is a liquid by-product of cheese production, obtained when draining the cheese curd. The worldwide production of cheese whey in 2012 was reported to be 4 × 10⁷ tons and about 50% hereof was used for animal feed or otherwise disposed of as waste. The latter is a serious problem as whey is discarded as liquid waste and has a high BOD (biochemical oxygen demand) and COD (chemical oxygen demand). The composition of whey varies according to the source of the milk and the technology used for its production. Normally it contains approximately 90% water, 4% lactose, 1% protein, 0.7% minerals and small amounts of vitamins. Separation of whey proteins generates whey-permeate and further extraction of lactose leads to permeate mother liquor (residual whey permeate, RWP), which comprises about 60% lactose on a dry weight basis, as a leftover product [Ling, 2008].

Hydrolysis of the main carbon source in whey (lactose) to glucose and galactose by the enzyme beta-galactosidase is well-known. However, industrial recombinant enzymes are costly. Meanwhile, microbial cells are cheap and easy to maintain, and may further be recycled such as by gel encapsulation. Corynebacterium glutamicum is a GRAS organism that is safe and has been used for amino acid production for decades; it has great potential as a cell factory for production of value-added compounds, due to its well-characterized metabolic network and relative ease of genetic manipulation. However, C. glutamicum is naturally unable to transport and use lactose as carbon source.

Accordingly, there exists a need to develop a C. glutamicum strain capable of efficiently hydrolyzing lactose, such as hydrolyzing cheap fermentation media based on nutrient-rich waste substrates like whey and whey-derived substrates.

### SUMMARY OF THE INVENTION

In a first aspect, the present invention provides a genetically modified Corynebacterium for hydrolyzing lactose, wherein said modified Corynebacterium comprises transgenes encoding polypeptides, wherein said polypeptides include
i) a modified lactose transporter, wherein the amino acid sequence of said modified lactose transporter has at least 80% sequence identity to SEQ ID NO: 2 wherein amino acid residue Pro148 is substituted by an amino acid selected from the group consisting of glycine, alanine, valine, cysteine, leucine, isoleucine, and methionine; and
ii) a polypeptide having beta-galactosidase activity (E.C. 3.2.1.23).

A second aspect of the invention provides a mutant gene encoding a modified lactose transporter wherein the amino acid sequence of said lactose transporter has at least 80% sequence identity to SEQ ID No: 2 wherein amino acid residue Pro148 is substituted by an amino acid selected from the group consisting of glycine, alanine, valine, cysteine, leucine, isoleucine, and methionine.

A third aspect of the invention provides a method for hydrolyzing lactose. In one embodiment, the hydrolysis of lactose is performed at elevated temperatures, the method comprising the steps of
a. bringing a genetically modified Corynebacterium into contact with a lactose-containing medium, wherein said modified Corynebacterium comprises transgenes encoding polypeptides, wherein said polypeptides comprise
   i. a lactose transporter and
   ii. a polypeptide having beta-galactosidase activity (E.C. 3.2.1.23),
b. incubating the culture of step (a) under aerobic conditions at a temperature between 20-37 degree Celsius,
c. increasing the temperature of the culture of step (b) to 50 degree Celsius or higher to reach an optimum temperature for said beta-galactosidase enzyme, and
d. optionally purifying products of lactose hydrolysis from the culture of step (c).

### DESCRIPTION OF THE INVENTION

### Description of the figures:

Figure 1: illustrates growth and lactose hydrolysis by a Coynebacterium of the present invention under different culture conditions. (A) aerobic growth at 30 °C, (B) anaerobic fermentation at 30 °C, and (C) lactose hydrolysis at 60 °C.
Figure 2: shows the growth of the reference strain, mutant strain (expressing lacSZ and galMKTE), and adapted mutant on different carbon sources over a period of 48 hours.
Figure 3: shows (A) Plasmid map of construct I comprising genes encoding xylose isomerase (xylA) and arabinose isomerase (araA), (B) Plasmid map of construct II comprising genes encoding xylose isomerase (xylA) and arabinose isomerase (araA), as well as four additional promoter fragments for their expression (trc core promoter), (C) Plasmid map of construct III comprising genes encoding xylose isomerase (xylA) and arabinose isomerase (araA), as well as four additional promoter fragments for their expression (trc core promoter), and further a gene encoding beta-galactosidase (lacZ).
Figure 4: shows the lactose hydrolysis profile of adapted mutant strains *C. glutamicum* JS0093AI, JS0093AII, and JS0093AIII, comprising constructs I, II, and III, respectively, over a period of 48 hours.
Figure 5: shows the maximum lactose hydrolysis rates by adapted mutant strains *C. glutamicum* JS0093AI, JS0093AII, and JS0093AIII, comprising constructs I, II, or III, respectively.
Figure 6: shows the maximum isomerization rates (productivity) of glucose to fructose and galactose to tagatose by adapted mutant strains *C. glutamicum* JS0093AI, JS0093AII, and JS0093AIII, comprising constructs I, II, or III, respectively.
Figure 7: shows the simultaneous production of fructose and tagatose from whey by *C. glutamicum* strain JS0093AIII

### Abbreviations, terms, and definitions:

**LacS:** gene encoding a polypeptide facilitating transport of lactose (a "lactose transporter").
**LacZ:** gene encoding a polypeptide having beta-galactosidase activity (E.C. 3.2.1.23 for hydrolyzing lactose to glucose and galactose.
**GalM:** gene encoding a polypeptide having galactose mutarotase activity (E.C. 5.1.3.3) for converting beta-D-galactose to alpha-D-galactose.
**GalK:** gene encoding a polypeptide having galactokinase activity (E.C. 2.7.1.6) for converting phosphorylating alpha-D-galactose to galactose-1-phosphase.
**GAlT:** gene encoding a polypeptide having galactose-1-phosphate uridylyltransferase activity (E.C. 2.7.7.10) for converting galactose-1-phosphate to glucose-1-phosphate.
**GalE:** gene encoding a polypeptide having UDP-galactose-4-epimerase activity (E.C. 5.1.3.2) for epimerizing UDP-galactose to UDP-glucose.

**"LacSZ"** means LacS and LacZ. LacSZ should only be considered a combined terminology for LacS and LacZ; it does not refer to whether or not the genes may be linked, such as e.g. transcriptionally or translationally linked. The genes may be fused or be present individually; they may be regulated, transcribed and/or translated dependently or independently.

**"galMKTE"** means galM, galk, galt and galE. galMKTE should only be considered a combined terminology for galM, galk, galt and galE; it does not refer to whether or not the genes may be linked, such as e.g. transcriptionally or translationally linked. The genes may be fused or be present individually; they may be regulated, transcribed, and/or translated dependently or independently.

**Amino acid sequence identity:** The term "sequence identity" as used herein, indicates a quantitative measure of the degree of homology between two amino acid sequences of substantially equal length. The two sequences to be compared must be aligned to give a best possible fit, by means of the insertion of gaps or alternatively, truncation at the ends of the protein sequences. The sequence identity can be calculated as ((Nref-Ndif)100)/(Nref), wherein Ndif is the total number of non-identical residues in the two sequences when aligned and wherein Nref is the number of residues in one of the sequences. Sequence identity can alternatively be calculated by the BLAST program e.g. the BLASTP program (Pearson W.R and D.J. Lipman (1988)) (www.ncbi.nlm.nih.gov/cgi-bin/BLAST). In one embodiment of the invention, alignment is performed with the sequence alignment method ClustalW with default parameters as described by Thompson J., et al 1994, available at http://www2.ebi.ac.uk/clustalw/.
Preferably, the numbers of substitutions, insertions, additions or deletions of one or more amino acid residues in the polypeptide as compared to its comparator polypeptide is limited, i.e. no more than 1, 2, 3, 4, 5, 6, 7, 8, 9, or 10 substitutions, no more than 1, 2, 3, 4, 5, 6, 7, 8, 9, or 10 insertions, no more than 1, 2, 3, 4, 5, 6, 7, 8, 9, or 10 additions, and no more than 1, 2, 3, 4, 5, 6, 7, 8, 9, or 10 deletions. Preferably the substitutions are conservative amino acid substitutions: limited to exchanges within members of group 1: Glycine, Alanine, Valine, Leucine, Isoleucine; group 2: Serine, Cysteine, Selenocysteine, Threonine, Methionine; group 3: Proline; group 4: Phenylalanine, Tyrosine, Tryptophan; Group 5: Aspartate, Glutamate, Asparagine, Glutamine.

**gi number:** (genInfo identifier) is a unique integer which identifies a particular sequence, independent of the database source, which is assigned by NCBI to all sequences processed into Entrez, including nucleotide sequences from DDBJ/EMBL/GenBank, protein sequences from SWISS-PROT, PIR and many others.

**Deleted gene:** the deletion of a gene from the genome of a microbial cell leads to a loss of function (knockout) of the gene and hence where the gene encodes a polypeptide the deletion results in a loss of expression of the encoded polypeptide. Where the encoded polypeptide is an enzyme, the gene deletion leads to a loss of detectable enzymatic activity of the respective polypeptide in the microbial cell. A deleted gene in the genome of a microbial cell is characterized by a loss of function due to the deletion of, or substitution of, or addition of, at least one nucleotide leading to a loss of expression of a polypeptide encoded by the gene.

**Oxygen deprived conditions:** are in the present context defined as growth conditions with such low oxygen content that cell proliferation is suppressed. The cell may consume a variety of sugars such as glucose, fructose, mannose and sucrose under oxygen deprived conditions to produce lactate, succinate and acetate while cellular proliferation remains suppressed during the bioconversion reaction.

**Aerobic conditions:** are in the present context defined as growth conditions where oxygen is present in sufficient amounts to support cell proliferation and growth of the cells.

**Value-added compounds:** are in the present context microbially synthesized compounds; they may be produced naturally by the microbes or production may be facilitated through genetic engineering of the microbes. Preferably they are produced by utilization of a low value substrate. The compounds may include (but are not limited to) organic acids, sugars, amino acids, peptides, alcohols, alkaloids, terpenoids, flavonoids, polyketides, biofuels, and other chemicals.

**Whey and whey permeate and residual whey permeate:** whey is a by-product of cheese manufacture; and comprises whey proteins having a high nutritional value and lactose. Removal of whey proteins, typically by means of ultrafiltration or diafiltration produces a whey protein concentrate and whey permeate that is lactose-rich. The lactose content of the whey permeate is dependent on the treatment conditions and typically it can reach as high as hundreds of grams per liter by reverse osmosis, such as 200 g/L. Removal of fat from whey, or from lactose-rich permeate, typically by centrifugation, yields a fat-free composition (whey or permeate). Residual whey permeate (also called permeate mother liquor) is obtained after the extraction of lactose from whey permeate (typically by lactose crystallisation); and has a lower lactose content of about 150 g/L.

### Detailed description of the invention:

### I. A genetically modified Corynebacterium capable of hydrolyzing lactose

The present invention provides a genetically modified Corynebacterium characterized by its ability to hydrolyze lactose.

### I i. Expression of transgenes for lactose hydrolysis

The bacterium of the invention is characterized by having transgenes LacSZ for lactose hydrolysis: (1) LacZ encoding a beta-galactosidase (E.C. 3.2.1.23) for hydrolysis of lactose to glucose and galactose and (2) LacS encoding a lactose transporter facilitating transport of lactose from the media into the cells.

The bacterium of the invention expresses a polypeptide having beta-galactosidase activity (E.C. 3.2.1.23) that converts lactose to glucose and galactose. The amino acid sequence of the polypeptide has at least 70, 72, 74, 76, 78, 80, 82, 84, 86, 88, 90, 92, 94, 96, 98, 99 or 100% sequence identity to the amino acid sequence of the beta-galactosidase encoded by the Streptococcus thermophilus LacZ gene (SEQ ID NO: 8). Alternatively, the amino acid sequence of the polypeptide having beta-galactosidase activity (E.C. 3.2.1.23), has at least 70, 72, 74, 76, 78, 80, 82, 84, 86, 88, 90, 92, 94, 96, 98, 99 or 100% sequence identity to an amino acid sequence selected from among SEQ ID NO: 10 (derived from Streptococcus infantarius CJ18) and SEQ ID NO: 12 (derived from Streptococcus salivarius CCHSS3).

The lacZ transgene encoding beta-galactosidase (E.C. 3.2.1.23) may be present in one or more copies on the genome, such as present in two copies on the genome, such as present in three, four, five or more copies on the genome. The lacZ transgene copies may be identical copies of the same gene or copies of different genes encoding polypeptides having beta-galactosidase activity.

The bacterium of the invention further expresses a polypeptide facilitating transport of lactose (a "lactose transporter").
According to one embodiment of the invention, the amino acid sequence of the lactose transporter has at least 70, 72, 74, 76, 78, 80, 82, 84, 86, 88, 90, 92, 94, 96, 98, 99 or 100% sequence identity to the amino acid sequence of the lactose transporter encoded by the Streptococcus thermophilus LacS gene (SEQ ID NO: 2). Alternatively, the amino acid sequence of the polypeptide facilitating transport of lactose, has at least 70, 72, 74, 76, 78, 80, 82, 84, 86, 88, 90, 92, 94, 96, 98, 99 or 100% sequence identity to the amino acid sequence selected from among SEQ ID NO: 4 (derived from Streptococcus salivarius HSISS4) and SEQ ID NO: 6 (derived from Streptococcus infantarius CJ18).

According to another embodiment of the invention, the amino acid sequence of the lactose transporter has at least 70, 72, 74, 76, 78, 80, 82, 84, 86, 88, 90, 92, 94, 96, 98, 99 or 100% sequence identity to the amino acid sequence of the lactose transporter encoded by the Streptococcus thermophilus LacS gene (SEQ ID NO: 2) wherein amino acid residue Pro148 is substituted by any amino acid other than proline, such as preferably substituted by a non-polar amino acid, such as preferably substituted by an amino acid selected from the group consisting of glycine, alanine, valine, cysteine, leucine, isoleucine, and methionine, such as preferably substituted by an amino acid selected from the group consisting of glycine, alanine, valine, leucine, and isoleucine, such as most preferably substituted by leucine.

Alternatively, the amino acid sequence of the polypeptide having beta-galactosidase activity (E.C. 3.2.1.23), has at least 70, 72, 74, 76, 78, 80, 82, 84, 86, 88, 90, 92, 94, 96, 98, 99 or 100% sequence identity to the amino acid sequence selected from among SEQ ID NO: 4 (derived from Streptococcus salivarius HSISS4) and SEQ ID NO: 6 (derived from Streptococcus infantarius CJ18), wherein amino acid residue Pro148 is substituted by Leu148 any amino acid other than proline, such as preferably substituted by a non-polar amino acid, such as preferably substituted by an amino acid selected from the group consisting of glycine, alanine, valine, cysteine, leucine, isoleucine, and methionine, such as preferably substituted by an amino acid selected from the group consisting of glycine, alanine, valine, leucine, and isoleucine, such as most preferably substituted by leucine..

### I ii. Expression of transgenes for galactose metabolism

Corynebacterium naturally metabolizes glucose, but lacks genes encoding enzymes required for galactose metabolism. For improved growth on the lactose hydrolysis products: glucose and galactose, it is preferred that the Corynebacterium of the present invention is able to metabolize both glucose and galactose.

In one embodiment, the bacterium of the invention is further characterized by having transgenes encoding one or more enzymes in the galactose metabolic pathway comprising (1) galM encoding galactose mutarotase (E.C. 5.1.3.3) for converting beta-D-galactose to alpha-D-galactose, (2) galK encoding galactokinase (E.C. 2.7.1.6) for phosphorylating alpha-D-galactose to yield galactose-1-phosphase, (3) galt encoding galactose-1-phosphate uridylyltransferase (E.C. 2.7.7.10) for converting galactose-1-phosphate to glucose-1-phosphate, and (4) galE encoding UDP-galactose-4-epimerase (E.C. 5.1.3.2) for epimerizing UDP-galactose to UDP-glucose.

According to the above, the bacterium of the invention may express a polypeptide having galactose mutarotase activity (E.C. 5.1.3.3) that converts beta-D-galactose to alpha-D-galactose, wherein the amino acid sequence of the polypeptide has at least 70, 72, 74, 76, 78, 80, 82, 84, 86, 88, 90, 92, 94, 96, 98, 99 or 100% sequence identity to the amino acid sequence of the galactose mutarotase encoded by the Lactococcus lactic subsp. cremoris NZ9000 galM gene (SEQ ID NO: 20). Alternatively, the amino acid sequence of the polypeptide having galactose mutarotase activity (E.C. 5.1.3.3), has at least 70, 72, 74, 76, 78, 80, 82, 84, 86, 88, 90, 92, 94, 96, 98, 99 or 100% sequence identity to an amino acid sequence selected from among SEQ ID NO: 22 (derived from Lactococcus lactic subsp. lactis) and SEQ ID NO: 24 (derived from Lactococcus lactis subsp. cremoris strain JM1).

Further according to the above, the bacterium of the invention may express a polypeptide having galactokinase activity (E.C. 2.7.1.6) that phosphorylates alpha-D-galactose to galactose-1-phosphase, wherein the amino acid sequence of the polypeptide has at least 70, 72, 74, 76, 78, 80, 82, 84, 86, 88, 90, 92, 94, 96, 98, 99 or 100% sequence identity to the amino acid sequence of the galactokinase encoded by the Lactococcus lactis subsp. cremoris NZ9000 galK gene (SEQ ID NO: 26). Alternatively, the amino acid sequence of the polypeptide having galactokinase activity (E.C. 2.7.1.6), has at least 70, 72, 74, 76, 78, 80, 82, 84, 86, 88, 90, 92, 94, 96, 98, 99 or 100% sequence identity to an amino acid sequence selected from among SEQ ID NO: 28 (derived from Lactococcus lactis subsp. lactis) and SEQ ID NO: 30 (derived from Lactococcus garvieae).

Further according to the above, the bacterium of the invention may express a polypeptide having galactose-1-phosphate uridylyltransferase activity (E.C. 2.7.7.10) that converts galactose-1-phosphate to glucose-1-phosphate, wherein the amino acid sequence of the polypeptide has at least 70, 72, 74, 76, 78, 80, 82, 84, 86, 88, 90, 92, 94, 96, 98, 99 or 100% sequence identity to the amino acid sequence of the galactose-1-phosphate uridylyltransferase encoded by the Lactococcus lactis subsp. cremoris NZ9000 galt gene (SEQ ID NO: 32). Alternatively, the polypeptide having galactose-1-phosphate uridylyltransferase activity (E.C. 2.7.7.10), has at least 70, 72, 74, 76, 78, 80, 82, 84, 86, 88, 90, 92, 94, 96, 98, 99 or 100% sequence identity to an amino acid sequence selected from among SEQ ID NO: 34 (derived from Lactococcus lactis subsp. cremoris KW2) and SEQ ID NO: 36 (derived from Lactococcus lactis subsp. lactis).

Further according to the above, the bacterium of the invention may express a polypeptide having UDP-galactose-4-epimerase activity (E.C. 5.1.3.2) that converts UDP-galactose to UDP-glucose, wherein the amino acid sequence of the polypeptide has at least 70, 72, 74, 76, 78, 80, 82, 84, 86, 88, 90, 92, 94, 96, 98, 99 or 100% sequence identity to the amino acid sequence of the UDP-galactose-4-epimerase encoded by the Lactococcus lactis subsp. cremoris NZ9000 galE gene (SEQ ID NO: 38). Alternatively, the amino acid sequence of the polypeptide having UDP-galactose-4-epimerase activity (E.C. 5.1.3.2), has at least 70, 72, 74, 76, 78, 80, 82, 84, 86, 88, 90, 92, 94, 96, 98, 99 or 100% sequence identity to an amino acid sequence selected from among SEQ ID NO: 40 (derived from Lactococcus lactis strain AI06) and SEQ ID NO: 42 (derived from Lactococcus garvieae).

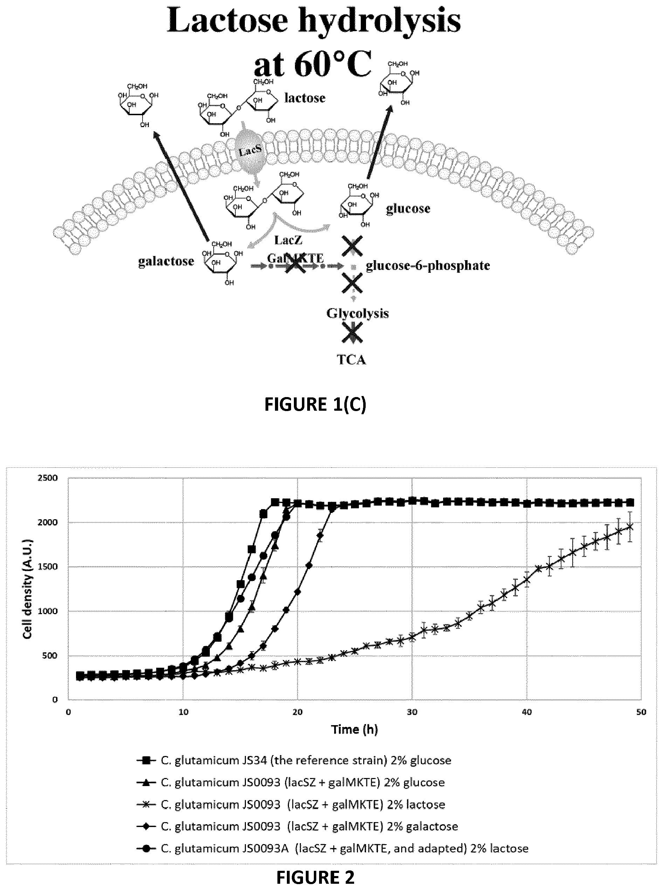

As mentioned previously, Corynebacterium cannot naturally hydrolyze and utilize lactose as carbon source to support growth of the cell. Figure 2 of Example 1 shows that genetically modifying reference Corynebacterium strain JS34 by introducing transgenes LacSZ and galMKTE as described above in embodiments of the invention, enables the modified Corynebacterium (strain JS0093) to grow on lactose. Further development of the strain JS0093 by adapted evolution (Example 2) yielded strain JS0093A which surprisingly grows much faster on lactose and even demonstrates growth rates on lactose comparable to growth rates of the reference Corynebacterium strain (JS34) cultured in glucose. The improvement was surprisingly identified to be caused by a single amino acid substitution in the lactose transporter polypeptides (Example 3); more specifically, the improvement is caused by amino acid residue Pro148 of the lactose transporter being substituted by Leu148 as specified previously in an embodiment of the invention.

### I iii. Expression of transgenes for production of value-added compounds

The bacterium of the invention may further be characterized by having transgenes for conversion of the products of lactose hydrolysis to value-added compounds, such as conversion of glucose and/or galactose to value-added products. In one embodiment, value-added compounds may be produced by making use of metabolic pathways within the bacterium, such as by overexpressing selected existing pathways or by introducing new pathways into the bacterium. Such transgenes encoding polypeptides whose enzyme activities are related to existing or introduced metabolic pathways within the cell may facilitate conversion of the lactose hydrolysis products to value-added products such as amino acids, alcohols, proteins, etc. In another embodiment, value-added compounds may be produced by introducing transgenes encoding peptides associated with enzymatic action unrelated to cellular metabolism, such as conversion of glucose and galactose to other value-added compounds such as other sugars, including but not limited to fructose and tagatose.

In one embodiment, the bacterium of the invention expresses one or more polypeptides having xylose/glucose isomerase activity (E.C. 5.3.1.5) for conversion of D-glucose to D-fructose and/or one or more polypeptides having arabinose isomerase activity (E.C. 5.3.1.4) for conversion of D-galactose to D-tagatose.

According to the above, the bacterium of the invention may express a polypeptide having xylose/glucose isomerase activity (E.C. 5.3.1.5) that converts D-glucose to D-fructose, wherein the amino acid sequence of the polypeptide has at least 70, 72, 74, 76, 78, 80, 82, 84, 86, 88, 90, 92, 94, 96, 98, 99 or 100% sequence identity to the amino acid sequence of the xylose isomerase encoded by the Arthrobacter sp XylA gene (SEQ ID NO: 44). Alternatively, the polypeptide having xylose isomerase activity (E.C. 5.3.1.5), has an amino acid sequence selected from among SEQ ID NO: 46 (derived from Arthrobacter Sp. N.R.R.L. B3728) and SEQ ID NO: 48 (derived from Arthrobacter sp. ZXY-2).

Further according to the above, the bacterium of the invention may express a polypeptide having xylose/glucose isomerase activity (E.C. 5.3.1.5) that converts D-glucose to D-fructose, such as a polypeptide selected from the group consisting of D-glucose-6-phosphate-ketol-isomase, D-glucose ketol-isomerase, and glucose isomerase.

Even further according to the above, the bacterium of the invention may express a polypeptide having arabinose isomerase activity (E.C. 5.3.1.4) that converts D-galactose to D-tagatose. The amino acid sequence of the polypeptide has at least 70, 72, 74, 76, 78, 80, 82, 84, 86, 88, 90, 92, 94, 96, 98, 99 or 100% sequence identity to the amino acid sequence of the codon optimized arabinose isomerase originating from Bacillus coagulans (SEQ ID NO: 50).

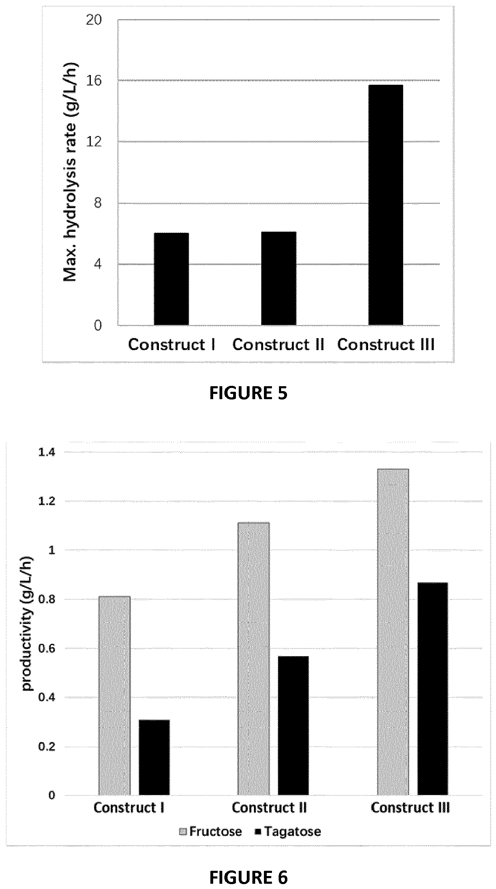

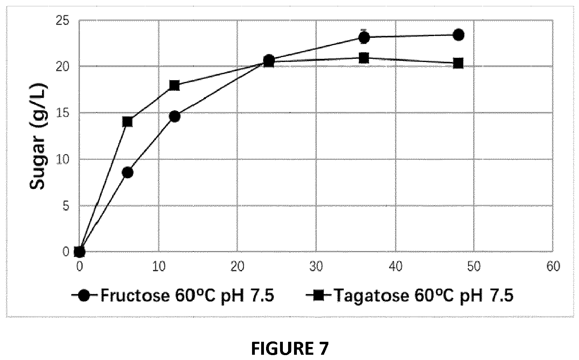

Production of value-added compounds as provided in one embodiment of the invention by introducing transgenes encoding peptides associated with enzymatic action unrelated to cellular metabolism is demonstrated in Examples 5 and 6, where transgenes encoding polypeptides having xylose isomerase and arabinose isomerase activities were introduced into a Cyanobacterium of the present invention. Figures 6 and 7 show the effects of the transgenes, reported as isomerization rates for conversion of glucose to fructose and galactose to tagatose, as well as yields of fructose and tagatose from whey.

### I iv. Deletion of endogenous genes to block cell metabolism at oxygen deprived conditions

According to one embodiment of the invention, the bacterium may be characterized by having a sugar metabolism that is turned off under oxygen deprived or anaerobic conditions; this is facilitated by deletion of one or more genes encoding enzymes of the lactate pathway.

Deletion of the lactate pathway: According to one embodiment, the bacterium of the invention is characterized by knockouts of one or more endogenous native genes encoding a polypeptide having lactate dehydrogenase activity causing a block in the lactate synthesis pathway in the bacterium. Deletion of at least one gene encoding a lactate dehydrogenase enzyme (E.C 1.1.1.27) provides a bacterium of the invention that is depleted in lactate production. For example, where the Corynebacterium of the invention belongs to a given species, the deleted endogenous gene is one encoding a polypeptide having lactate dehydrogenase activity in the respective species of Corynebacterium. Preferably the amino acid sequence of the polypeptide having lactate dehydrogenase activity (E.C 1.1.1.27) has at least 70, 72, 74, 76, 78, 80, 82, 84, 86, 88, 90, 92, 94, 96, 98, 99 or 100% amino acid sequence identity to a sequence selected from among: SEQ ID NO: 14 in Corynebacterium glutamicum, SEQ ID NO: 16 in Corynebacerium callunae, and SEQ ID NO: 18 in Corynebacterium kutscheri.

In one embodiment, an endogenous gene, encoding a polypeptide having lactate dehydrogenase enzymatic activity (E.C 1.1.1.27), is deleted from the bacterium of the invention. For example, where the bacterium of the invention belongs to the species Corynebacterium glutamicum, the deleted lactate dehydrogenase gene encodes a polypeptide whose sequence has at least 70, 72, 74, 76, 78, 80, 82, 84, 86, 88, 90, 92, 94, 96, 98, 99 or 100% amino acid sequence identity to SEQ ID NO: 14. Further, for example, where the bacterium of the invention belongs to the species Corynebacterium callunae, the deleted lactate dehydrogenase gene encodes a polypeptide whose sequence has at least 70, 72, 74, 76, 78, 80, 82, 84, 86, 88, 90, 92, 94, 96, 98, 99 or 100% amino acid sequence identity to SEQ ID NO: 16. Even further, for example, where the bacterium of the invention belongs to the species Corynebacerium kutscheri, the deleted lactate dehydrogenase gene encodes a polypeptide whose sequence has at least 70, 72, 74, 76, 78, 80, 82, 84, 86, 88, 90, 92, 94, 96, 98, 99 or 100% amino acid sequence identity to SEQ ID NO: 18.

Deletion of an endogenous gene encoding a polypeptide having lactate dehydrogenase (ldh) enzymatic activity as provided in one embodiment of the invention is demonstrated in Example 4. The advantageous effect of deleting the ldh gene is reported in Table 1, showing complete elimination of lactate formation and an increase in sugar recovery rate as compared to a strain comprising the native ldh gene.

### II. A genetically modified Corynebacterium capable of hydrolyzing lactose

The genetically modified bacterium according to the invention, capable of hydrolyzing lactose, is a member of the genus Corynebacterium. The bacterium of the invention may for example be a Corynebacterium species selected from the group consisting of C. accolens, C. afermentans, C. ammoniagenes, C. amycolatum, C. argentoratense, C. aquaticum, C. auris, C. bovis, C. diphtheria, C. equi (now Rhodococcus equi), C. efficiens, C. flavescens, C. glucuronolyticum, C. glutamicum, C. granulosum, C. haemolyticum, C. halofytica, C. kroppenstedtii, C. jeikeium (group JK), C. macginleyi, C. matruchotii, C. minutissimum, C. parvum (Propionibacterium acnes), C. paurometabolum, C. propinquum, C. pseudodiphtheriticum (C. hofmannii), C. pseudotuberculosis, C. pyogenes - Trueperella pyogenes, C. urealyticum (group D2), C. renale, C. resistens, C. spec, C. striatum, C. tenuis, C. ulcerans, C. urealyticum, C. uropygiale, and C. xerosis.

Preferably, the bacterium of the invention is selected from the group consisting of Corynebacterium glutamicum, Corynebacterium acetoacidophilum, Corynebacterium callunae, and Corynebacterium ammoniagenes.

Even more preferably the bacterium of the invention is Corynebacterium glutamicum.

### III. Methods for producing a Corynebacterium of the invention

### III i. Introduction of transgenes

Cells of a micro-organism are genetically engineered by the introduction of heterologous DNA (RNA) into the cells. A nucleic acid molecule, that encodes one or more polypeptide(s) associated with lactose hydrolysis, galactose metabolism, and/or conversion of lactose sugars to value added compounds according to the invention, can be introduced into a cell or cells and optionally integrated into the host cell genome using methods and techniques that are standard in the art. For example, nucleic acid molecules can be introduced by standard protocols such as transformation including chemical transformation and electroporation, transduction, particle bombardment, etc.

In some embodiments, one or more of the genes encoding one or more polypeptide associated with the invention is expressed in a recombinant expression vector. As used herein, a "vector" may be any of a number of nucleic acids into which a desired sequence or sequences may be inserted by restriction and ligation for transport between different genetic environments or for expression in a host cell. Vectors are typically composed of DNA, although RNA vectors are also available. Vectors include, but are not limited to: plasmids, fosmids, phagemids, virus genomes and artificial chromosomes. A suitable vector includes one which is able to replicate autonomously (self-replicating vector) or is integrated (integration vector) in the genome in a host cell, and which is further characterized by one or more endonuclease restriction sites at which the vector may be cut in a determinable fashion and into which a desired DNA sequence may be ligated such that the new recombinant vector retains its ability to replicate in the host cell. In the case of plasmids, replication of the desired sequence may occur many times as the plasmid increases in copy number within the host cell such as a host bacterium; or may occur just a single time per host before the host reproduces by mitosis. In the case of phage, replication may occur actively during a lytic phase or passively during a lysogenic phase.

Vectors and other tools suitable for cloning and introducing one or more gene(s) encoding one or more a polypeptide(s) associated with lactose hydrolysis, galactose metabolism, and/or conversion of lactose sugars to value added compounds by a Corynebacterium according to the invention are commercially available and known to those skilled in the art [e.g., Sambrook et al., 1989].

The precise nature of the regulatory sequences needed for gene expression may vary between species or cell types, but shall in general include, as necessary, 5' non-transcribed and 5' non-translated sequences involved with the initiation of transcription and translation respectively, such as a TATA box, capping sequence, CAAT sequence, and the like. In particular, such 5' non-transcribed regulatory sequences will include a promoter region which includes a promoter sequence for transcriptional control of the operably joined gene. Expression of the nucleic acid molecule(s) encoding the polypeptide(s) of the claimed invention may be inducible, such as promoters controlled by the presence or absence of a molecule, or constitutive i.e. the promoter is unregulated allowing for continual transcription of its associated gene. The promoter can be a native promoter, i.e., the promoter of the gene in its endogenous context, which provides normal regulation of expression of the gene. In some embodiments the promoter can be constitutive. Regulatory sequences may also include enhancer sequences or upstream activator sequences as desired. The vectors of the invention may optionally include 5' leader or signal sequences. The choice and design of an appropriate expression system is within the ability and discretion of one of ordinary skill in the art.

Expressing the nucleic acid molecule(s) encoding the polypeptide(s) of the claimed invention may be accomplished by integrating the nucleic acid molecule into the genome. Expressing the nucleic acid molecule(s) encoding the polypeptide(s) of the claimed invention may be accomplished by maintaining the nucleic acid molecule(s) on a vector.

Genetic tools developed for metabolic optimization of Corynebacterium may preferably be applied, such as the synthetic promoter libraries by [Rytter et al 2014] where the interspace nucleotides between the -10 and -35 regions of the promoters are randomized, providing the ability to modulate gene expression; and further the repetitive chromosomal integration system by [Shen et al 2017], facilitated by a vector containing a synthetic cassette including a phage attachment site attP for integration, a bacterial attachment site attB for subsequent integration, a MCS, and two modified loxP sites to facilitate easy removal of undesired vector elements. When the Corynebacterium has an attB site inserted in its chromosome, the vector can easily integrate into the attB site by the corresponding integrase. The phase integrase, as well as Cre recombinase for promoting recombination between the loxP sites, may be expressed by non-replicating circular expression units.

Methods of heterologous expression of genes encoding one or more polypeptide(s) associated with lactose hydrolysis, galactose metabolism, and/or conversion of lactose sugars to value added compounds by a Corynebacterium of the invention is demonstrated in Examples 1 and 5.

### III ii. Deletion of endogenous gene(s)

Deletion of endogenous genes in a host Corynebacterium can be achieved by a variety of methods; for example by transformation of the host cell with linear DNA fragments containing a locus for resistance to an antibiotic, or any other gene allowing for rapid phenotypic selection, flanked by sequences homologous to closely spaced regions on the cell chromosome on either side of the gene to be deleted. By selecting for a double-crossover event between the homologous sequences, shown by the antibiotic resistance or other detectable phenotype, a chromosome disruption can be selected for which has effectively deleted an entire gene. Further inactivation or deletion of the recA gene can prevent recombination or incorporation of extrachromosomal elements from occurring, thereby resulting in a bacterial strain which is useful for screening for functional activity or production of genetically engineered proteins in the absence of specific contaminants. An example describing the deletion of lactate dehydrogenase (ldh) from Corynebacterium glutamicum given in Example 4, illustrates one method for deleting endogenous genes.

### IV. Use of the genetically modified Corynebacterium of the invention for hydrolyzing lactose

The genetically modified Corynebacterium of the invention will hydrolyze lactose to glucose and galactose when supplied with a suitable source of lactose. Suitable lactose sources may comprise milk, butter, cheese, yoghurt, whey, whey permeate, residual whey permeate, and whey mother liquor.

### V. Use of the genetically modified Corynebacterium of the invention for producing value-added compounds

The genetically modified Corynebacterium capable of hydrolyzing lactose may according to one embodiment of the invention be further genetically modified to produce other value-added compounds from the lactose hydrolysis products. Glucose and galactose may be converted to other value-added compounds, such as (1) mediated by the native metabolic apparatus of the genetically modified Corynebacterium, where the sugars are consumed and converted into value-added products such as amino acids, alcohols, proteins, or (2) by enzymatic action unrelated to cellular metabolism, such as by means of one or more transgene(s) expressed by the genetically engineered Corynebacterium of the invention, that encode one or more polypeptide(s) having enzymatic activity associated with conversion of glucose and galactose to other value-added compounds such as other sugars, including but not limited to fructose and tagatose.

### VI. Methods for hydrolyzing lactose and (optionally) producing value-added compounds using the genetically modified Corynebacterium of the invention

Growth of the Corynebacterium, hydrolysis of lactose, and optionally production of value-added compounds may occur concurrently or at different stages.

In one embodiment, the genetically modified Corynebacterium of the invention is cultured under conditions favorable for growth of the Corynebacterium as one of ordinary skill in the art would recognize; growth, hydrolysis of lactose, and optionally production of value-added compounds will occur concurrently.

In another embodiment, the genetically modified Corynebacterium of the invention is first cultured under conditions favorable for growth of the Corynebacterium as one of ordinary skill in the art would recognize; then when a desired amount of cell biomass is reached, the culture conditions are changed to limit cell metabolism and favour hydrolysis and (optionally) conversion of the resulting sugars to value-added compounds, as further described below.

### VI i. Hydrolysis of lactose and production of value-added compounds at elevated temperature

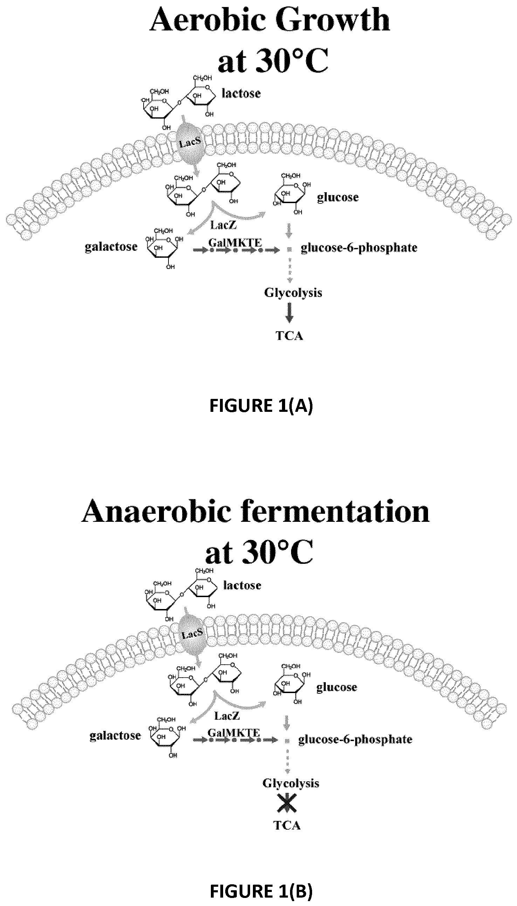

In one aspect of the invention, the Corynebacterium of the invention is first cultured at a mesophilic temperature to obtain an increase in cell biomass, such as at a temperature between 20-37 degree Celsius, between 25-35 degree Celsius, between 28-35 degree Celsius; or such as preferably at 30 degree Celsius, providing optimal growth conditions for the Corynebacterium (Figure 1(A)). The temperature is then increased to favor enzyme activity of the beta-galactosidase polypeptide encoded by the transgene expressed by the genetically engineered Corynebacterium of the invention (Figure 1(C)). By increasing the temperature, metabolism of the sugar products (glucose and/or galactose) by the Coynebacterium will cease due to cessation of cell growth or even cell death, but hydrolysis of lactose will continue as well as optionally conversion of the sugars to value-added products by the enzymatic activities expressed by the Corynebacterium. The initial mesophilic culturing temperature is chosen as one of ordinary skill in the art would recognize to favor growth of the Corynebacterium. At a chosen time, the temperature may then be increased to a preferred temperature of the heterologous expressed beta-galactosidase enzyme, such as increased to 40, 42, 45, 48, 50, 52, 55, 58, 60°C or even higher if thermostable enzymes are used. Hydrolysis and optionally sugar conversion is allowed to proceed during incubation at the chosen temperature for a selected time period, whereafter the value-added products may be purified.

In one embodiment of the invention, a method for hydrolyzing lactose comprises the steps of (a) bringing a genetically modified Corynebacterium of the invention into contact with a lactose-containing medium, (b) incubating the culture of step (a) under aerobic conditions at temperature 20-37 degree Celsius, (c) increasing temperature of the culture of step (b) to 50 degree Celsius or higher to reach optimum temperature for said beta-galactosidase enzyme, and (d) optionally purifying products of lactose hydrolysis from the culture of step (c), said products comprising any one of glucose, galactose, fructose, tagatose or a combination of any two or more.

### VI ii. Hydrolysis of lactose and production of value-added compounds under oxygen deprived conditions

In another aspect of the invention, the genetically modified Corynebacterium of the invention, which is deleted for endogenous genes essential for the lactate pathway, is first cultured under aerobic conditions to obtain an increase in cell biomass. At a chosen time, culture conditions are then changed to anaerobic or oxygen-deprived conditions whereby no more of the sugar products (glucose and/or galactose) will be metabolized by the Coynebacterium (Figure 1(B)), instead favoring continued hydrolysis of lactose and optionally the conversion of the sugars to value-added products by the enzymatic activities of the polypeptide(s) encoded by the transgene(s) expressed by the genetically engineered Corynebacterium of the invention. Hydrolysis and optionally sugar conversion is allowed to proceed during incubation for a selected time period, whereafter the value-added products may be purified.

In one embodiment of the invention, a method for hydrolyzing lactose comprises the steps of (a) bringing a genetically modified Corynebacterium of the invention into contact with a lactose-containing medium, specifically a bacterium lacking genes essential for the lactate pathway, such as lacking genes encoding polypeptides having an enzymatic activity of lactate dehydrogenase (E.C. 1.1.1.27), (b) incubating the culture of step (a) under aerobic conditions, (c) shifting culture conditions to incubate the culture obtained in (b) under oxygen-deprived culture conditions, and (d) optionally purifying products of lactose hydrolysis from the culture of step (c), said products comprising any one of glucose, galactose, fructose, tagatose or a combination of any two or more.

In a preferred embodiment of the invention, the two methods for hydrolysing lactose (at increased temperature and oxygen-deprived conditions) may be combined, such that the method for hydrolyzing lactose comprises the steps of (a) bringing a genetically modified Corynebacterium of the invention into contact with a lactose-containing medium, specifically a bacterium lacking genes essential for the lactate pathway, such as lacking genes encoding polypeptides having an enzymatic activity of lactate dehydrogenase (E.C. 1.1.1.27), (b) incubating the culture of step (a) under aerobic conditions at temperature 20-37 degree Celsius, (c) increasing the temperature to 50 degree Celsius or higher to reach optimum temperature for said beta-galactosidase enzyme and shifting culture conditions to incubate the culture obtained in (b) under oxygen-deprived culture conditions, and (d) optionally purifying products of lactose hydrolysis from the culture of step (c), said products comprising any one of glucose, galactose, fructose, tagatose or a combination of any two or more.

### VII. A method of detecting products produced by the genetically modified Corynebacterium

Methods for detecting and quantifying sugars produced by a Corynebacterium of the invention include high performance liquid chromatography (HPLC) combined with refractive index detection to identify and quantify the product compared to standards, as one of ordinary skill in the art would be familiar with. Example 4 comprises the outline of one method of detection and quantification of sugars.

### VIII. A mutant gene encoding a modified lactose transporter

The invention provides a mutant gene encoding a modified lactose transporter, wherein the amino acid sequence of said lactose transporter has at least 80% sequence identity to SEQ ID No. 2 (derived from Streptococcus thermophilus) wherein amino acid residue Pro148 is substituted by any amino acid other than proline, such as preferably substituted by a non-polar amino acid, such as preferably substituted by an amino acid selected from the group consisting of glycine, alanine, valine, cysteine, leucine, isoleucine, and methionine, such as preferably substituted by an amino acid selected from the group consisting of glycine, alanine, valine, leucine, and isoleucine, such as most preferably substituted by leucine.

In one embodiment, the invention provides a mutant gene encoding a modified lactose transporter, wherein the amino acid sequence of said lactose transporter has at least 82, 84, 86, 88, 90, 92, 94, 96, 98, 99 or 100% sequence identity to the amino acid sequence of the lactose transporter encoded by the Streptococcus thermophilus LacS gene (SEQ ID NO: 2) wherein amino acid residue Pro148 is substituted by any amino acid other than proline, such as preferably substituted by a non-polar amino acid, such as preferably substituted by an amino acid selected from the group consisting of glycine, alanine, valine, cysteine, leucine, isoleucine, and methionine, such as preferably substituted by an amino acid selected from the group consisting of glycine, alanine, valine, leucine, and isoleucine, such as most preferably substituted by leucine.

In a preferred embodiment, the amino acid sequence encoding a modified lactose transporter of the invention has at least 80% sequence identity to SEQ ID No: 2 (derived from Streptococcus thermophilus) wherein amino acid residue Pro148 is substituted by Leu148.

### EXAMPLES

### Example 1: Genetic modification of a Corynebacterium glutamicum strain to enable efficient growth on lactose

The genetic modifications required to produce a C. glutamicum strain capable of growing on lactose include introduction of transgenes for (i) lactose uptake as well as (ii) lactose hydrolysis to glucose and galactose. Further, introduction of transgenes for (iii) galactose utilization enables the strain to utilize both sugars derived from lactose hydrolysis.

These transgenes were inserted on the chromosome of C. glutamicum via a site-specific integration as described below.

### 1.a. Host strains and plasmids

The wild-type Corynebacterium glutamicum strain ATCC 13032 modified by insertion of a bacterial attachment site attB for subsequent site-integration in its chromosome was used as parent strain for the genetic construction of a strain capable of growing on lactose. This strain is referred to as strain **JS34** [Shen et al., 2017]. E. coli strain TOP10 was used for the cloning purposes. Plasmid pJS31 [Shen et al., 2017] containing a cassette for repetitive integration into the chromosome of Corynebacterium glutamicum **JS34** (or other strains carrying the aatB site) was used for integration of transgenes. AatP and attB sites in the cassette enable phage TP901-1 integrase-mediated integration, while loxP sites on each end of the cassette guides Cre-recombinase-facilitated recombination. The cassette further comprises a MCS, an origin of replication, and a kanamycin resistance gene as well as a gene conferring sucrose sensitivity, which both can be used for counter selection.

### 1.b. Inserting transgenes LacSZ

An expression library comprising an operon of LacSZ (a lacS gene (SEQ ID NO: 1, encoding lactose transporter) and a lacZ gene (SEQ ID NO: 7, encoding beta-galactosidase) both from Streptococcus thermophilus), was PCR amplified using the synthetic promoter technology developed for C. glutamicum [Rytter et al., 2014]. Briefly described, degenerate primers harboring synthetic promoter sequences, in which the interspace nucleotides between the -10 and -35 regions of a promoter are randomized, were assembled to the target genes using PCR amplification. The amplicons comprised a library, where the target genes were fused to synthetic promoters with different activities [Rytter et al., 2014]. The library was cloned into the Xbal site of the vector pJS31 [Shen et al., 2017], and first transformed into *E. coli* TOP10. The *E. coli* cells were propagated on the LB agar with 50 µg/mL kanamycin. The colonies on the agar plates were washed in 0.9% saline solution, and the plasmid library was recovered using GeneJET Plasmid Kit (Thermo Fisher Scientific). The DNA sequence of the TP901-1 integrase was PCR amplified and cyclized using T4-ligase. The library was co-transformed into the *C. glutamicum* strain JS34 with the cyclized TP901-1 integrase sequence. The *lacSZ* library was integrated into the *att*B site on the chromosome of *C. glutamicum* upon the expression of the TP901-1 integrase [Shen et al., 2017]. The lactose-utilizing transformants were screened on the CGXII agar [Eggeling & Bott, 2005] containing 1% lactose. Afterwards, the marker genes on the chromosome were excised when the Cre recombinase was expressed in *C. glutamicum* [Shen et al., 2017].

### 1.c. Inserting transgenes galMKTE

The insertion of the galMKTE operon into the *C. glutamicum* genome was performed using the same procedure as described for lacSZ, except that screening was performed on the CGXII agar containing 1% galactose. Here, the expression library comprised a galM gene (SEQ ID NO: 19, encoding galactose mutarotase), a galK gene (SEQ ID NO: 25, encoding galactokinase), a galt gene (SEQ ID NO: 31, encoding galacose-1-phosphate uridylyltransferase), and a galE gene (SEQ ID NO: 37, encoding UDP-glucose 4-epimerase) all from Lactococcus lactis.

The C. glutamicum strain comprising transgenes LacSZ and galMKTE is referred to as strain **JS0093**.

### Example 2: Improving growth on lactose by adapted evolution

### 2.a. Adapted evolution

Adapted evolution of C. glutamicum strain **JS0093** was conducted using a serial-transfer regime. More specifically, a single colony of strain **JS0093** from BHI agar (Sigma) was inoculated into a test tube containing 5 ml CGXII medium [Eggeling & Bott, 2005] with 2% lactose. The tube was incubated in a rotary shaking machine (220 rpm) at 30°C. When the culture entered the stationary phase, it was diluted 100 times in 5 mL fresh medium, and cultivated under same conditions. The passage was repeated 15 times.
When the evolution was ceased after 15-time passages, cultures were plated on the BHI agar. Five colonies were randomly picked and their growth on CGXII with 2% lactose was tested using a microbioreactor (Biolector), and compared to the parent strain **JS0093.** The strain with the highest growth rate was chosen for further development.

### 2.b. Properties of adapted C. glutamicum strain JS0093A

Growth experiments were performed in a 1 ml microbioreactor (Biolector), testing different carbon sources. The adapted strain **JS0093A** was compared to strain **JS0093** and reference strain **JS34**. The strains were inoculated in 1 ml BHI broth (beef heart infusion, 5 g/L; calf brains infusion, 12.5 g/L; disodium hydrogen phosphate, 2.5 g/L; glucose, 2 g/L; peptone, 10 g/L; sodium chloride, 5 g/L; pH = 7.4) was inoculated into shake flasks with 50-ml CGXII (in 1 liter: 20 g (NH₄)2SO₄; 5 g urea; 1 g KH₂PO₄; 1 g K₂HPO₄; 0.25 g MgSO₄×7H₂O; 42 g MOPS; 1ml CaCl₂ (10 g/L); 1 ml biotin (0.2 g/ml); 1 ml trace elements (10 g/L FeSO₄×7 H₂O; 10 g/L MnSO₄×H₂O; 1 g/L ZnSO₄×7 H₂O; 0.2 g/L CuSO₄; 0.02 g/L NiCl₂×6 H₂O)) [Eggeling & Bott, 2005] supplied with 2% carbon source (lactose, glucose or galactose), incubated at 30 °C, 1500 rpm for 48 hours. Cell density was measured and recorded every 20 minutes. The results are compiled in Figure 2.

The growth rate of strain **JS0093** when cultured in glucose (Figure 2, triangle) and galactose (Figure 2, diamond), respectively, is only slightly reduced compared to the reference strain **JS34** cultured in glucose (Figure 2, square). Meanwhile, when strain **JS0093** is cultured in lactose (Figure 2, asterisk), a large reduction of the growth rate compared to the reference strain **JS34** is observed. Similar growth profiles were observed by Barrett et al. 2004 when lactose permease and beta-galactosidase were expressed in C. glutamicum, which indicates that either lactose transport or hydrolysis could be the bottleneck for C. glutamicum growing on lactose.

By adaptive evolution, the faster growing lactose-metabolizing mutant strain **JS0093A** was identified. **JS0093A** grew much faster on lactose (Figure 2, circle) compared to the parent strain **JS0093**. Cultured in lactose, adapted strain **JS0093A** even demonstrates growth rates comparable to the reference strain **JS34** cultured in glucose.

### Example 3: Genome sequencing of adapted strain JS0093A

The procedure for sample preparation, genome sequencing and data analysis has been described previously [Chen et al 2017]. Genomic DNA was purified from the mutant using a DNeasy blood and tissue kit (Qiagen, Hilden, Germany), and the quality was assessed using DNA electrophoresis and a NanoDrop 1000 spectrophotometer (Thermo Fisher Scientific, Waltham, MA, USA). Genome sequencing was performed by Macrogen (Seoul, South Korea). Briefly, 2 µg of genomic DNA was randomly sheared using a nebulizer (Illumina, San Diego, CA, USA), and the ends were repaired using polynucleotide kinase (Illumina) and Klenow enzyme (Illumina). The 5' ends of the DNA fragments were phosphorylated, and a single adenine base was added to the 3' ends using Klenow exo+ (Illumina). Following the ligation of a pair of Illumina adaptors to the repaired ends, the DNA was amplified in 10 cycles using adaptor primers (Illumina), and fragments of approximately 150 bp were isolated using agarose gel electrophoresis. Sequencing libraries were quantified with a 2100 BioAnalyzer DNA 1000 chip (Agilent, Santa Clara, CA, USA), as well as the PicoGreen fluorescence assay (Invitrogen, Carlsbad, CA, USA). Clusters were generated on an Illumina cluster station (Illumina) using 11 pmol of sequencing libraries. Thirty-eight sequencing cycles were performed using the Illumina Genome Analyzer IIx system (Illumina) according to the manufacturer's specifications. CLC Genomics Workbench (Qiagen, Hilden, Germany) was used to map the reads, detect single-nucleotide variations (SNVs) and insertion/deletion events (INDELs), and identify genomic rearrangements.

Only one mutation was identified in the mutant **JS0093A**. The mutation was a single nucleotide variation (SNV), and found in the lactose transport gene *LacS* (C to T in position 443 of the gene), resulting in a change of the deduced amino acid sequence (Pro to Leu in position 148).

The mutated lactose transporter LacS gene (C to T in position 443 of the gene; Pro to Leu in position 148 of the corresponding amino acid sequence) identified in strain **JS0093A** will be referred to as LacS*

The difference in cell membrane structure among different species of bacteria makes it difficult to express membrane proteins, such as lactose transporter/permease, heterologously. As the lactose transporter/permease expressed in C. glutamicum originate from E. coli [Barrett et al 2004; Brooker, 1990] or S. thermophilus (this study), the variations in the chemical structure of bilayer lipids can profoundly affect the activity of the lactose transporter/permease [Brooker, 1990]. The occurrence of beneficial mutations for the growth on lactose in the lacS gene indicates that the native lactose transporter from S. thermophilis is not optimized for function in C. glutamicum, which represents the rate-limiting step for the engineered C. glutamicum growing well on lactose.

### Example 4: Lactose hydrolysis by modified Corynebacterium glutamicum

Two different hydrolysis conditions were tested: Hydrolysis at elevated temperature and hydrolysis under oxygen deprived conditions.

### 3.a. Deletion of ldh gene

In order to eliminate sugar metabolism by the Corynebacterium strain **JS0093A** under oxygen deprived conditions, the lactate dehydrogenase gene was deleted.

The ldh gene (SEQ ID NO: 13, encoding lactate dehydrogenase) was deleted based on the method of homologous recombination [Schäfer et al 1994]. The 800-bp upstream and downstream fragments of the ldh gene were assembled and inserted into the XbaI site of the vector pK18-mobsacB [Schäfer et al 1994]. The construct was transformed into *C*. *glutamicum.* The construct was integrated into the chromosome through either of the 800-bp homologous arms, and the mutants were selected on the BHI agar supplied with 25 µg/ml kanamycin. Afterwards, the counter selection for the mutants was performed on the BHI agar supplied with 10% sucrose. The ldh-deleted mutant was verified by PCR and Sanger sequencing.

### 3.b Cultivation

Independent of the later hydrolysis condition, the initial cultivation conditions was a follows: 1-ml overnight culture of C. glutamicum strain in BHI broth (beef heart infusion, 5 g/L; calf brains infusion, 12.5 g/L; disodium hydrogen phosphate, 2.5 g/L; glucose, 2 g/L; peptone, 10 g/L; sodium chloride, 5 g/L; pH = 7.4) was inoculated into shake flasks with 50-ml CGXII (per 1 liter: 20 g (NH₄)₂SO₄; 5 g urea; 1 g KH₂PO₄; 1 g K₂HPO₄; 0.25 g MgSO₄×7H₂O; 42 g MOPS; 1ml CaCl₂ (10 g/L); 1 ml biotin (0.2 g/ml); 1 ml trace elements (10 g/L FeSO₄×7 H₂O; 10 g/L MnSO₄×H₂O; 1 g/L ZnSO₄×7 H₂O; 0.2 g/L CuSO₄; 0.02 g/L NiCl₂×6 H₂O)) [Eggeling & Bott, 2005] supplied with 5% lactose and 1mM IPTG . After 24-hour cultivation at 30°C, 220 rmp; the cells were then centrifuged at 5,000 g for 10 minutes; and the pellets were washed once with 0.9% NaCl solution.

### 3.c Hydrolysis

For the hydrolysis of lactose, the cell pellets were resuspended at a concentration of 15 g (cell dry weight)/L in 40 mM (3-(N-morpholino) propanesulfonic acid) (MOPS) solution (pH=7.5) containing 10% lactose, and incubated under static, oxygen deprived conditions at 50°C.

### 3.d Analysis of sugar products

The concentrations of sugars (carbohydrate monomers and dimers) and lactate were determined using an Ultimate 3000 high-pressure liquid chromatography system (Dionex, Sunnyvale, CA) equipped with an Aminex HPX-87H column (Bio-Rad, Hercules, CA) and a Shodex RI-101 detector (Showa Denko KK, Tokyo, Japan). The column oven temperature was set to 30°C, and the mobile phase consisted of 5mM H2SO4 with a flowrate of 0.5 ml/min.

### 3.e Results

**Table 1. The difference in sugar recovery and lactate production between the strains with and without ldh deletion.**

| Strain | Hydrolysis condition | Initial total sugar content (g/l) | Sugar content after 24h hydrolysis (g/l) | Lactate produce d (g/l) | Sugar recovery % |
|---|---|---|---|---|---|
| **JS0093A** (lacS*Z +galMKT) | Elevated temp and oxygen deprived | 114.20 ± 0.86 | 108.13 ± 0.59 | 3.05 ± 0.06 | 94.68 |
| **JS0093A Δldh** (lacS*Z + galMKT, ldh deleted) | Elevated temp and oxygen deprived | 114.20 ± 0.86 | 111.01 ± 0.07 | 0 ± 0 | 97.21 |

Lactate was found to be a major by-product of lactose hydrolysis by the strain **JS0093A** comprising the native ldh gene. When lactate dehydrogenase was still active in C. glutamicum, 3.2 g/L lactate was produced after a 24-hour lactose hydrolysis at 50°C (Table 1). Deletion of the ldh gene resulted in a complete elimination of lactate formation, and the sugar recovery rate was raised to 97.2% in strain **JS0093A Δldh** compared to the strain **JS0093A** comprising the native ldh (94.7%).

The activities of native glycolytic enzymes are not immediately inactivated upon the rise of temperature above 50°C. Howell et al. show that most glycolytic enzymes from mesophilic bacteria maintain 50% activities after a 0.5-hour incubation at the temperatures above 50°C [Howell et al. 1969]. In C. glutamicum, the lactate dehydrogenase is the major enzyme participating in the regeneration of NAD⁺, which propels the catabolism of sugars via glycolysis under anoxic conditions [Tsuge et al., 2015]. The deletion of ldh shifts the sugar catabolism via glycolysis, and increases the sugar recovery after hydrolysis (Table 1). Hence, a combination of high-temperatures and oxygen deprived conditions (and a ldh deletion strain) results in only 3% carbohydrates loss after the hydroysis of 10% lactose.

### Example 5: Genetic modification of a Corynebacterium glutamicum strain for production of tagatose and fructose

### 5.1 Plasmids for expression of xylA and araA

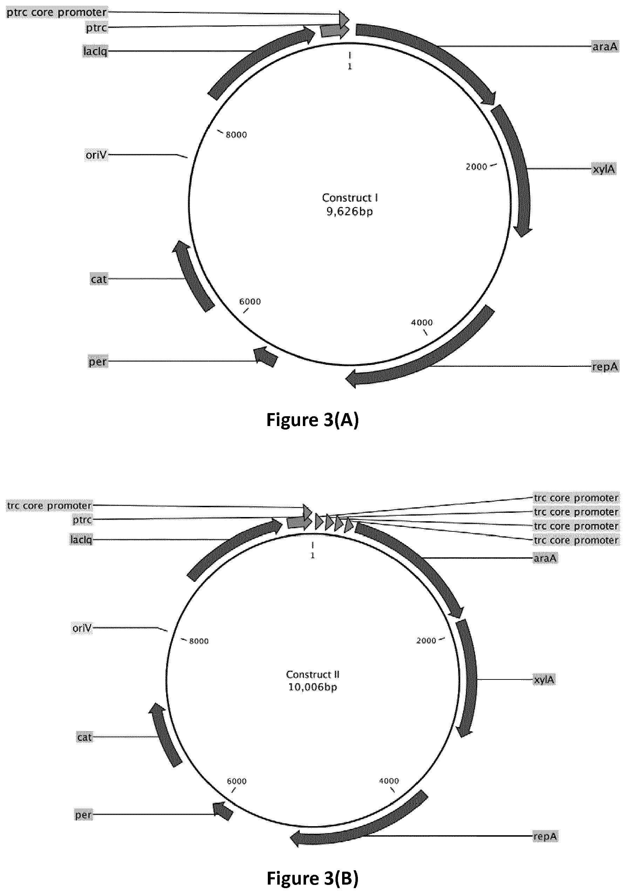

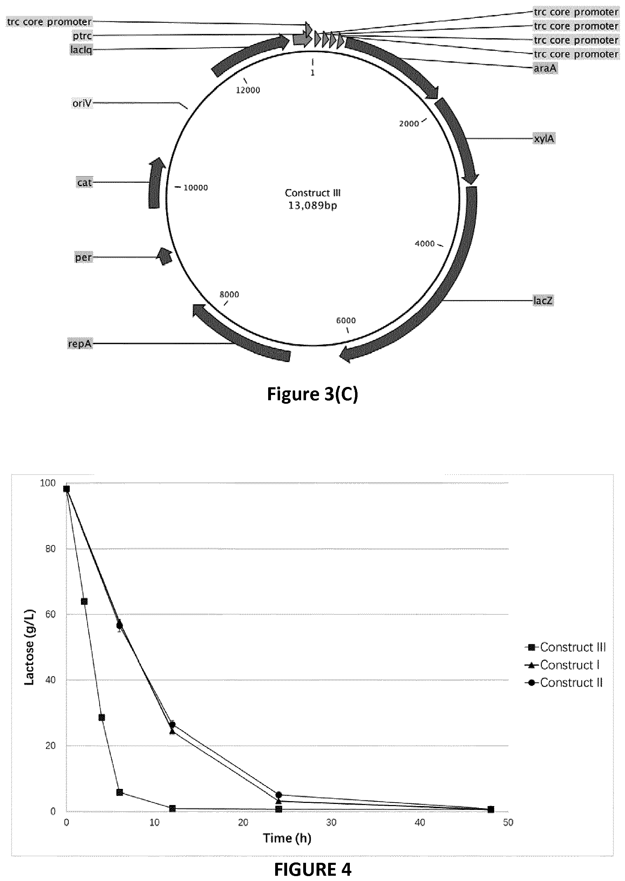

Three expression plasmids: Construct I, II, III have been constructed for simultaneous hydrolysis of lactose and isomerization of glucose and galactose to fructose and tagatose, respectively. The C. glutamicum - E. coli shuttle vector pEC-XC99E [Kirchner & Tauch, 2003] was used as a backbone for the constructs. Cloning was performed using Gibson assembly [Gibson et al., 2009]. For Construct I, the gene fragments of araA (SEQ ID NO: 49, encoding arabinose isomerase) and xylA (SEQ ID NO: 43, encoding xylose isomerase) were cloned into the XbaI site of pEC-XC99E. For Construct II, two trc promotor core fragments (SEQ UD NO: 51) were first cloned into the BamHI site of pEC-XC99E, which resulted in a pEC-XC99E derivative with three trc promotors and a new BamHI site. Two additional copies of the trc promotor core fragment were then cloned into the new BamHI site of the derived vector, which resulted in a pEC-XC99E derivative with a total of five trc promotors (one full length plus four core fragments). Finally, the araA (SEQ ID NO: 49) and xylA (SEQ ID NO: 43) genes were cloned into the SalI site of the pEC-XC99E with the five trc promotors. For Construct III, a lacZ gene from S. thermophilus (SEQ ID NO: 7) was cloned into the PstI site of Construct II. The three constructs are sketched in Figure 3A-C.

### 5.2 Inserting plasmids Construct I, II, and III

Electrocompetent cells were prepared using the protocol as previously described [Eggeling & Bott, 2005]. The constructs were electroprorated into C. glutamicum **JS0093A Δldh** on an electroporator with the voltage of 2.5 Kv (MicroPulser, Bio-Rad), and transformants were selected on the BHI agar with 10 µg/ml chloramphenicol.
The C. glutamicum strain deleted for ldh and comprising transgenes LacS*Z, galMKTE and Construct I, I or III are referred to as strains **JS0093AI**, **JS0093AII** and **JS0093AIII**, respectively.

### Example 6: Production of tagatose and fructose from lactose by genetically modified Corynebacterium glutamicum

### 6.1 Cultivation, hydrolysis and isomerization conditions

1-ml overnight cultures of C. glutamicum strains **JS0093AI**, **JS0093AII** and **JS0093AIII** in BHI broth (Sigma) were inoculated into shake flasks with 50-ml CGXII [Eggeling & Bott, 2005] supplied with 5% lactose and 1mM IPTG. After 24-hour cultivation at 30°C, 220 rmp; the cells were centrifuged at 5,000 g for 10 minutes. The pellets were washed once with 0.9 % NaCl solution.

For the hydrolysis of lactose and further isomerization, the cell pellets were resuspended at a concentration of 15 g (cell dry weight)/L in 40 mM (3-(N-morpholino) propanesulfonic acid) (MOPS)solution (pH=7.5) containing 10% lactose or whey permeate (pH=7.5), and incubated under static (oxygen deprived) conditions at 50°C. The sugar products were analyzed by HPLC as previously described.

### 6.2 Lactose hydrolysis by C. glutamicum strains JS0093AI, JS0093AII and JS0093AIII

The lactose hydrolysis profile of C. glutamicum strains **JS0093AI**, **JS0093AII** and **JS0093AIII** is illustrated in Figure 4. In the lactose-utilizing mutant, **JA0093A**, only one copy of the lacZ gene was integrated into the chromosome, which could potentially be the rate-limiting step of the cascade reaction. Therefore, an additional lacZ gene was introduced on the expressing vector (Construct III) to expedite the lactose hydrolysis. Lactose hydrolysis is significantly improved in strain JS0093AIII comprising the extra lacZ gene. This is further confirmed when calculating the maximum lactose hydrolysis rates of C. glutamicum strains **JS0093AI**, **JS0093AII** and **JS0093AIII**, arriving at 6.0, 6.1 and 15.7 g/L/h, respectively, as presented in Figure 5.

### 6.3 Fructose and tagatose production from lactose by C. glutamicum strains JS0093AI, JS0093AII and JS0093AIII

For the sugar isomerization, lactose is first hydrolyzed into glucose and galactose, and the two monosaccharides, glucose and galactose, are further isomerized into fructose and tagatose, respectively. The maximum isomerization rates (identified using linear regression of several data points between 0 and 48 hours) for glucose to fructose and galactose to tagatose of strains **JS0093AI, JS0093AII** and **JS0093AIII** are presented in Figure 6.

The low copy number of C. glutamicum plasmids (10 - 30 copies per chromosome) [Patek & Nesvera, 2013] limits the expression level of heterologous proteins. Expression can be enhanced by using multiple promoters [Li et al., 2012]. By introducing additional four trc promotors (construct II), the maximum production rate for fructose and tagatose were increased approximately by 40% and 80%, respectively (Figure 6, Construct II vs. Construct I).

As the lacZ, xylA and araA genes were concurrently expressed in C. glutamicum, the isomerization occurred upon the release of glucose and galactose. By introduction of an extra lacZ and thereby faster release of glucose and galactose with Construct III, the maximum isomerization rates for both sugars were further increased (Figure 6; Construct III vs. Construct II).

### 6.4 Fructose and tagatose production from whey by C. glutamicum strain JS0093AIII

The simultaneous production of fructose and tagatose from whey using C. glutamicum strain JS0093AIII is illustrated in Figure 7. After a 35-h incubation at 60°C, approximately 23 g/L fructose, and 22 g/L tagatose were produced from 98 g/L lactose in the whey permeate, respectively, in which 25 g/L glucose and 26 g/L galactose remained. The total recovery of sugar is approximately 98%.

### REFERENCES

Barrett et al 2004. Heterlogous expression of lactose and galactose utilizing pathways from lactic bacteria in Corynebacterium glutamicum for production of lysine in whey. Appl Envion Microbiol Vol 70, pp 2861-6.
Brooker, R.J., 1990. The lactose permease of Escherichia coli. Research in Microbiology. Vol 141, pp 309-315.
Eggeling, L. & Bott M., 2005. Handbook of Corynebacterium glutamicum. CRC Press
Gibson et al., 2009. Enzymatic assembly of DNA molecules up to several hundred kilobases. Nature methods Vol 6, pp 343 - 345.
Howell N., Akagi JM., & Himes RH, 1969. Thermostability of glycolytic enzymes from thermophilic clostridia. Canadian journal of microbiology. Vol 15, pp 461 - 464.
Kirchner O. & Tauch A., 2003. Tools for genetic engineering in the amino acid-producing bacterium Corynebacterium glutamicum. Journal of Biotechnology. Vol 104, pp 287 -299.
Li M. et al., 2012. A strategy of gene overexpression based on tandem repetitive promoters in Escherichia coli. Microbial Cell Factories. Vol 11, pp 1 - 10.
Ling KC, 2008. Whey to Ethanol: A Biofuel Role for Dairy Cooperatives?. USDA Rural Development
Patek M. & Nesvera J., 2013. Promoters and Plasmid Vectors of Corynebacterium glutamicum. Microbiology Monographs. Vol 23,
Sambrook et al., 1989. Molecular Cloning: A Laboratory Manual, Second Edition, Cold Spring Harbor Laboratory Press.
Schäfer A et al 1994. Small mobilizable multi-purpose cloning vectors derived from the Escherichia coli plasmids pK18 and pK19: selection of defined deletions in the chromosome of Corynebacterium glutamicum. Gene. Volume 145, pp 69 - 73.
Shen et al 2017: A novel genetic tool for metabolic optimization of Corynebacterium glutamicum: efficient and repetitive chromosomal integration of synthetic promoter-driven expression libraries. Appl Microbiol Biotechnol. 2017 Jun;101(11):4737-4746. doi: 10.1007/s00253-017-8222-8. Epub 2017 Mar 30.
Rytter et al 2014: Synthetic promoter libraries for Corynebacterium glutamicum. Appl Microbiol Biotechnol. 2014 Mar;98(6):2617-23. doi: 10.1007/s00253-013-5481-x. Epub 2014 Jan 24.
Tsuge Y et al., 2015. Glucose consumption rate critically depends on redox state in Corynebacterium glutamicum under oxygen deprivation. Vol 99, pp 5573 - 5582.

## Claims

1. A genetically modified Corynebacterium for hydrolyzing lactose, wherein said modified Corynebacterium comprises transgenes encoding polypeptides, wherein said polypeptides comprise
i. a modified lactose transporter polypeptide, wherein the amino acid sequence of said modified lactose transporter has at least 80% sequence identity to SEQ ID NO: 2, and wherein amino acid residue Pro148 is substituted by an amino acid selected from the group consisting of glycine, alanine, valine, cysteine, leucine, isoleucine, and methionine; and
ii. a polypeptide having beta-galactosidase activity (E.C. 3.2.1.23).

2. The genetically modified Corynebacterium according to claim 1, wherein said modified Corynebacterium further comprises transgenes encoding polypeptides having enzymatic activity of
iii. galactose mutarotase (E.C. 5.1.3.3),
iv. galactokinase (E.C. 2.7.1.6),
v. galactose-1-phosphate uridylyltransferase (E.C. 2.7.7.10), and
vi. UDP-galactose-4-epimerase (E.C. 5.1.3.2).

3. The genetically modified Corynebacterium according to claim 1 or 2, wherein said modified Corynebacterium further comprises one or more transgenes encoding polypeptides having enzymatic activity of
vii. xylose isomerase (E.C. 5.3.1.5) and/or
viii. arabinose isomerase (E.C. 5.3.1.4).

4. The genetically modified Corynebacterium according to any one of claims 1-3, wherein said modified Corynebacterium is deleted for or lacks genes encoding polypeptides having an enzymatic activity of lactate dehydrogenase (E.C. 1.1.1.27).

5. The use of a genetically modified Corynebacterium according to any one of claims 1-4 for hydrolyzing lactose in a lactose-containing medium.

6. The use of a genetically modified Corynebacterium according to claim 5, wherein said lactose-containing medium is whey permeate.

7. A mutant gene encoding a modified lactose transporter wherein the amino acid sequence of said lactose transporter has at least 80% sequence identity to SEQ ID No: 2 wherein amino acid residue Pro148 is substituted by an amino acid selected from the group consisting of glycine, alanine, valine, cysteine, leucine, isoleucine, and methionine.

8. A method for hydrolyzing lactose comprising the steps of
a. bringing a genetically modified Corynebacterium into contact with a lactose-containing medium, wherein said modified Corynebacterium comprises transgenes encoding polypeptides, wherein said polypeptides comprise
i. a lactose transporter polypeptide and
ii. a polypeptide having beta-galactosidase activity (E.C. 3.2.1.23),
b. incubating the culture of step (a) under aerobic conditions at a temperature between 20-37 degree Celsius,
c. increasing the temperature of the culture of step (b) to 50 degree Celsius or higher to reach an optimum temperature for said beta-galactosidase enzyme, and
d. optionally purifying products of lactose hydrolysis from the culture of step (c).

9. The method according to claim 8, wherein said Corynebacterium is further deleted for or lacks genes encoding polypeptides having lactate dehydrogenase activity (E.C. 1.1.1.27); and wherein the culture conditions in step (c) are additionally shifted to oxygen-deprived.

10. The method according to any one of claims 8-9, wherein the amino acid sequence of said lactose transporter polypeptide has at least 80% sequence identity to SEQ ID NO: 2 wherein amino acid residue Pro148 is substituted by an amino acid selected from the group consisting of glycine, alanine, valine, cysteine, leucine, isoleucine, and methionine.

11. The method according to any one of claims 8-10, wherein the genetically modified Corynebacterium further comprises transgenes encoding polypeptides having enzymatic activity of
iii. galactose mutarotase (E.C. 5.1.3.3),
iv. galactokinase (E.C. 2.7.1.6),
v. galactose-1-phosphate uridylyltransferase (E.C. 2.7.7.10), and
vi. UDP-galactose-4-epimerase (E.C. 5.1.3.2).

12. The method according to any one of claims 8-11, wherein the genetically modified Corynebacterium further comprises one or more transgenes encoding polypeptides having enzymatic activity of
vii. xylose isomerase (E.C. 5.3.1.5) and/or
viii. arabinose isomerase (E.C. 5.3.1.4).

13. The method according to any one of claims 8-12, wherein said lactose-containing medium is whey permeate.
